(19) Europäisches Patentamt
European Patent Office
Office européen des brevets

(11) Publication number : 0 578 515 A2

(12) # EUROPEAN PATENT APPLICATION

(21) Application number : 93401328.5

(51) Int. Cl.$^5$ : **C12N 15/13,** C12P 21/08

(22) Date of filing : 24.05.93

The applicant has subsequently filed a sequence listing and declared, that it includes no new matter.

(30) Priority : 26.05.92 US 888233

(43) Date of publication of application : 12.01.94 Bulletin 94/02

(84) Designated Contracting States : AT BE CH DE DK ES FR GB GR IE IT LI LU MC NL PT SE

(71) Applicant : **Bristol-Myers Squibb Company**
**345 Park Avenue**
**New York, N.Y. 10154 (US)**

(72) Inventor : **Harris, Linda J.**
**1214 16th Ave E.**
**Seattle, WA 98112 (US)**
Inventor : **Bajorath, Jurgen**
**928 137th St. SW**
**Seattle, WA 98204 (US)**
Inventor : **Ku-Chuan, Hsiao**
**18730 50th Avenue NE**
**Seattle, WA 98155 (US)**

(74) Representative : **Beauchamps, Georges**
**Cabinet Z.Weinstein 20, avenue de Friedland**
**F-75008 Paris (FR)**

(54) **Humanized monoclonal antibodies.**

(57) A method of preparing humanized monoclonal antibodies is described which utilizes comparative model burding methodology. A humanized anti-CD18 antibody, 60.3, has been formulated and demonstrated to have analogous binding characteristics to the original murine monoclonal antibody, while displaying essentialy complete human Ig heavy and light chains.



Jouve, 18, rue Saint-Denis, 75001 PARIS

## Technical Field

The present invention is directed to a method for producing humanized monoclonal antibodies by utilizing comparative model building to construct the humanized antibody from homologous regions of human proteins by rational design. Specific humanized monoclonal antibodies are prepared.

## Background of the Invention

Murine derived monoclonal antibodies have been utilized as diagnostic and therapeutic agents for numerous human pathologic conditions including acute inflammatory responses associated with numerous diseases. Administration of murine derived monoclonal antibodies (mAbs) as therapeutic agents in man has been severely limited by the development of antibody within the recipient to the mouse antigens of the murine derived monoclonal antibody. In attempts to circumvent this outcome mAbs have been restructured by recombinant DNA technology in such a way as to decrease their immunogenicity in humans. Immunoglobulins are well defined both chemically and biologically with the general structures illustrated in Molecular Cell Biology. Darnell, Lodish and Baltimore, Eds.Scientific American Book, Inc. W.H. Freeman, New York, NY (1986). Initially, this involved the construction of chimeric antibodies, Morrison et al. Proc. Natl. Acad. Sci. USA 81: 6851-6855 (1984). Recombinant technology was employed to replace the murine heavy and light chain constant regions with corresponding human constant regions. Upon expression, such interspecies antibody chimeras yielded molecules with the antigen binding specificities of the parent murine antibody. The following references generally describe chimeric antibody technology: Lobuglio et al. Proc. Natl. Acad. Sci. USA86: 4220-4224 (1989) : United States Patent 4,816,567 ; PCT International Publication No. WO 87/02671, published May 7, 1987 ; European Patent Publication No. 255,694, published February 10, 1988 ; European Patent Publication No. 274,394, published July 13, 1988 ; European Patent Publication No. 323,806, published July 12, 1989 ; PCT International Publication No. WO/89/00999, published February 9, 1989; European Patent Publication No. 328,404, published August 16, 1989; European Patent Publication No. 322, 424 published September 13, 1989, and European Patent Publication No. 438,310 published July 24, 1991.

The immunogenicity of chimeric antibodies can be further reduced by grafting rodent hypervariable regions into the variable region frameworks of human light and heavy chains, Jones et al. Nature 321 : 522-525 (1986). These hypervariable regions have also been termed complementarity determining regions (CDR). The technique involves the substitution or recombinant grafting of antigen-specific murine CDR sequences for those existent within "generic" human heavy and light chain variable regions, European Patent Publication No. 239,400, published September 30, 1987. In this approach, little, if any, concern is shown for the variable region frameworks (FRs) within which the murine CDR's are placed.

Studies by Queen et al, Proc. Natl. Acad. Sci. USA 86 : 10029-10022 (1989), have shown the CDRs from a murine anti-Tac monoclonal antibody can be grafted into a human framework. The human framework variable regions were chosen to maximize identity with the murine sequence. The authors also utilized a computer model of the mMab to identify several amino acids which, while outside the CDRs, are close enough to interact with the CDRs or antigen . These residues were mutated to the residue found in the murine sequence. The grafted anti-Tac antibody had an affinity for the antigen which was only about 1/3 that of the murine anti-Tac mAb.

Leukocyte infiltration into an inflammatory site is dependent on the adhesion of the leukocytes to the endothelium prior to extravasation. The rapid binding of polymorphonuclear leukocytes (PMN) to the endothelium and diapedesis occurs within minutes after the introduction of a chemotactic stimulus in tissue, Cybulski et al., Am. J. Pathol. 124 : 367 (1986). This rapid extravasation appears to depend on the response of the PMNs to chemoattractants and on the presence of the CD11/CD18 family of glycoproteins on the leukocyte surface. The family of glycoproteins associated with PMNs are termed leukocyte integrins and include LFA-1 (CD11a/CD18), Mac-1 (CD11b/CD18) and p150,95 (CD11c/CD18). Each of these heterodimers has a unique alpha chain (CD11 a, b, c) and an invariant beta-2 chain (CD18). Stimulation of PMNs with various chemotactic factors causes increased expression of leukocyte integrins (CD11b/CD18) fostering strong adhesion to unstimulated endothelium in vitro; Harian, Blood 65 :513 (1985). Essentially all of the chemoattractant-induced adhesion is inhibited by treating the PMNs with mMAbs specifically reactive with the CD11/CD18 complex, Harian et al., Blood 66 : 167 (1985); Zimmerman and McIntyre J. Clin. Invest 81 : 531 (1988); Smith et al., J. Clin . Invest 82 : 1746 (1988) and Lo et al., J. Exp. Med. 169 : 1779 (1989).

Murine hybridomas producing monoclonal antibodies reactive with the beta chain common to the Mac-1, LFA-1 and the p150,95 integrins have been described. The mMAbs are designated 1B4, 60.3, TS1/18, H52 and ATCC TIB 218. The 1B4 is an IgG1 antibody and was prepared by Wright et al. Proc. Natl. Acad. Sci. USA 80 : 5699-5703 (1983), the 60.3 is also IgG2a and was prepared by Beatty et al. J. Immunol. 131 :2913-2918 (1983), TS1/18 is an IgG1 antibody and was prepared by Sanchez-Madrid et al., J. Exp. Med. 158 : 1785-1803 (1983), and ATCC TIB 218, a IgG2a kappa prepared by Springer et al , J. Exp. Med. 158 : 586-602 (1983). These antibodies appear to be functionally equivalent and cross-react with the beta 2 - chain found on human, sheep, pig, rabbit and dog leukocytes but not with the beta-2 chain found on murine and rat leukocytes.

## Summary of the Invention

The present invention is directed to a method for producing humanized monoclonal antibodies by utilizing a process of comparative model building. In this method computer data bases are searched to locate homologous human protein sequences that correspond to specified regions of the non-human derived (usually murine) antibody, and a series of models is formulated, tested and modified to produce a model of a humanized antibody which is then constructed by recombinant DNA technology. In a preferred embodiment, a humanized monoclonal antibody corresponding to the murine anti-CD18 antibody 60.3 was prepared.

The variable (V) region sequences from both the heavy (H) and light (L) chains were determined from cDNA (amplified by PCR), and spliced onto human constant (C) regions, resulting in a chimeric 60.3 Ab (IgG1, kappa). The chimeric Ab was expressed in tissue culture (Ag8.653 mouse myeloma cells, detected by ELISA), and examined in binding assays. The results from competition and inhibition assays showed that the chimeric Ab was as effective as the murine 60.3 mAb.

The deduced murine $V_H$ and $V_L$ protein sequences were compared to the protein sequence data base, and two human Ig protein sequences were selected to be used as templates. The present inventors modeled a murine 60.3 Fv according to the deduced $V_H$ and $V_L$ protein sequences. Based on the 60.3 Fv model and the two human template sequences selected from the protein data base, a humanized Fv was modeled.

Construction of the humanized 60.3 was done by piecing 5 pairs of complementary oligonucleotides together (spanning the entire V region) to form the VH and VL. These were then attached onto vectors containing genes for appropriate C regions to form humanized Ab (IgG1, kappa). The humanized proteins were again expressed in Ag8.653 cells and binding assays were done. FACS analyses indicated that the humanized Ab recognized cells expressing CD18. About a dozen of the humanized 60.3 Ab master wells were transferred and assayed for Ig.

## Brief Description of the Drawings

In the drawings:

Figure 1 illustrates an amino acid comparison of the murine 60.3 antibody heavy chain (m60.3) with the human variable heavy chain consensus sequence for the framework regions of human subgroup $V_H1$ (hVh1/Jh4), the human template (M030) used for humanization (h60.3 template), a germline sequence homologous to M030 (21-2 'CL), and phases I to IV of the humanization process. All amino acids which are identical to the phase IV sequence are shaded. In addition, sequences which are different than the previous phase sequence are shown in bold.

Figure 2 illustrates an amino acid residue comparison of the murine 60.3 antibody light chain (m60.3) with the human variable light chain consensus sequence for the framework regions of human subgroup $V_kIII$(hVkIII/Jk), phases I-IV of the humanization process and the human template used for the humanization (h60.3 template). All amino acids which are identical to the phase IV sequence are shaded. In addition, sequences which are different than the previous phase sequence are shown in bold.

FIGURE 3 illustrates five pairs of complementary oligonucleotides corresponding to the variable regions of the light chain.

FIGURE 4 illustrates five pairs of complementary oligonucleotides corresponding to the variable region of the heavy chain.

FIGURE 5 illustrates the binding of murine, chimeric and humanized 60.3 antibody to HL60 human myelomonocytic cells. Fluorescein isothiocyanate (FITC) labelled antibody was incubated with cells at the concentrations indictated on the abscissa and the amount of antibody bound is indicated by relative fluorescence intensity on the ordinate.

FIGURE 6 illustrates the competition by preincubation of cells with chimeric and humanized 60.3 of the binding of FITC-labelled murine 60.3 to HL60 cells. HL60 cells were preincubated with 1 ug/ml of either chimeric 60.3 (circles) or humanized 60.3 (squares), followed by incubation with various concentrations of FITC-labelled murine 60.3. In the absence of competing antibody, FITC - m60.3 binding to the HL60 cells increased with increasing concentration (x).

FIGURE 7 illustrates the direct competition of FITC-murine 60.3 binding to HL60 cells by chimeric and humanized 60.3. The dashed line shows the fluorescent intensity of binding by FITC-murine 60.3 in the absence of competitor, while additions of increasing concentrations of chimeric 60.3 (squares) and humanized 60.3 (circles) inhibited FITC-m60.3 binding.

FIGURE 8 illustrates the results of a chemiluminescence binding assay of murine (closed square), chimeric (open square) and humanized (closed diamond) 60.3 antibody upon HL60 cells. The anticancer antibody L6 (open diamond), which does not bind to HL60 cells, was used as a control.

FIGURE 9 illustrates a series of restriction maps for plasmids utilized in the production of the variable light chain plasmid pGK.11.

FIGURE 10 illustrates the nucleotide sequence for the humanized variable light chain.

FIGURE 11 illustrates a series of restriction maps for plasmids utilized in the production of the variable heavy chain plasmid pNγ1.16.

FIGURE 12 illustrates the nucleotide sequence for the humanized variable heavy chain.

## Description of Preferred Embodiments

The present invention is directed to a method of producing humanized monoclonal antibodies (mAbs) by utilizing a process of comparative model building and rational design. In a preferred embodiment this method is utilized to produce a humanized molecule of the anti-CD18 murine monoclonal antibody 60.3. The mouse mAb 60.3 (IgG2a), which recognizes a functional epitope on the beta subunit (CD18) of leukocyte integrins, prevents the adherence and aggregation of polymorphonuclear neutrophils (PMN), resulting in a blockage of neutrophil mediated damage during shock and reperfusion injury. It would be advantageous to modify mAb 60.3 to reduce the potential for HAMA (human anti-mouse Abs) response. Therefore, in one embodiment, the present invention was directed to "humanize" the 60.3 mAb by creating an Ab whose constant (C) region is human, but whose variable (V) region is composed of both human (principally framework sequences) and mouse (principally CDR loops) sequences. For the studies described in the present invention, murine, chimeric and humanized antibodies were purified from solution by protein A chromatography on IPA - 400 Fast Flow Immobilized rProteinA (Repligen, Cambridge, MA) using the manufacturer's recommended protocol.

In the present invention recombinant methods are utilized to produce humanized monoclonal antibodies that contain complementarity determining regions (CDRs) analogous to the originally derived monoclonal antibody, and which have homologous human heavy and light chain framework regions. The resulting antibodies demonstrate the binding affinity and specificity of the original antibody yet are completely humanized monoclonal antibodies.

As used herein the term "humanized" and its various grammatical forms as it relates to antibodies is defined to mean that the amino acid residues of the antibody in the heavy and light chains are replaced with amino acid residues corresponding to homologous human protein regions without altering the binding activity of the antibody. For the humanized 60.3 monoclonal antibody of the present invention there is approximately 80% sequence identity of the variable regions of the heavy and light chains with those of the human mAb, while the constant regions are distinctly human. Some variation of individual amino acids in the antigen binding and framework regions are contemplated by this invention and are within the scope of this invention when such variations do not interfere or inhibit the binding to antigen, such as the Ile for Glu substitution at position 106 of the light chain.

As used herein the term "canonical loop conformation" refers to a small repertoire of main chain conformations for five of the six loops (all except H3). The particular conformation adopted is determined by only a limited number of residues within the loop or the framework.

As used herein the term "framework residues" means residues which are located outside the structurally defined CDR loops. These residues can be part of the hypervariable regions for the antibody.

As used herein the term "monoclonal antibody" refers to all recombinant antibodies derived from an initial single cell and includes murine monoclonal antibodies, chimeric antibodies and humanized antibodies.

In the present invention a procedure of comparative model building was utilized to construct the appropriately designed humanized antibody. As a preferred embodiment, the modeling of the murine 60.3 antibody is summarized.

The Brookhaven Protein Database (Bernstein et al. (1977) J. Mol. Biol. 122 : 535-542) was searched for the antibody crystal structures which show reasonably high homology (> 50% sequence identity) to the variable regions of murine 60.3. If the variable light chain and heavy chain templates which fulfill these criteria are from different antibodies, these structures are combined by superposition of the set of structural invariants at the $V_L$- $V_H$ domain interface (Novotny et al. (1985) Proc. Natl. Acad. Sci. USA 82 : 4592-4596). This provides the "structural template" for model building of murine 60.3 (and humanized 60.3 below).

The CDR loops and their known structural framework determinants of murine 60.3 are determined by defining the CDR loops structurally according to the method of Chothia et al. (Chothia et al. (1989) Nature 342 :

877- 883). The structurally defined CDR loops consist on average of shorter sequence segments than the hypervariable regions defined by Kabat (Kabat et al (1987) Sequences of Proteins of Immunological Interest). The five canonical CDR loops (L1-13, H1-H2, i.e. all except H3) in the 60.3 variable light and heavy chain are assigned to known canonical loop conformations, and the framework residues which are crucial for the conformations of the CDR loops are determined.

The non-canonical H3 loop region within the 60.3 sequence is defined and a model of murine 60.3 is then built. The CDR loops of the structural template are replaced with canonical CDR backbone templates as determined using interactive computer graphics (INSIGHT II, Ver. 2.0 Biosym Technologies, Inc. 1991). Loop searches (Jones TA (1986) Embo J. : 819-822) in the Brookhaven Protein Data Bank are carried out to extract an initial backbone approximation for the non-canonical CDR loop H3.

All non-conserved amino acid side chains in similar positions are replaced using interactive computer graphics. The model then consists of a combination of backbone fragments of different antibodies with replaced side chains. The model is solvated with a 7 Å water layer and the structure is refined using an energy minimization (Mackay et al. (1989) Prediction of Protein Structure and the Principles of Protein Conformation. New York: Plenum Press pp.317-358) protocol where, over the course of 1600 cycles of conjugate gradients minimization, constraints of 80 kcal/mol/Å$^2$ on all protein non-hydrogen atoms are gradually released until, at the final stage of the minimization procedure, all atoms of the system are free to move.

The most homologous human variable region sequences are found by searching the sequence data base for the most homologous human sequences for the variable light and the variable heavy chains of the 60.3 antibody and these sequences are combined to obtain a "human template". The structural template for murine 60.3 is confirmed to be suitable for the human template. The sequences of the structural template chosen initially showed > 50% sequence identity to the variable regions of the "human template". Furthermore the percent homology is chosen to be similar to that found for comparison of the structural template with the murine sequence. The CDR loops and known structural determinants are then grafted onto the human template (Jones et al. (1986) Nature 321 : 522- 535). The CDR loop regions and structural determinants in the "human template" sequence are replaced by the analogous sequences from the murine antibody, as determined above. This provides the Phase I h60.3 sequence. A Phase I model of humanized 60.3 is built using the same model building protocol as described for murine 60.3.

In Phase II the murine and Phase I h60.3 models were compared. These models now consist of the murine binding site and murine framework (murine 60.3 or m60.3) and of murine binding site and human framework (Phase I humanized 60.3 or h60.3). The of murine and Phase I h60.3 were superimposed using the structural invariants of the immunoglobulin fold (Novotny et al. (1985) Natl. Acad. Sci. USA 82 : 4592-4596). The models of the binding site regions were compared residue by residue from the N-terminus to the C-terminus. By this comparison, all framework residues and residues within the framework - CDR junctions which can interact with the murine CDR loops and may therefore be important for the structural integrity of the murine binding site were identified. These residues typically include all the known structural determinants for the specified canonical CDR loop conformations (Chothia et al. (1989) Nature 342 : 877-883) and other residues found to be critical in the comparison (due to proximity to the CDR loops and potential for interaction with them). These residues were then "re-mutated" to the murine residues, forming the Phase II h60.3 model.

The murine 60.3 model and the modified humanized sequence were then further refined by again subjecting the models to the energy minimization procedure described above. This construct represents the Phase II h60.3 model.

In Phase III, further improvements of the structural model of h60.3 were made A conformational search (Bruccoleri RE and Karplus M. (1987) Biopolymers 26 : 137- 168) was carried out over regions of the binding site which cannot be directly assigned to known structural templates. Typically, this is the CDR loop H3 and perhaps one or more CDR loops which may not belong to known canonical structure types. Side chain conformations of the antigen binding site loops and the framework - CDR junctions are also further refined using an iterative conformational search protocol (Bruccoleri RE and Karplus M. (1987) Biopolymers 26 : 137-168). The refined model structure may be called Phase III h60.3.

In Phase IV, analysis of the binding site features of the Phase III h60.3 model was carried out. The binding site features of the construct were analyzed in detail in order to classify the antibody structure, for example, as a "groove-type" or "cavity-type" or "flat" antibody. This allows one, in the absence of detailed structural knowledge of the antibody-antigen complex, to postulate which parts of the CDR surface or residues at the CDR-framework junctions are unlikely to be involved in antigen binding. In the Phase III and earlier models, these positions may be occupied by murine residues which can now be changed to human residues.

This improves the "degree of humanization" of the antibody since parts of some CDR loops and other entire CDR loops can be "humanized". At this stage, the final version of humanized 60.3, Phase IV h60.3 was obtained.

Comparative molecular modeling has been used here to enable a detailed three-dimensional comparison of a murine antibody and its humanized version. This comparative study has enabled the present inventors to analyze residue-residue interactions which are likely to be critical to retain the murine specificity in the structural context of a largely human antibody. Furthermore, the different modeling concepts based on structural homology (experimental structural data) and conformational search (which represents an *abinitio* method) have been combined to obtain the best possible picture of the 60.3 binding site in order to, gain some insight into which of the binding site residues may be not involved in antigen binding.

In addition to the application summarized above, comparative model building can be applied to other problems. For example, many of the antibody structures which are modeled today are used to guide mutagenesis experiments in order to explore affinity and antigen specificity. Such antibodies are often modeled because experimental structures are not available for these antibodies. Comparative model building provides an opportunity to assess the confidence level of such theoretically derived structures.

For example, the combining site of a clinically relevant antibody can be derived starting from different structural templates and employing the different methods based on structural homology and conformational search. By pairwise combination of two different templates with two different methods, four model structures can be derived in an independent way and then compared by superposition of structural invariants. This comparison allows for the determination of how well the independently derived structures agree and which parts of the models do not show satisfying agreement. In the absence of experimental structural data, such comparative model building exercises presently provide the only way to assess the confidence level of antibody models. If the independently derived structures agree well, a high confidence level can be assigned to the model and a "consensus model" can be prepared. The consensus model would then typically represent a combination of structural elements derived by structural homology and conformational search. On the other hand, disagreement of the models allows for the identification of the particular critical regions in model structures which are less well defined and need to be improved or, if this is not possible, treated with caution. Such knowledge, obtained by comparative model building, is very important for the use of model structure for experimental design.

Although the humanized 60.3 Ab was prepared by grafting the murine CDRs onto the human frameworks, certain amino acids were not changed from the murine protein sequence to their human counterpart (due to their importance in retaining the conformation of the CDR loops). Therefore, three humanized 60.3 L "mutants" (based on computer modeling) were constructed in an effort to 1) further reduce its divergence and 2) determine the contribution of these amino acids on antigen (Ag) binding.

There are 4 amino acids in the L chain which are changed: one is in the CDR2 (postulated not to be involved in binding) and the remaining amino acids all reside in the framework 2 region. The three humanized 60.3 L "mutants" are as follows:

1) Mutant 1: amino acid change is only at position 50, from Arg (R) to Asp (D).

2) Mutant 2 : 3 amino acids changes are carried out at positions 50 (as in Mutant 1), 54 [from Leu (L) to Arg (R)] and 55 [from Glu (E) to Ala (A)].

3) Mutant 3: contains the changes in Mutant 2, and at position 68 Arg (R) is changed to Gly (G).

From this set of mutants, effects can be seen on binding by (1) Arg 68 alone (by comparing results from Mutants 3 and 2) and (2) Leu 54 and Glu 55 (by comparing results from Mutants 2 and 1).

Total cellular RNA was extracted from a 60.3 producing cell-line according to the method of Davis et al (1986). The first strand cDNA was synthesized using a cDNA synthesis kit from InVitrogen and an oligo (dT) primer. The cDNA was then amplified by polyermase chain reaction (PCR) using degenerate primers (Larrick, et al.(1991) Scand. J.. Immun. 32 :121-128; Colloma and Larrick (1991) Biotechniques 11 : 152-156). For the heavy chain, the sense primers (MH-SP-ALT.1 and MH-SP-SLT.2) were from the signal peptide and had an Xho I restriction site and the 5' end. The antisense primer (MH- gamma-CONST) was a consensus sequence from the CH1 domain of murine $\gamma$ heavy chains and had a Pst I site at the 3' end.

For the PCR amplification of the light chain, the sense primers (EcoRI/FR1-ML (k)) were from the 5' end of the first framework region and had an Xho I restriction site at the 5' end. The antisense primer (HindIII/M1(k-)CONST) was from the k constant region, but had a Sal I restriction site instead of the Hind III site described by Larrick et al.

For both the heavy and light chains, the PCR products were either restricted (Xho I and Pst I for the heavy chain; Sal I and Xho I for the light chain) and cloned into similarly restricted pUC 18 or treated with nucleotide kinase followed by blunt ended ligation into Sma I digested pUC 18. Ligation products were used to transform competent DH$\alpha$ E. coli cells.

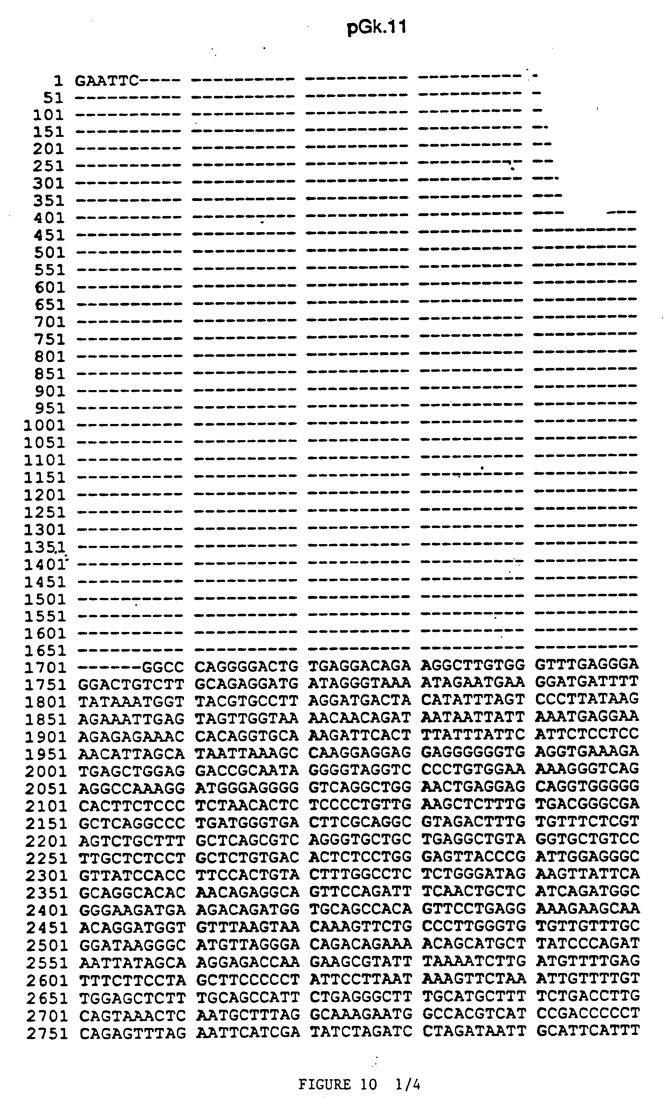

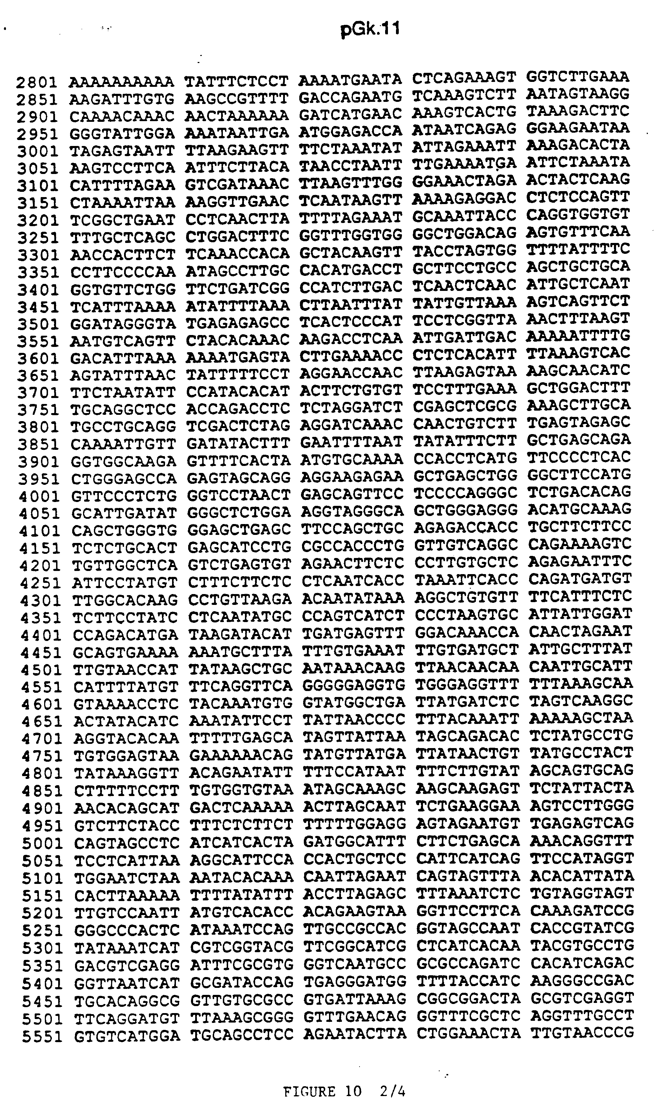

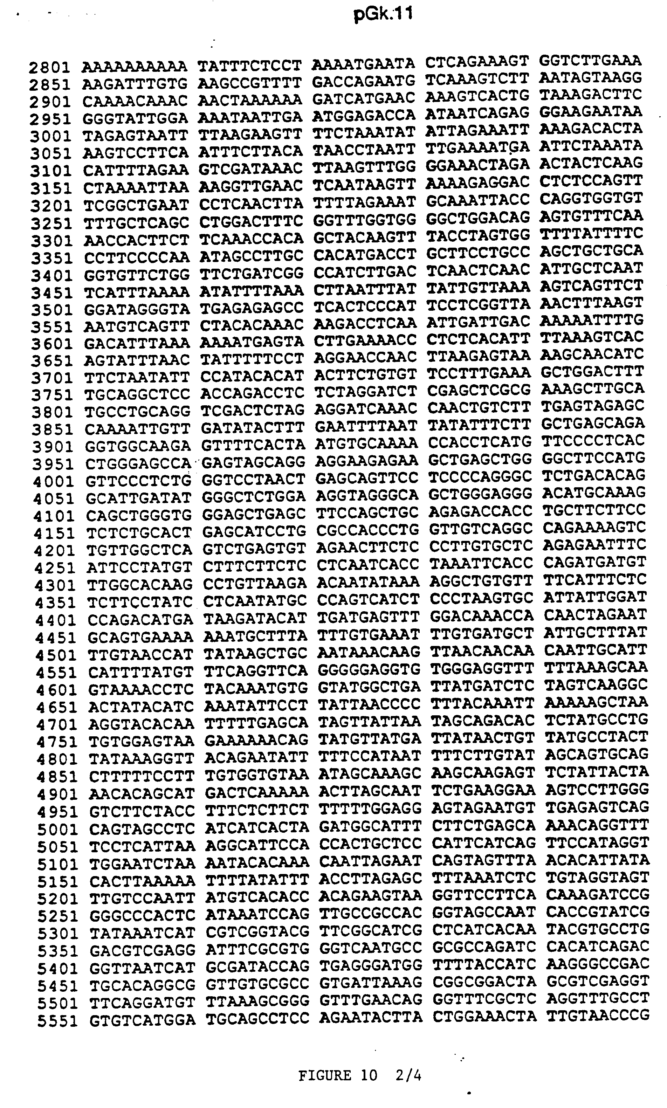

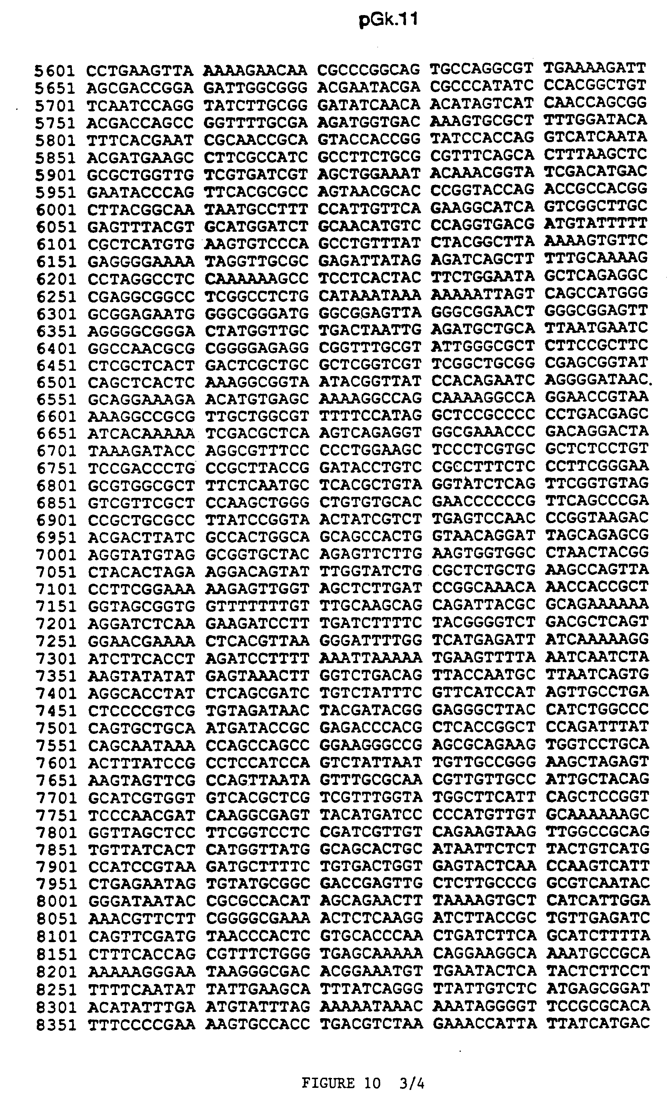

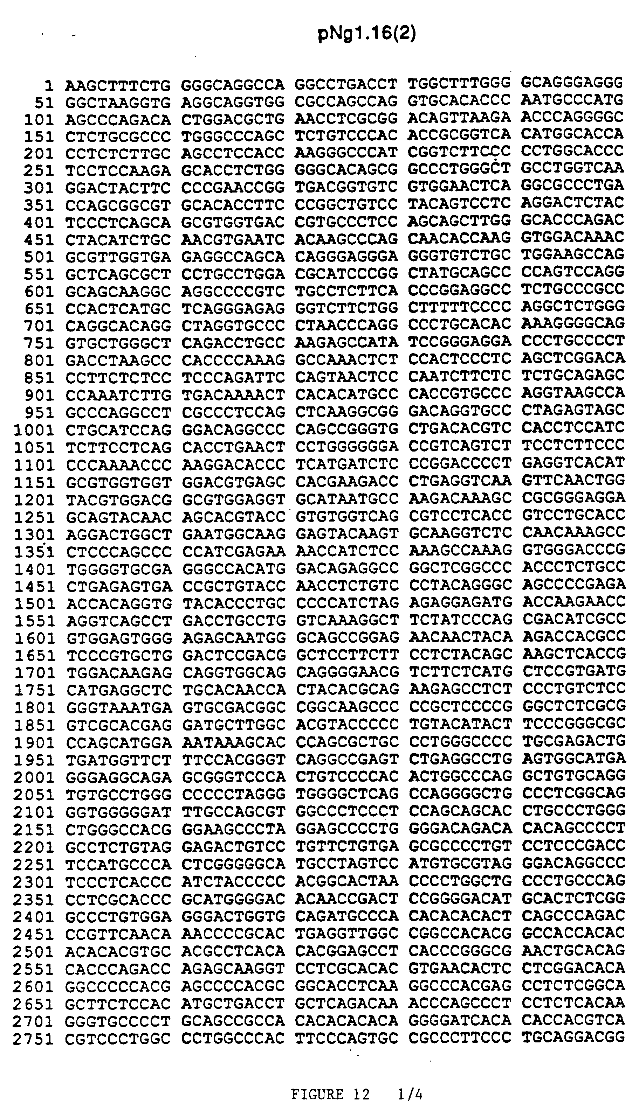

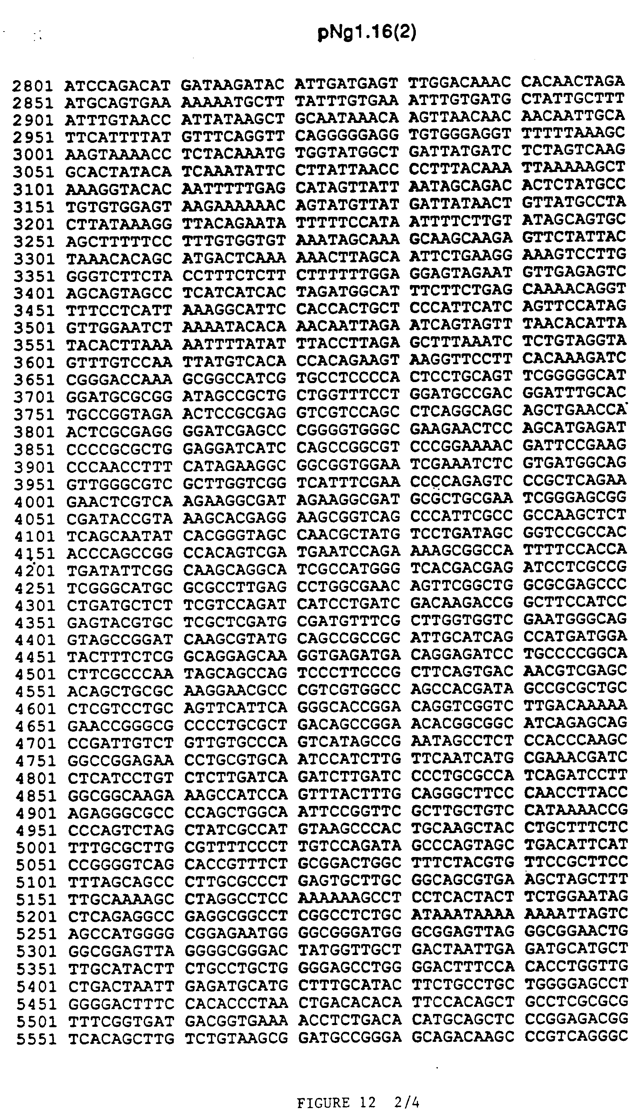

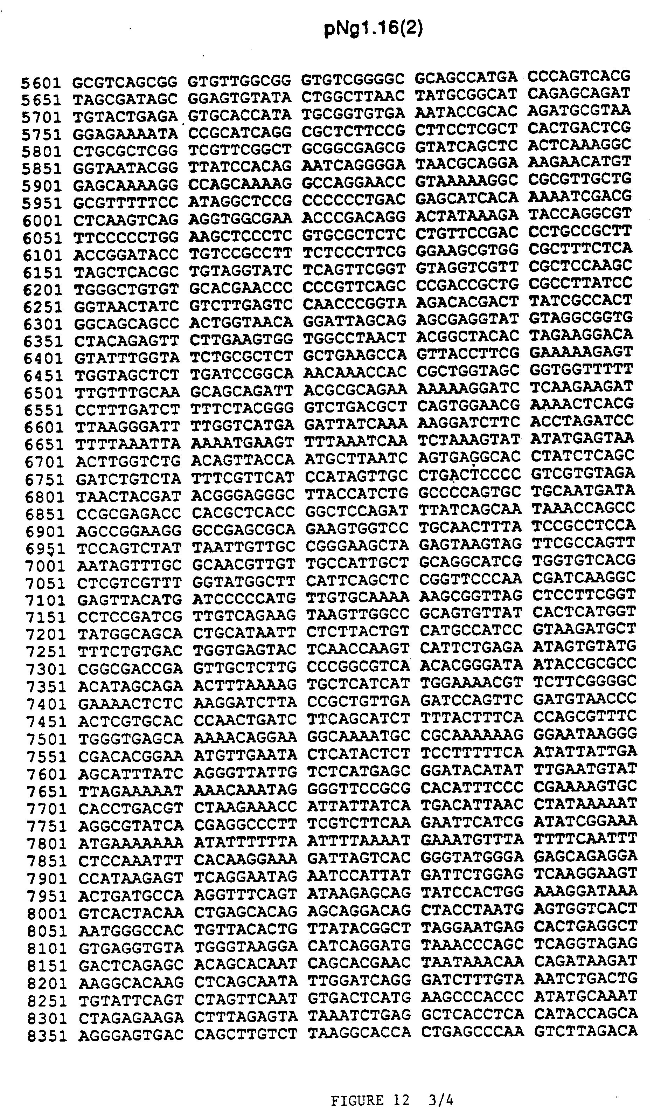

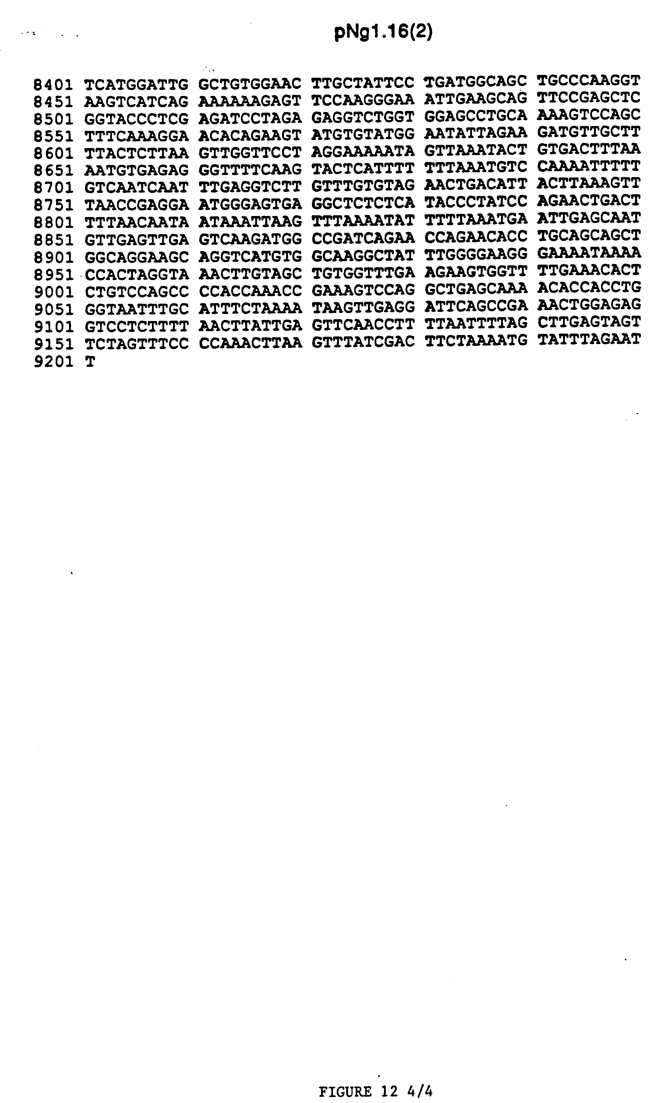

Clones containing inserts were selected using X-Gal/PTG; positive clones were screened for appropriately sized EcoR I-Sal I restriction fragments. EcoR I and Sal I flank the cloning sites in pUC 18 and are therefore expected to release the PCR product producing an approximately 0.5kh fragment. Selected-clones were subjected to the double stranded DNA sequencing (Hsiao (1991) Nucl. Acid Res 19 :2787) using Sequenase (U.S.Biochemical). The sequence is shown in Figures 10 and 12, and Sequence I.D. numbers 9 and 10. The V gene sequences were compared to sequences of other murine Ig genes (Kabat et al, (1987) Sequences of Proteins of Immunological Interest, 4th ed., Nat. Inst. of Health, Bethesda, MD.) The heavy chain was found to belong to the VH IIa subgroup and the light chain to belong to the Vk IIIb subgroup.

In order to be sure that the correct V genes had been cloned and sequenced, heavy and light chain from purified 60.3 were subjected to N-terminal amino acid sequencing. The amino acid sequence of the heavy chain was identical to that deduced from the DNA sequence. However, there was a discrepancy for the 7th and 8th amino acids of the L chain. For the DNA, these amino acids (Tyr and Gln, respectively) are encoded by the sense primer used for the PCR reaction. Ser and Pro were found at these positions by amino acid sequencing. Furthermore, almost all other Vk genes of this subgroup had Ser and Pro at this position. It was concluded that the primers used were not entirely appropriate for the V gene. They were however, similar enough to the cDNA that annealing and priming could occur. The codons for these 4 amino acids occur at the 3' end of the primer and are as follows:

| | | | |
|---|---|---|---|
| Tyr Gln | : | TA (C/T) | CA (A/G) |
| Ser Pro | : | TCX | CCX |

To determine the real sequence at this position, the entire procedure (cDNA synthesis, PCR amplifying, cloning, and sequencing) was repeated using primers which terminated before the codons in question. This showed that Ser and Pro were encoded at positions 7 and 8, respectively. The initial PCR product was rePCRed, using a sense primer which encoded Ser and Pro rather than Tyr and Gln at positions 7 and 8.

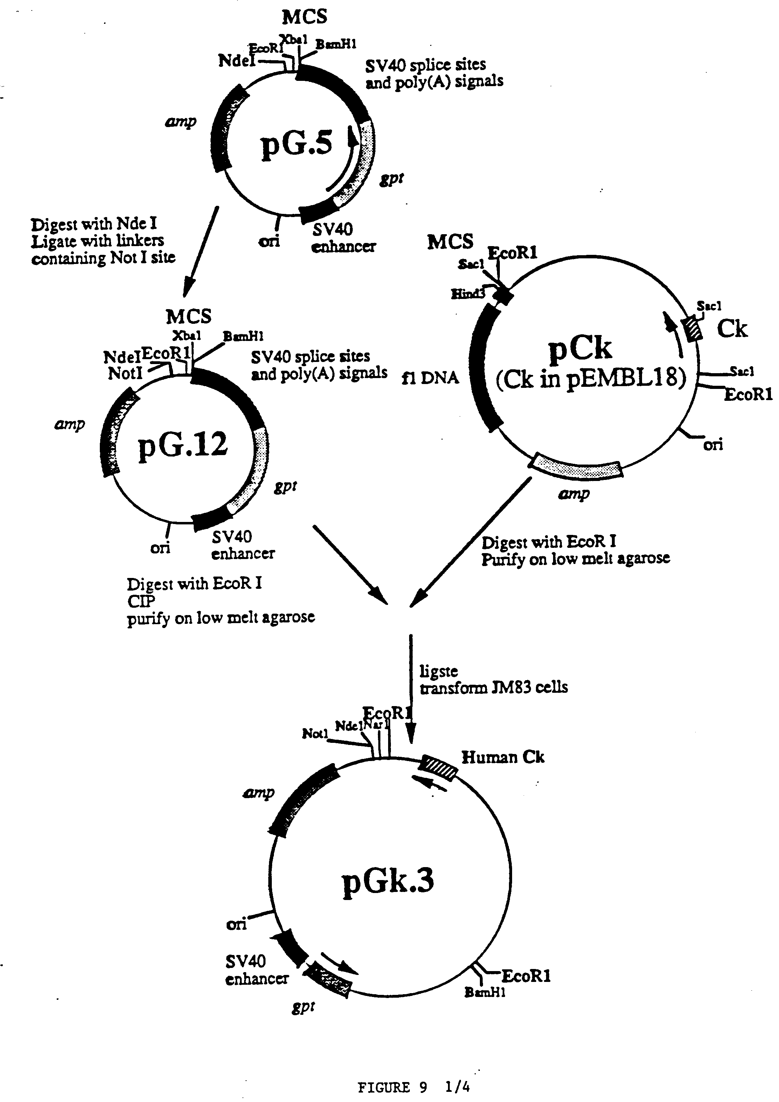

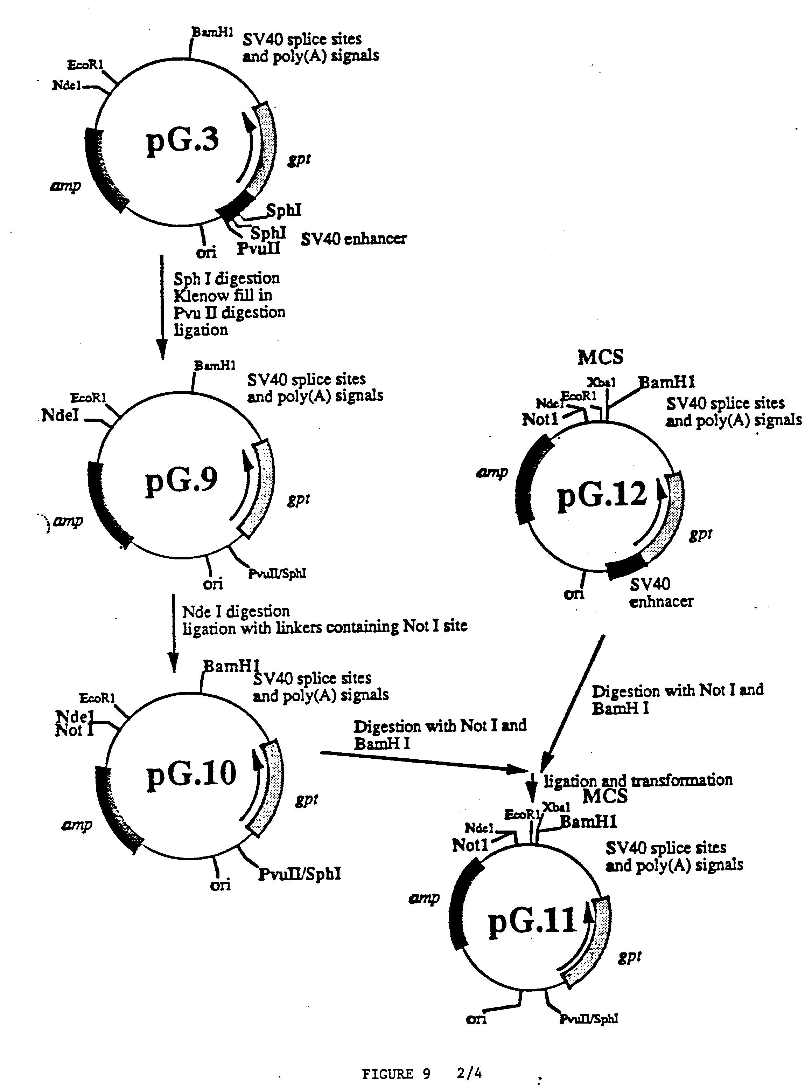

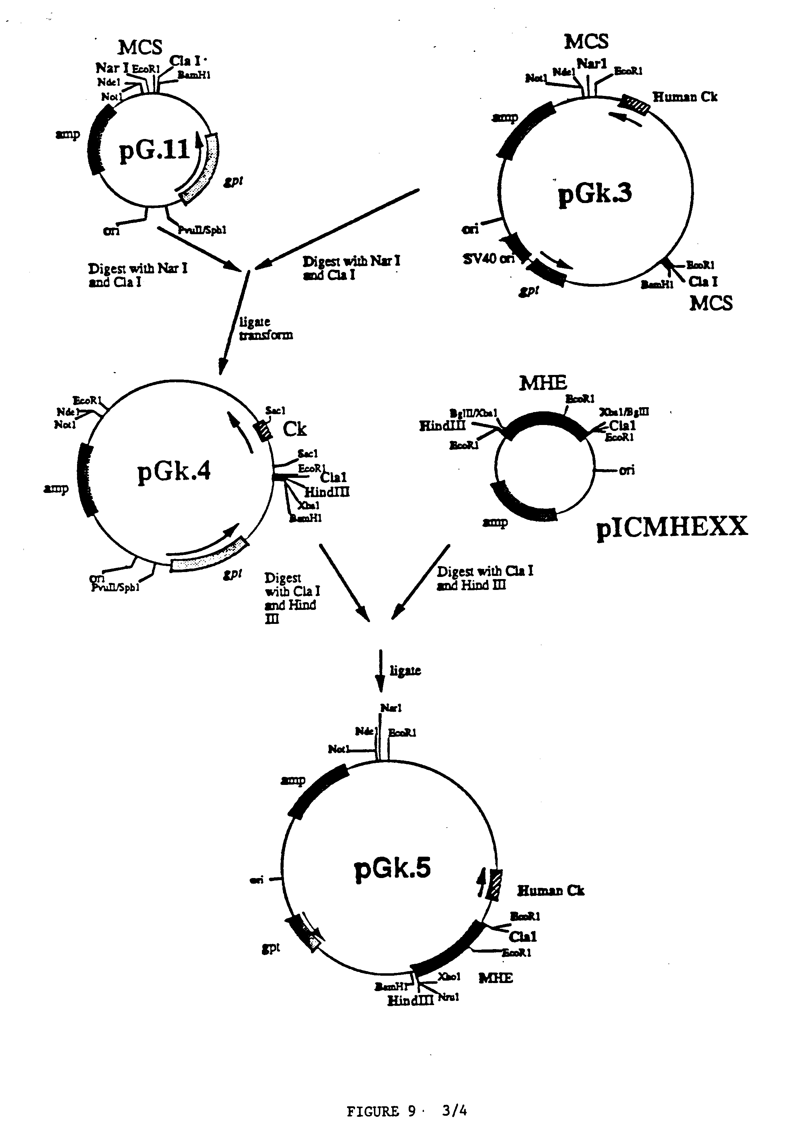

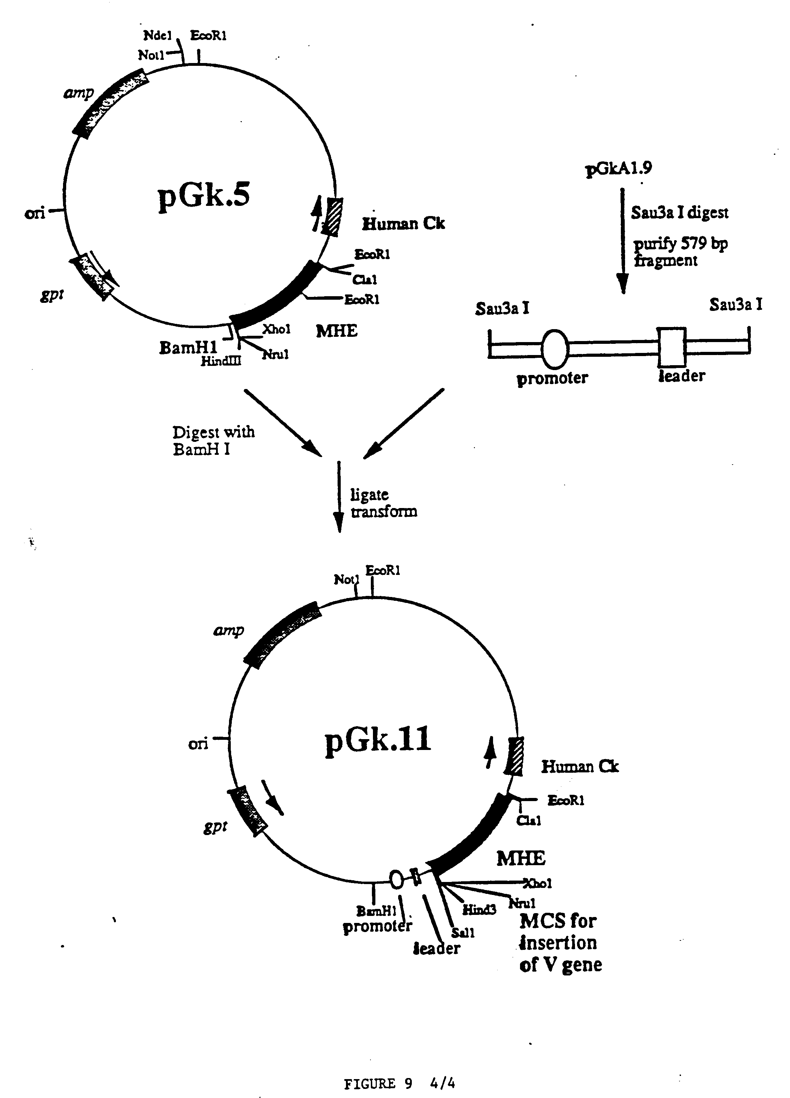

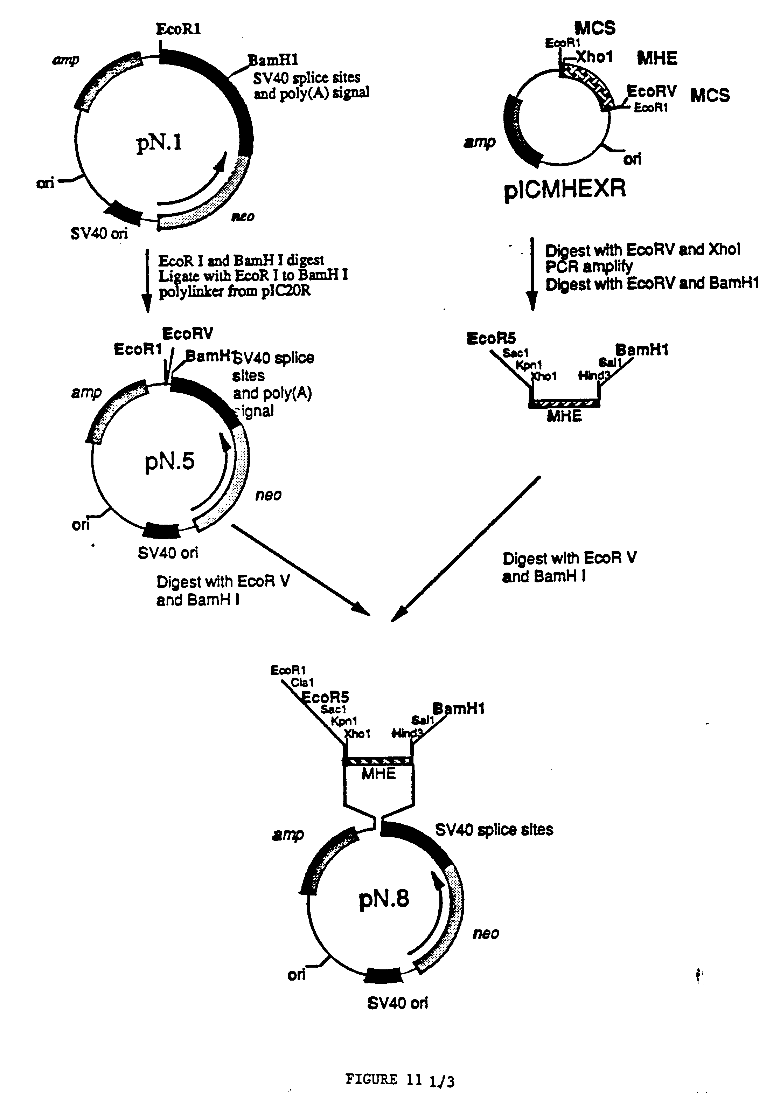

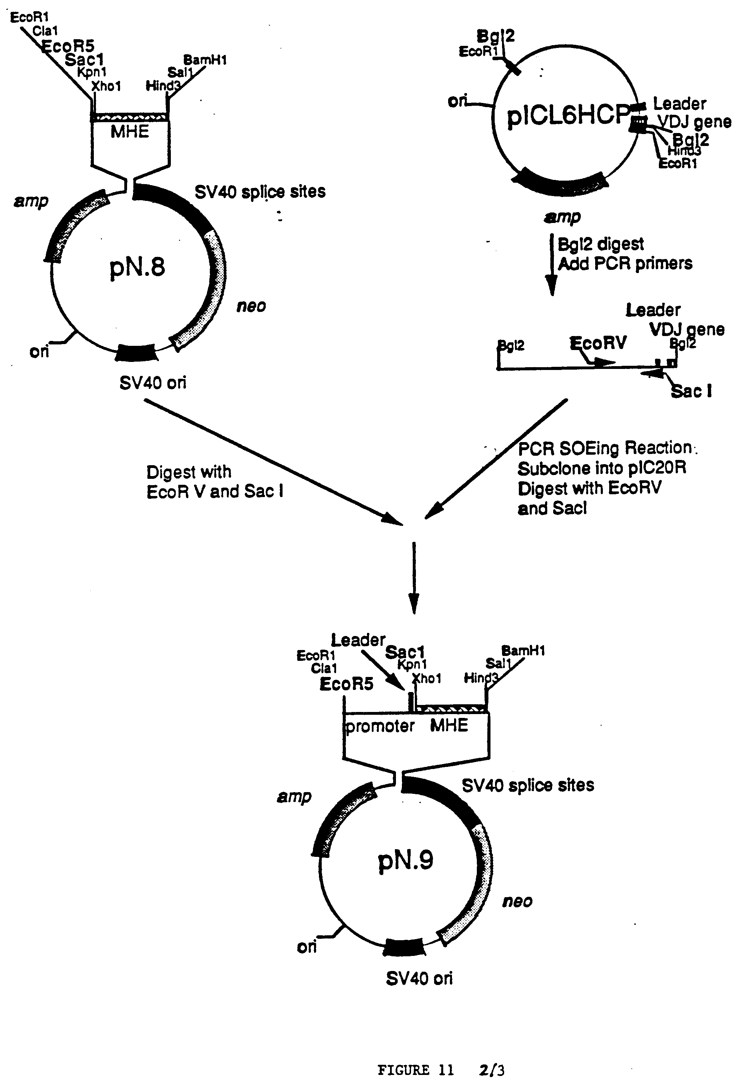

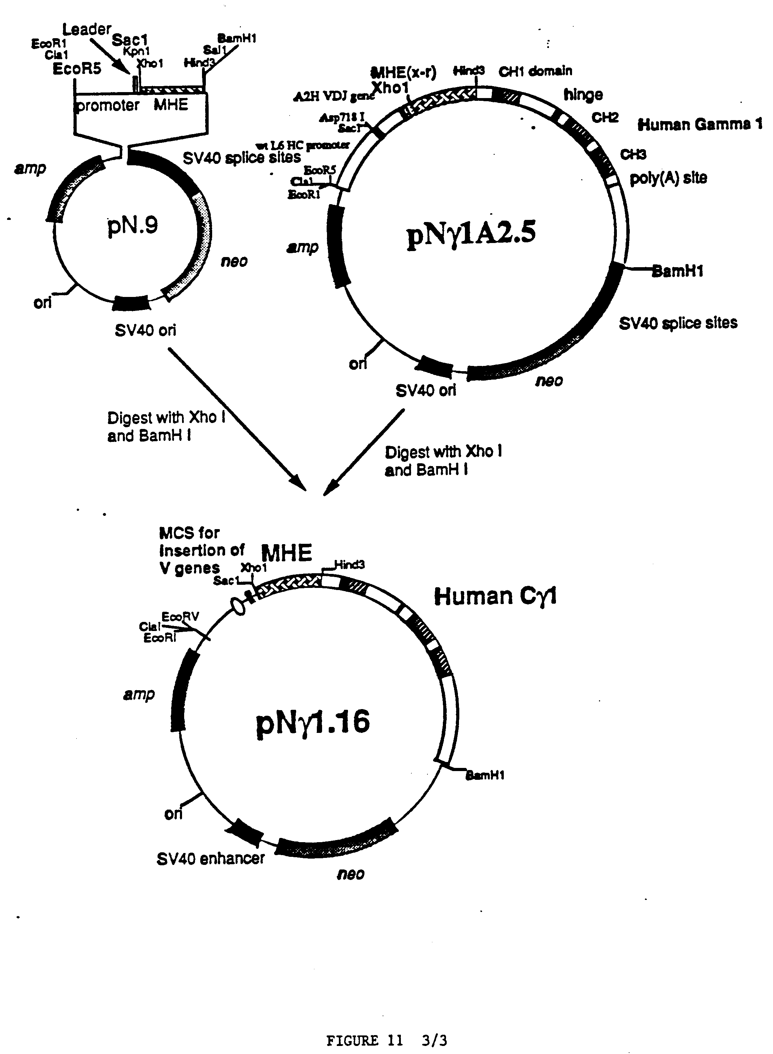

For each V gene, 2 PCR primers were synthesized. The amplification of genes by PCR, cloning into Puc18, as well as the double stranded DNA sequencing were all done as described above. The PCRed V genes were then cloned into the expression vectors PNγ 1.16 and PGk11 which have human constant regions (Fig. 9 and 11) before transfection into the mouse myeloma cell line, Ag8.653.

The VL and VH genes were inserted into pGk.11 and pNγ1.16, respectively, by amplifying the genes by PCR adding restriction site and intron sequences at the 5' and 3' ends.

The sense primers for both the H and L chains contained within their sequences the following in the 5' - 3' direction:

1. N6,
2, restriction site for cloning (EcoRI and SacI for the H chain; EcoRI and HindIII for the L chain; EcoRI is for cloning into pUC18, SacI and Hindm are for cloning into the expression vectors),
3. branch point lariat signal,
4. polypyrimidine tract,
5. splice acceptor site,
6. leader peptide 2,
7. the beginning of the FR1 of the V gene.

The reverse complement of the antisense primer contained:

1. 3' end of VDJ gene for the H chain and of the VJ gene for the L chain,
2. splice donor signal,
3. restriction site for cloning (SalI and XhoI for both H and L chains; SalI is for cloning into pUC 18, XhoI is for cloning into the expression vectors),
4. N6.

The sense primer for the heavy chain was:

5'$a_6$GAATTCGAGCTCTTTTTCTGATAACGTTGTCCTTCTGTTTCTTGCAGGT
GTCCAGTGTCAGGTCCAACTTCAGCAGCCTGGG3'

The anti-sense primer for the heavy chain was:

5'$A_6$GTCGACCTCGAGTGTGAGGACTCACCTGAGGAGACGGTGACTGAGGT
GCCT3'

The sense primer for the light chain was:

5'$a_6$GAATTCAAGCTTTCCTGACTACATGAGTGCATTTCTGTTTTATTTCCA
ATTTCAGATACCACCGGAGACATTGTGCTAACACAATCTCCA3'.

The anti-sense primer for the light chain was:

5'$A_6$GTCGACCTCGAGATCACTTACGTTTGATTTCCAGCTTGGTGCCTCCAC 3'

The amplification of genes by PCR, cloning into pUC18 and the double stranded DNA sequencing were done as described above. The PCRed V genes were the cloned into the expression vectors. For the heavy chain, the V gene was directionally cloned into the Sac I and Xho sites of pNγ1.16. For the light chain, the V gene was directionally cloned into the HindIII and Xho I sites of pGk.11.

Humanized VH and VL genes were constructed by oligos and is described in detail herein below. The insertion of humanized $V_H$ and $V_L$ genes into pNγ1.16 and pGk.11, respectively were done by the procedures described for the chimeric Ab. The sequences needed for cloning and expression were built in (or included in ) in oligo #1 and #10.

The presence of the chimeric 60.3 Abs were detected by ELISA. In this assay, 96 well plates were coated with goat anti-human IgG. The chimeric 60.3 in the sample which bound to the plates were detected by using horse radish peroxidase (HRPO) conjugated human anti-kappa IgG. Purified chimeric L6 (an unrelated anti-tumor antibody) was used as a standard. The culture supernatants from Ab producing clones were selected for binding in FACS binding assays.

FACS Binding Assays

HL60 is a human myelomonocytic cell line which expresses CD 18 on the cell surface.

HL60 target cells, grown in culture media (DMEM, 1% fetal calf serum and 2% L-Glutamine), were made $10^7$/ml in the culture containing 0.1% NaAzide at 4°C. $10^6$ cells were used per binding assay. Bovine IgG (Sigma, 20 ug/ml final concentration) was added for 10 min. at 4°C to mask the Fc receptors before performing the assays. Binding assays were done as direct binding, pre-incubation or competition assays.

1) Standard binding assays

Various amounts of murine, chimeric or humanized 60.3 were incubated for 45 minutes with HL60 cells, washed and then incubated with FITC-labeled goat anti-mouse (for murine 60.3) antibody (Tago, Inc.) at 1:50 dilution or FITC-labeled goat anti-human antibody (for chimeric and humanized 60.3.) The fluorescence intensity was then monitored.

2) Pre-incubation assays

Pre-incubation experiments were done with murine 60.3. For pre-incubation with either chimeric 60.3, or humanized 60.3, 1000 ng/ml of either c60.3 or h60.3 was added to the HL60 cells for 45 minutes to 1 hr at 4°C. Cells were then washed twice with 1 ml of ice cold NaAzide-containing culture media and centrifuged in a Beckman table top centrifuge (Model TJ-6) at 1000 rpm for 5 min. After decanting washes, cells were resuspended in various concentrations of FITC-conjugated murine 60.3 Abs and incubated on ice for 45 min. to 1 hr. with intermittent mixing at 10min intervals. Cell pellets were fixed in 300 ul of 1 % paraformaldehyde at 4°C before analysis of FACS machines.

3) Co-incubation assays

Co-incubation assays were carried out by incubating various concentrations of chimeric 60.3 or humanized 60.3 with a saturating concentration (1 ug) of FITC-conjugated murine 60.3 Abs. Both types of Abs were added to the HL60 cells simultaneously; thus eliminating one incubation step. Incubation, washing and centrifugation were all done as in the pre-incubation assays. The cells were also suspended in 1 % ice cold paraformaldehyde before analysis by FACS.

The mean channel values obtained from FACS analyses were translated into linear fluorescence equivalence (LFE)values. The LFE values were further used to calculate the fluorescence intensity levels (FIL) according to the following equation:

FIL = LFE of sample/LFE of negative control (no second antibody)

Electroporation of cells was carried out on a BioRad electroporator, set on capacitance of about 960 fu and .25 volts. A count of viable cells was taken before starting. Cells were at least 90% viable for use. Cells were also in the 4-6 x $10^5$ /ml range; if they are overgrown, they will not show high transfection efficiency. 1 x $10^7$ cells/electroporation group were removed and one group was used for a control electroporation and one for an "unzapped" control. Cells were centrifuged at 1000 rpm for 10 minutes. Supernatant was removed by vacuum with a sterile, unplugged pasteur pipet and the pellet was resuspended in as large a volume of PBS as the tube will allow and washed again.

The pellet was resuspended in 0.8 ml PBS per 1 x $10^7$ cells and 0.8 ml aliquot were added into labeled cuvettes. 10 ug each of DNA was added to cuvette. Incubate cuvette on ice for 10 minutes after mixing. The cuvettes were electroporated noting time factor reading and put on ice for 10 minutes, and then transferred into 19 mls of IMDM/10% FBS, using 1 ml of medium to wash them out of the cuvettes. Cells were at 37° C for 48 hours, and then plated at $10^4$, 3 x $10^3$, and 1 x $10^3$ cells per ml in IMDM with 10% FBS and fed for 2-3 weeks before screening.

Enzyme-linked immunosorption assays (ELISA) were carried out as is known in the art. In an illustrative embodiment of ELISA in the present invention was performed as follows. Plates were created by diluting goat anti-human IgG 1:10,000 with 0.05M carbonate buffer, pH9.6 and transferring 100 ul to each well of a 96 well microtiter plate . The plate was then incubated at 4°C for 12 to 16 hours. The plates were then rinsed 1 to 3x and specimen diluent was added. The plates were then incubated at room temperature for 1 hr and rinsed 3x. On separate plates, 30 ul of cultural supernatant was diluted to 300 ul with the specimen diluent and 50 ul was transferred to the previously coated plates. 50 ul of specimen diluent was added and maintained at room temp for 1 to 2 hours and rinsed 3x. HRPO- conjugated human anti kappa was diluted with conjugate diluent 1:5,000 and 100 ul was added per well to plates and incubated at 37°C for 30 minutes and washed 3x. A chromagen (1:300) with buffer substrate, pH 5.5 (room temperature) and 100ul was added to the plates. The plates were incubated at room temperature for 15 minutes, 100 ul of 3M $H_2S0_4$ was added and the plates were read at a wavelength of 450 nm and 630 nm.

Chimeric and humanized Mab 60.3 were analyzed using size exclusion HPLC (secHPLC), sodium dodecylsulfate-polyacrylamide gel electrophoresis (SDS-PAGE), and isoelectric focusing (IEF). Test samples were compared to a murine 60.3.

**secHPLC**

A TSK3000SW Spherogel 7.5 x 600 mm column manufactured by Toso Haas was used. The mobile phase was 0.05 M phosphate buffered saline and samples were eluted at 0.5 ml/minute for 60 minutes. The chromatograms show that the test lots have multiple contaminating peaks and a major peak eluting at about 27.5 minutes. The major peak observed with the reference murine 60.3 Mac chromatogram, eluted at about 26.7 minutes.

**SDS-PAGE**

SDS-PAGE was performed using a 4-20% gradient gel and bands were detected by Coomassie blue staining. All lots were compared to the murine reference lot. Samples were run both reduced with 2-mercaptoethanol and non-reduced. Non-reduced gels showed that the major band ran consistent with the reference standard. The reduced gel showed data consistent with heavy and light chain separation.

**IEF**

Isoelectric focusing was performed using precast gels with a pH range of 3-10. Both 2 ug and 5ug of sample were applied and run at 100 volts for 60 minutes, 200 volts for 60 minutes, and 500 volts for 30 minutes. Gels were stained with Coomassie Blue R-250. The chimeric 60.3 sample had no visible bands. This suggests that the material did not migrate into the gel. The humanized 60.3 sample had visible bands at the top of the gel in a region difficult to determine pI's. This data suggest that the chimeric and humanized lots of antibody have pI's greater than 8.0 as determined by the reference markers. This is in contrast to the murine mAb 60.3 which has a pI range of 6.8 - 7.5.

Having described this invention and embodiments thereof, the present invention is further illustrated by the following Examples which are not intended to limit the scope of the invention.

EXAMPLE 1: Comparative Model Building

The structural templates for comparative model building of murine 60.3 were determined. The Brookhaven Protein Data Bank was searched for the sequences with known structure which are most homologous to murine 60.3 heavy and light chains. The antiphosphocholine murine myeloma antibody, McPC603 (Satow et al. (1986) J. Mol. Biol. 190 : 593-604) was found to be the most homologous to the light chain, with 68% sequence identity/homology. The anti-p-azophenylarsonate murine monoclonal IgG2, R19.9 (Lascombe et al (1989) Proc. Natl. Acad. Sci. USA 86 : 607-611), was found to be the most homologous to the heavy chain, with 59% sequence identity/ homology. MCPC603 VL and R19.9 VH were combined in order to obtain the structural template for model building of murine and humanized 60.3.

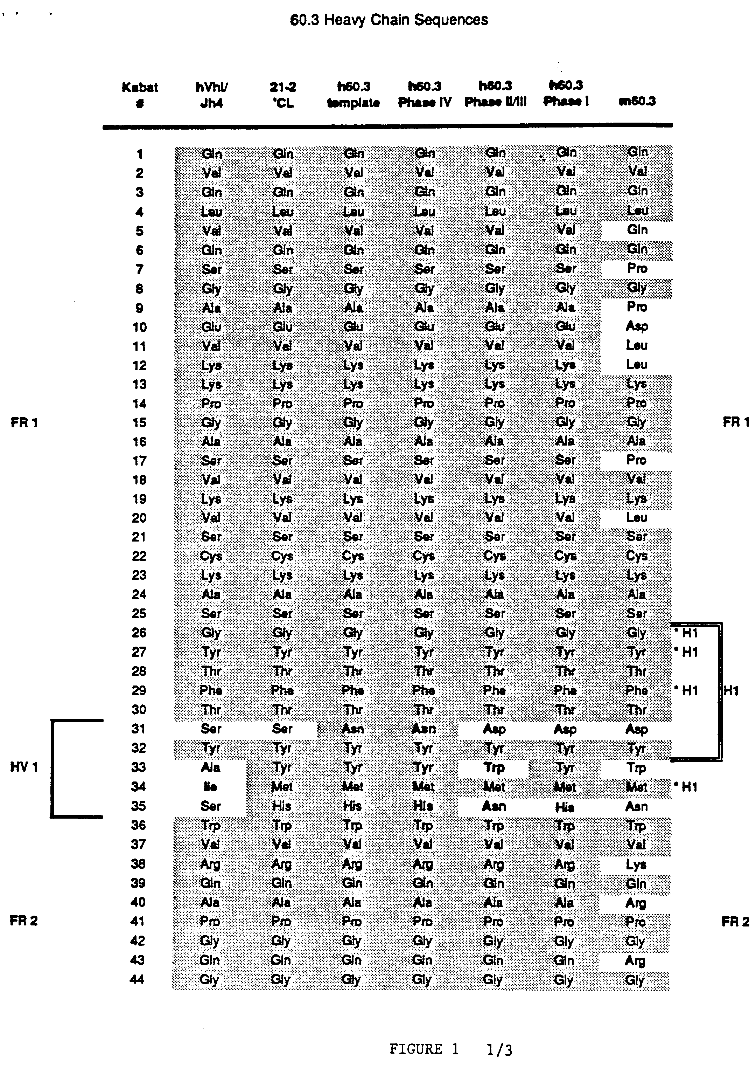

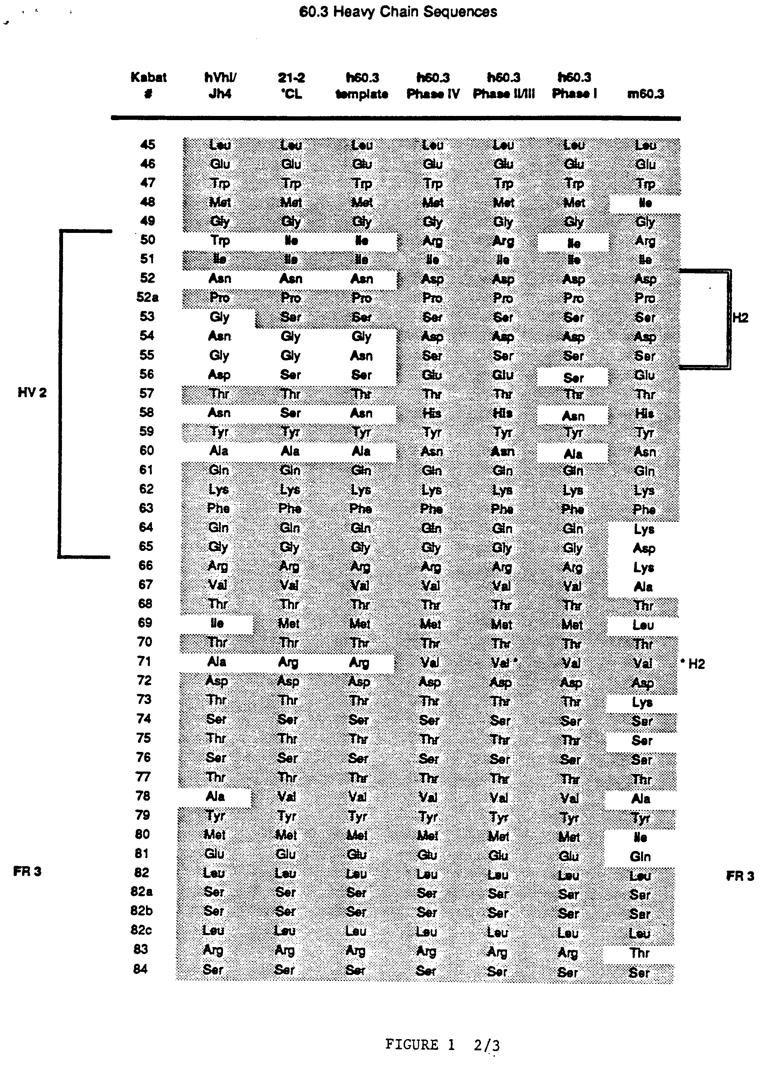

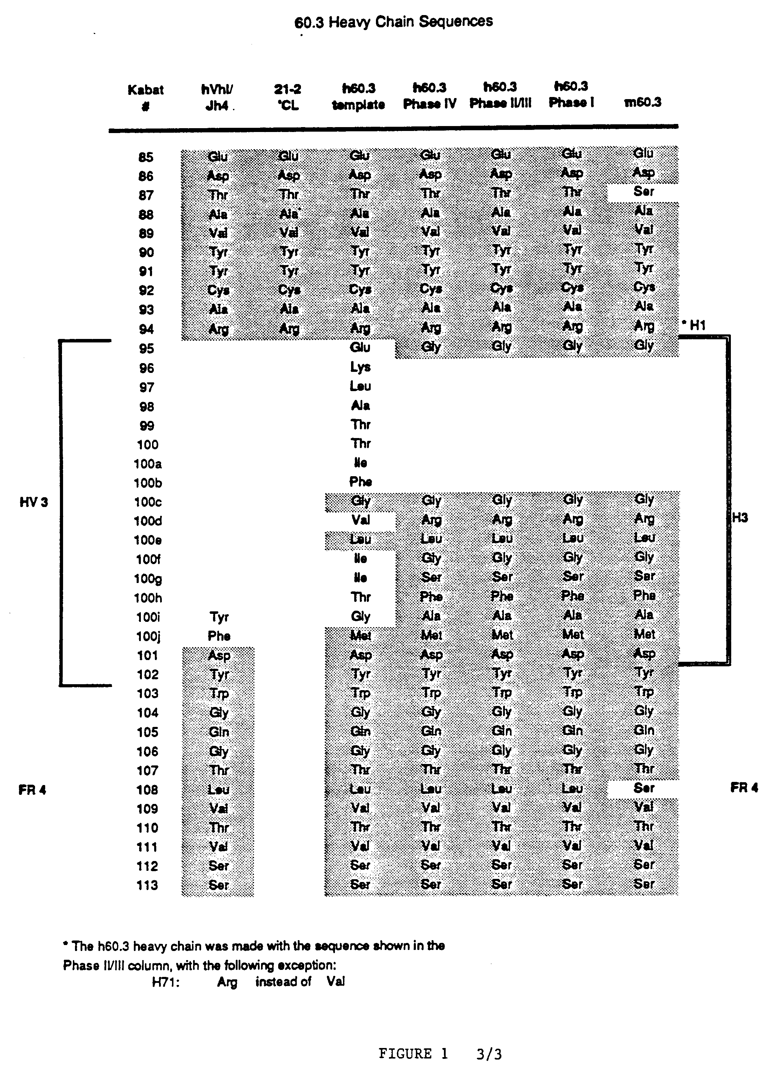

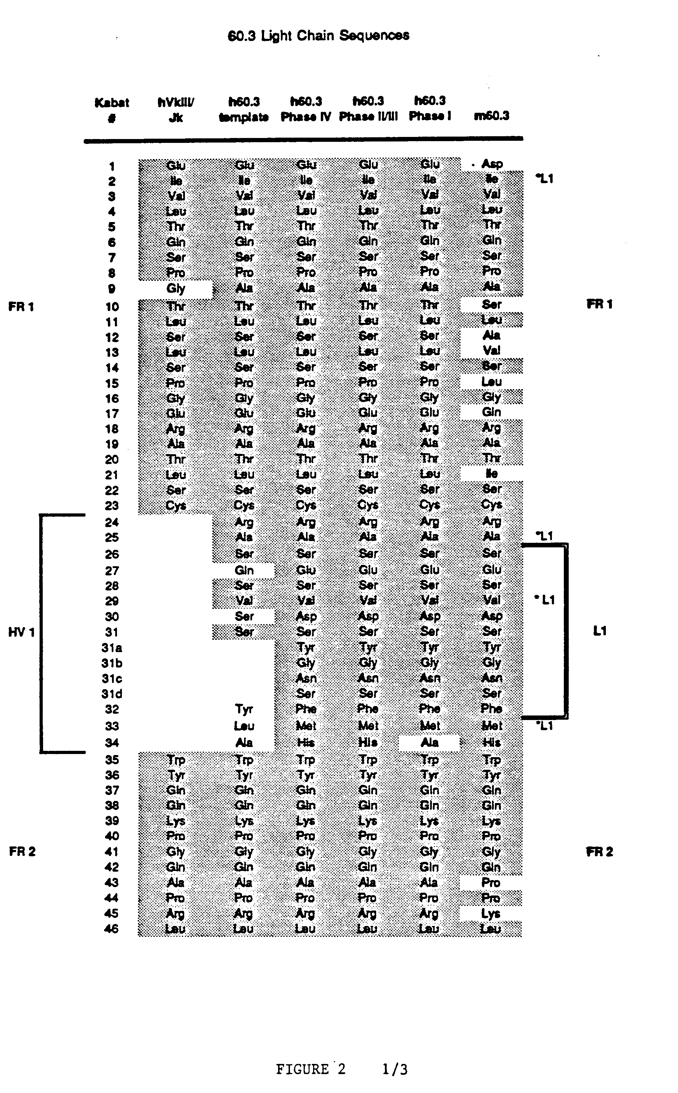

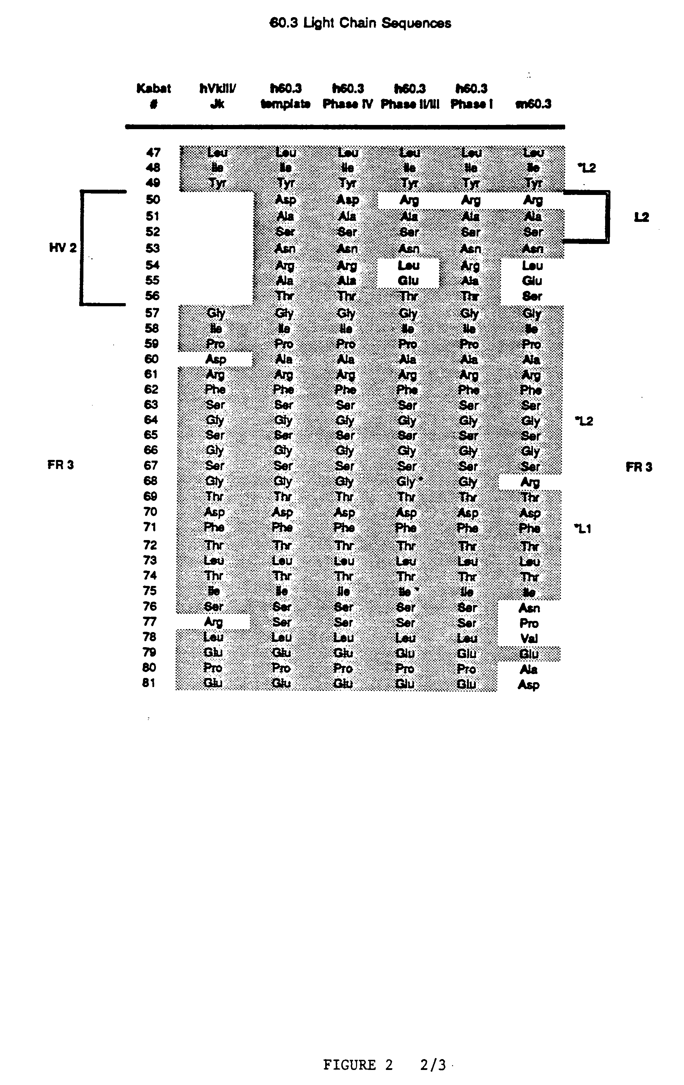

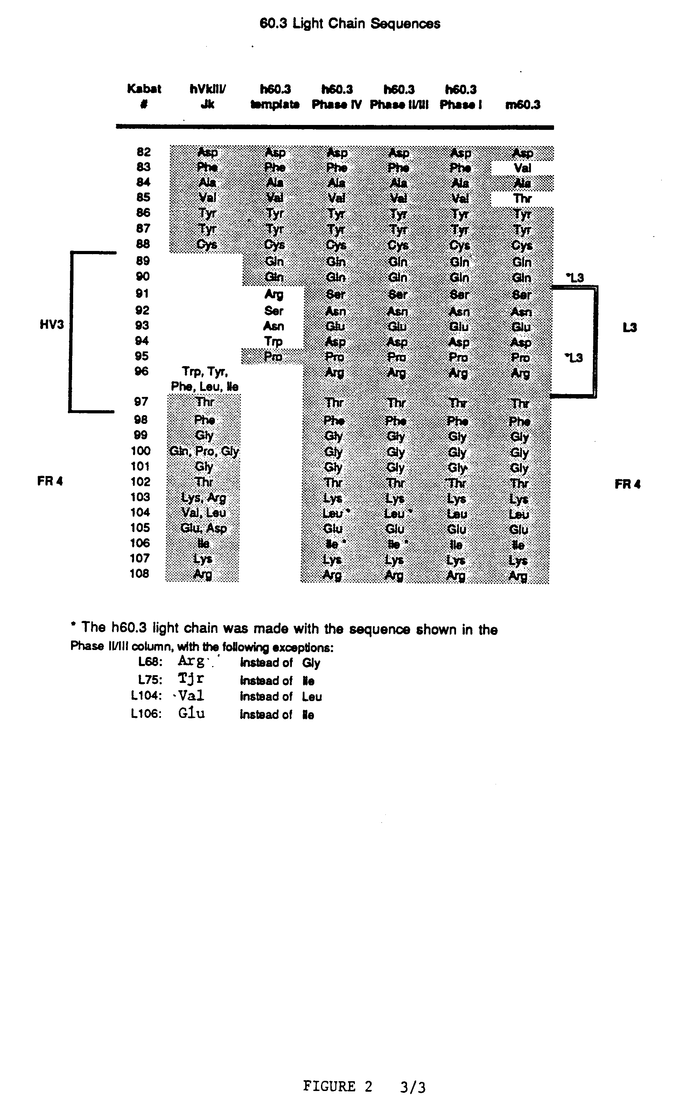

The CDR loops and known structural determinants of murine 60.3 were determined. Three CDR loops in 60.3 can be directly assigned to known canonical types (Chothia et al (1989) Nature 342 : 377-383). These are L2 (type 1), L3 (type 1) and H1 (type 1). The remaining CDR loops do not belong to known canonical structure types. The boundaries of these loops can be determined by aligning the 60.3 sequence with that of the structural model. These assignments are shown along with those of L2, L3 and H1 in Figs. 1 and 2. The framework residues which are crucial for the conformations of the CDR loops are also shown in Figs. 1 and 2 ( for example, * L1 indicates structural determinants for the L1 loop) and in Sequence I.D. Numbers 2, 4, 6, and 8.

A model of murine 60.3 was then built. Backbone loop templates for L2 and L3 were taken from McPC603, and H1 from R19.9. L1 was initially modeled by a two residue deletion of the L1 loop of McPC603. H2 was found to be similar to the corresponding loop region in R19.9 , which may represent a not yet classified canonical motif.

No loop closely related to H3 was found in the Brookhaven database. As a initial approximation for H3, an antibody non-CDR loop of the same size as 60.3 H3 (defined here as 96 to 102 in the Kabat numbering scheme or as H99 - H109 in the continuous sequence) was used. The backbone template for this loop was a 13 residue segment of the antibody NEW (Polijak RE. et al. (1974) Proc. Natl. Acad. Sci. USA 71 : 3340), beginning at residue L8. The loop was selected because it has the same length and showed a reasonable fit into the adjacent framework of H3. Energy minimization/conformation refinement of murine 60.3 resulted in: 1) residual rms derivatives of the energy function : O.63 kcal/mol A 2) Backbone rms deviations from the initial crystal coordinates: $V_L$: 0.86 Å ; $V_H$:1.17 Å.

The most homologous human variable region sequences were found. The human sequence most homologous to 60.3 $V_L$ is PIR Accession # A01900 (sequence identity/homology 66%). This is the Vg germline sequence described by Pech and Zachau (Pech, M. and Zachau, H.G. (1984) Nucleic Acids Res. 12 : 9229-9236). Vg belongs to human Vk subgroup IIIa.

The human sequence most homologous to 60.3 $V_H$ is PIR Accession # A32483 (homology 59%). This is the heavy chain from human monoclonal Ab MO30 (anti-HIV gp 120) (Larrick et al. (1989) BBRC 160 1250-1256). There are two germline sequences highly homologous (1 aa mismatch through FR3) to MO30 : 21-2 and 3-1 (Berman et al (1988) EMBO J. 7: 727-738). These sequences belong to human VH subgroup 1.

The structural template for murine 60.3 was confirmed to be suitable for the human template. The human template for the heavy chain (MO30) is 56% homologous to R19.9. The human template for the light chain (Vg) is 62% homologous to McPC603. These numbers are similar to the homology between murine 60.3 and the same structural templates. The CDR loops and the structural determinants for the human template are shown in Figs. 1 and 2. The CDR loops and known structural determinants were then grafted onto the human template (Jones et al. (1986) Nature 321 : 522-535). The results are shown in Figures 1 and 2 in the column marked Phase I h60.3. In these figures, the h60.3 sequences and all identical sequences from the other columns are shaded.

A Phase 1 model of humanized 60.3 was then built using the same model building protocol as for murine 60.3. The backbone CDR loop templates were the same as for m60.3. Energy minimization/conformational refinement of murine 60.3 and Phase I h60.3 resulted in:

residual rms derivatives of the energy function:

m60.3 : 0.63 kcal/molÅ
Phase 1h60.3 : 0.58 kcal/molÅ

Backbone rms deviations from the initial crystal coordinates:

m60.3 model $V_L$: 0.86 A; $V_H$: 1.17 Å
Phase I h60.3 $V_L$: 0.96 A; $V_H$: 1.15 Å

In Phase II, the murine and Phase I h60.3 models were compared and refined. A comparison of the models of murine and humanized 60.3 gave an rms deviation of 0.66 Å and 0.78 Å for $V_L$ and $V_H$, respectively. As a result of the comparison, it was postulated that certain residues which were still "human" were important for the conformation of the CDR loops. These were therefore changed to the murine residues, and are shown in bold in the column marked Phase II/III h60.3 in Figures 1 and 2. Sequence comparisons of m60.3 and Phase II 60.3 models (including the CDR loops) gave the following homologies:

Phase II 60.3 $V_L$ :
vs.murine 60.3 $V_L$ 82%
vs. human template $V_L$ 82%

Phase II h60.3 $V_H$
vs. murine 60.3 $V_H$ 78%
vs. human template $V_H$ 81%

Phase II h60.3 $F_V$
vs. murine 60.3 $F_V$ 80%
vs. human template $F_V$ 81%

In Phase III further improvements of the structural model of h60.3 were made. After further refinement of the murine and humanized models, the following parameters were obtained:

Final rms derivatives of the energy function:

murine 60.3 model: 0.55 kcal/mol Å

Phase III h60.3: 0.78 kcal/mol Å

Backbone rms deviations from the initial crystal coordinates:

murine $V_L$: 0.82 A, $V_H$: 1.09 A $F_V$: 0.98 Å

Phase III h60.3 $V_L$: 0.88 A, $V_H$: 1.07 A $F_V$: 1.00 Å

Finally, in Phase IV, the binding site features of the Phase III h60.3 model were analyzed. When comparing murine vs. humanized models at previous stages, the emphasis was more on the comparisons of the CDR - framework interactions in the murine antibody and the humanized contacts. The Phase IV model, where the L1 and H3 loops were remodeled using conformational search, allows a more detailed analysis of the CDR surface than the previous models.

Analysis of the model suggests that 60.3 is a distinct groove-type antibody and that certain CDR loops **(L2 and H1)** may not be involved in antigen binding. In the Phase IV model, the amino acids in these loops have been changed to the sequences from the human template.

**EXAMPLE 2**

CONSTRUCTION OF THE HUMANIZED 60.3 $V_H$ AND $V_L$ GENES

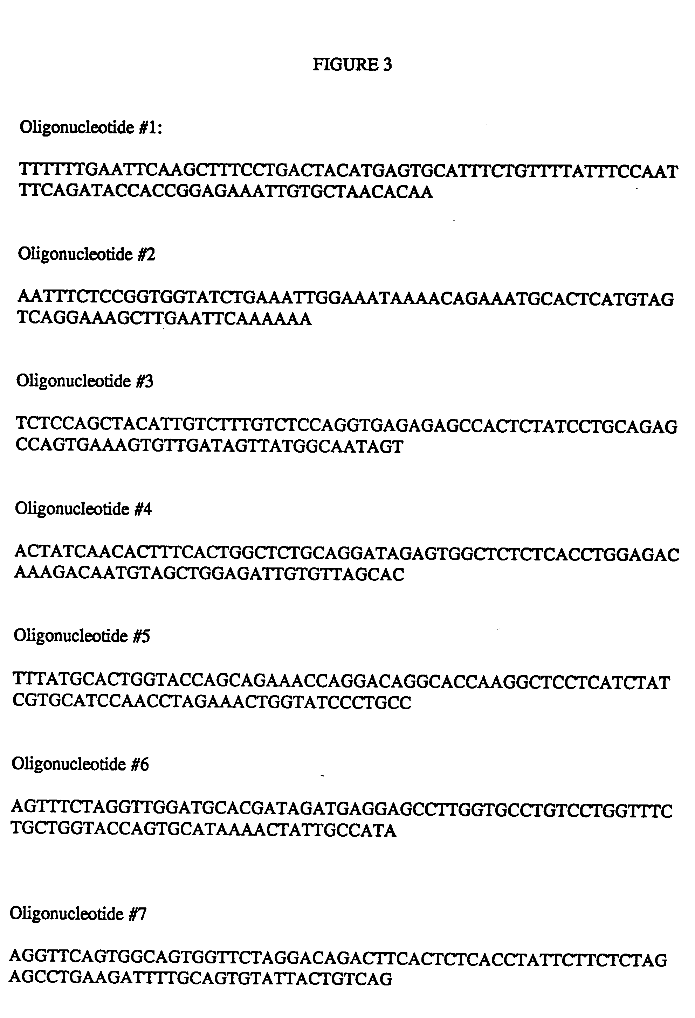

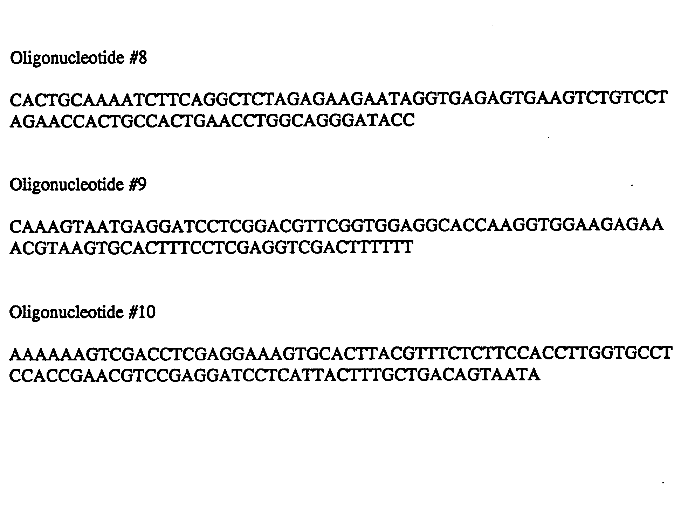

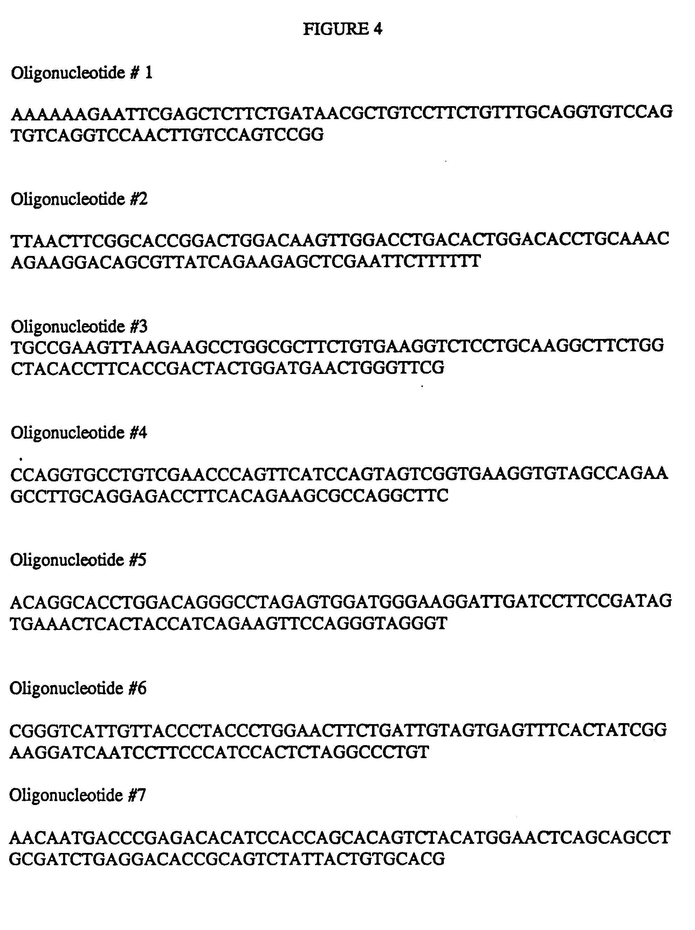

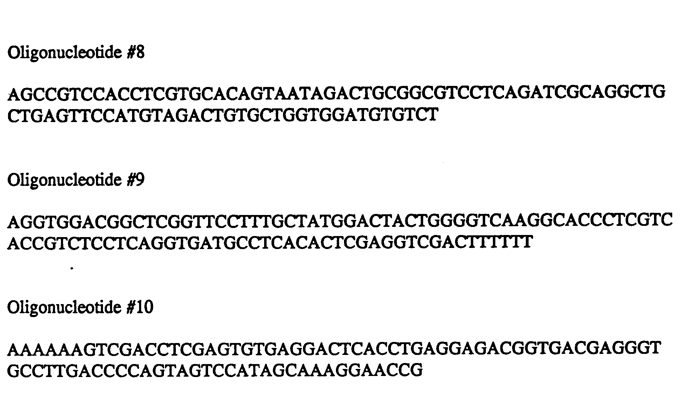

Construction of the humanized 60.3 V genes was done according to the modeling methodology described in Example 1 by piecing 5 pairs of complementary oligos together (see Figures 3 and 4 and Sequence I.D. Numbers 11 to 30) each oligo was about 90 to 100 nt in length, together they span the entire V regions, forming the $V_H$ and $V_L$. The step by step protocol used was as follows.

1. Five microfuge tubes were labeled and the following oligos (10 ng each, 10 ul) were added:
   - tube 1: oligo #2
   - tube 2: oligo #3 and #4
   - tube 3 : oligo #5 and #6
   - tube 4 : oligo #7 and #8
   - tube 5 : oligo #9
2. Tubes 2, 3 and 4 were heated to 100°C for 3 min. and then cooled slowly to room temp.
3. To all 5 tubes, 10x kinase buffer (40 m M Tris. Cl pH7.5, 10 mM $MgCl_2$, 10mMDTT, lug DNA, 0.5 mMATP, 50 ug/ml bovine serum algumin and 1 "weiss" unit T4 DNA ligase), 100 uM ATP (1 ul) and nucleotide kinase were added to phosphorylate the 5' ends of the oligos. One Weiss unit is equivalent to 60 cohesive-end units. Reactions proceeded at 37° for 1 hr.
4. The tubes were extracted with phenol/$CHCl_3$ and precipitated with ethanol.
5. Oligos #1 and #10 (10ng, 10 ul) were added to tubes 1 and 5, respectively. Tubes 1 and 5 were mixed and step 2 was repeated for tubes 1 and 5.
6. The contents from all 5 tubes were pooled into a single tube.
7. Ligate at 12° C, for 12 to 16 hours in a vol. of 25 ul.
8. Analyze 2 ul on 1.0 % agarose.
9. After successful ligation, restriction digest an aliquot with EcoRI and SalI for 45 min. at 37°C.
10. Apply onto 3.0% low melt agarose gel and cut out the correct sized band (approximately 0.4kb).
11. Ligate into Puc 18 (pre-digested with EcoRI and SalI).
12. Transform DH5α cells.
13. Select the potential positives based on Xgal/IPTG indicators.

14 Miniprep cultures were prepared and maintained for 12 to 16 hours.

15. Plasmid DNA was isolated from these cultures and the insert sizes were checked by cutting with EcoRI and SalI.
16. The plasmid DNA was sequenced for verification. Several of the clones had mutations such as single base deletions.
17. The synthetic H and L variable genes were cloned into appropriate expression vectors (pNG1.16 and pGk. 11, respectively.)
18. The potential positives were isolated after selection on ampicillin.
19. Steps 14 to 16 were repeated.
20. Transfection into mouse Ag8.653 myeloma cells was carried out, followed by selection with G418 (Raff, et al., (1991) J. Infect. Dis. 163 : 346-354).

21. Ig positives were then screened with ELISA (gamma, kappa capture). The DNA sequences of the murine 60.3 heavy and light chains are shown in Sequence I.D. numbers 5 and 7, respectively; and the DNA sequences for the humanized 60.3 H and L chains are shown in Sequence I.D. numbers 1 and 3, respectively.

**EXAMPLE 3**

BINDING ASSAYS OF 60.3

The binding activity of the humanized 60.3 antibody was measured by preincubation, competition and chemilluminescence assays.
HL60 is a human myelomonocytic cell line which expresses CD 18 on the cell surface.
HL60 target cells, grown in culture media (DMEM, 1% fetal calf serum and 2% L-Glutamine), were made $10^7$/ml in the culture media containing 0.1% NaAzide at 4°C. $10^6$ cells were used per binding assay. Bovine IgG (Sigma, 20 ug/ml final concentration) was added for 10 minutes at 4°C to mask the Fc receptors before performing the assays. Three types of binding assays were performed.

A) Standard curves

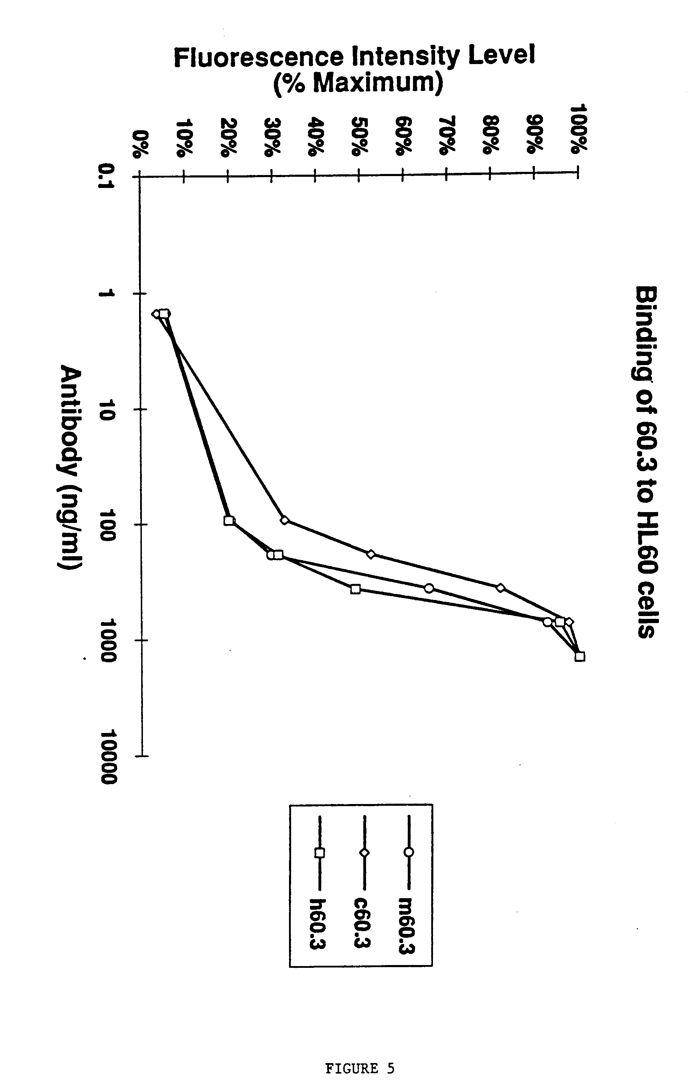

As illustrated in Figure 5, various amounts of murine, chimeric, and humanized 60.3 were incubated for 45 minutes with HL60 cells. The cells were washed and then incubated for 45 minutes with either -FITC conjugated goat anti mouse IgG (for m60.3) or FITC conjugated goat anti human IgG (for c60.3 and h60.3). Excess antibody was washed off and the cells were fixed with 1% paraformaldehyde and assayed by FACS. For each curve, the value obtained at 1500 ng/ml is taken as 100%. Data at other concentrations are plotted as % of this value. As can be seen in Figure 5, all three antibodies titrate over approximately the same range., indicating similar affinities (known to be about $10^9$ for m60.3).

B) Preincubation experiments

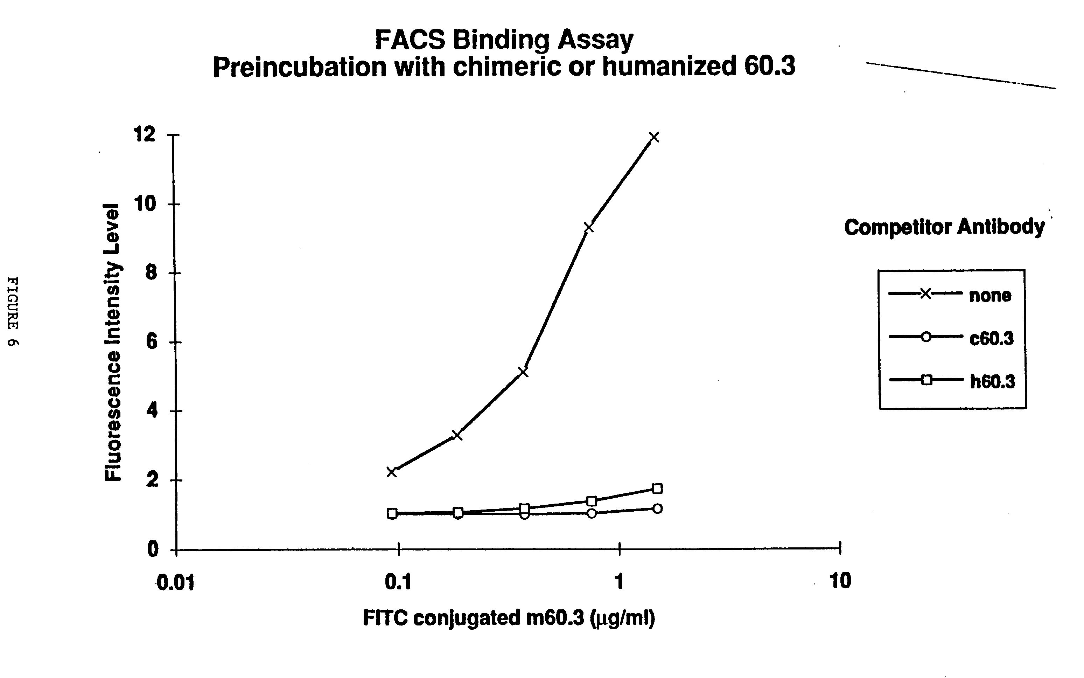

HL60 cells were preincubated for 45 minutes with 1 μg/ml of either c60.3 or h60.3. The indicated amount of FITC conjugated m60.3 (or no antibody) was then added and the cells were incubated for another 45 minutes. After washing, the cells were fixed with paraformaldehyde and assayed by FACS. As can be seen in Figure 6, both the chimeric and humanized antibodies were able to completely block the binding of FITC - m60.3 to HL60 cells (note FIL = 1 is equivalent to no binding).

C) Competition experiments

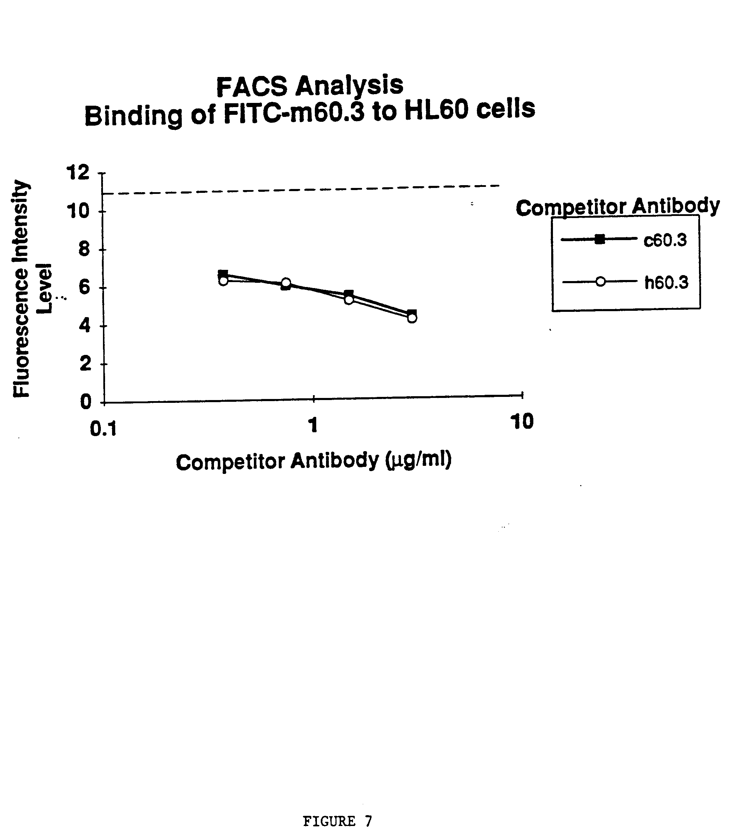

As illustrated in Figure 7, 1 μg of FITC conjugated m60.3 and the indicated amount of either c60.3 or h60.3 were coincubated with HL60 cells for 45 minutes. The cells were washed, fixed with paraformaldehyde and assayed by FACS. As can be seen in Figure 7, the chimeric and humanized antibody competed equivalently in this assay. The dashed line shows binding of 1 μg FITC - m60.3 in absence of competitor.

3) Chemilluminescence Assay

An assessment of activity of 60.3 by inhibition of Zymosan-induced, luminol-enhanced chemiluminescence of PMN was carried out. The material and compositions and procedures used were as follows.

<u>*Prepararion of Components:*</u>

**1. GGVB**

1.1. Materials

a)5x Veronal: Dissolve 41.2g NaCl and 5.095g 5,5-diethylbarbiturate (Paragon B-2 buffer) in 700 ml deopmozed (diH20). Adjust pH to 7.35 $\pm$ 0.05 with 1N HCl. Bring volume to 1 liter with diH20. Filter sterilize and store at 4°C. Stability but is at least 2 months.
b) Stock metals: mix equal volumes of 2M $MgCl_2$ (40.66g/100ml) and 0.3M $CaCl_2$ (4.4g/100ml). Filter sterilize and store at 4°C. Stability is at least 6 months.

c) gelatin
d) dextrose

1.2. Procedure

For 300 ml GGVB:

Add 0.3g gelatin and 0.3g dextrose to 240 ml diH20. Heat with mixing until just dissolved. Let cool to below 37C.

Add 60 ml 5x Veronal and 0.3 ml stock metals. Filter sterilize and store at 4C.

This is made up fresh for each day's assays.

**2.2. Zymosan**

2.1. Materials:

a) Zymosan A, SigmaZ-4250
b) diH20
c) 1 x PBS

2.2. Procedure:

Suspend zymosan to 25 mg/ml (1.5g/60ml) in $diH_20$. Heat in glass in a water bath at 100C for 60 min.

Transfer to 50 ml polypropylene centrifuge tubes. Centrifuge (8500 rpm in the TJ-6, 10 min, RT) and wash twice with 1 x PBS. Resuspend to 50 mg/ml (1.5g/30ml) in 1 x PBS. Store at 4C. Stability is at least 1-2 months at 4C.

**3. Complement (adsorbed human serum)**

3.1. Material:

a) Freshly drawn human blood, without anticoagulants. Type O may be best, or use the same donor that supplies PMN. Transfer blood immediately after drawing into sterile 50ml glass centrifuge tubes (available from blood lab).
b) 0.1 M EDTA, pH 7.35. Dissolve 3.72g $NA_2H_2$ EDTA in 80 ml di H20, with mixing. Adjust pH to 7.35 ± 0.05 with freshly prepared 2N NaOH. Bring to 100 ml volume with diH20.
c) Zymosan prep (see 2.1)

3.2. Procedure:

Allow blood to clot at room temperature for 1 hour. Rim the tube with a glass pipet to contract the clot. Centrifuge (8500 RPM in TJ-6, 20 min., RT) and carefully remove serum to a sterile polypropylene tube. Repeat centrifugation if necessary.

Add 0.1 M EDTA to serum to 10% mg/ml zymosan needed to adsorb serum at 0.2 mg zymosan/ml of serum. Add this volume of zymosan to each of four centrifuge tubes. Centrifuge zymosan tubes (8500 rpm in the TJ-6, 10 min,. RT) and remove supernatant. Keep the tubes on ice.

Resuspend the zymosan pellet of one tube with a small amount of serum. Add the reaminder of the serum and mix by inversion.

Incubate the serum/zymosan mixture on a rocker or rotator, 30 min, 4C.

Centrifuge as above. Repeat the adsorption with the other three tubes.

After the final centrifugation, filter the serum through a 0.45um syringe filter Aliquot into microfuge tubes or equivalent and store at -70C. The adsorbed serum has an unknown stability but it probably good for at least six months.

**4 Human PMN**

4.1. Materials

a) Freshly drawn heparinized blood.

b) Ficoll-Hypaque Mono-Poly Resolving Medium (MPRM), Flow Labs no. 16-980- 49
c) 1 x PBS
d) 3% HoAc in diH20
e) Serum-free, phenol red-free RPMI with 0.5% gelatin (RPMI-gel): Add gelatin (1.25g/250 ml) to serum-free, phenol red-free RPMI and heat with stirring until gelatin is just dissolved. Filter sterilize (0.45 um filter) and store in 50ml aliquots at 4C. Stability is least 2 weeks.

4.2. Procedure

Aliquot 4 ml MPRM into 15 ml polypropylene centrifuge tubes.

Overlay gently with 5 ml fresh blood.

Centrifuge (30 min, 2000 rpm in TJ-6, RT). If RBC are not completely pelleted, centrifuge an additional 10 minutes. If no RBC sedimentation has occurred, additional centrifugation will not work and another donor must be found.

Aspirate top (plasma) layer and first layer of cells (monocytes).

With a Pasteur pipet, carefully remove the second cell layer (PMN) to a 50ml centrifuge tube. Repeat with all tubes.

Add RT at room temperature, 1 x PBS to the PMN tube to a final volume of 50 ml and mix gently.

Remove 50ul and dilute appropriately in 3% HoAc (usually 1:20 is appropriate for a prep of 30ml whole blood) and count PMN in a hemocytometer. Calculate total PMN present in the 50ml tube.

Centrifuge PMN (10 min, 2000 rpm in TJ-6, RT) and resuspend to desired density (normally 2 x $10^6$ml) in RPMI-gel. Store at RT, swirling gently to resuspend occasionally (once or twice an hour). Viability should be > 90% after 6 hours; if used after 6-8 hours, check viability by trypan blue exclusion prior to use.

**5 Luminol**

5.1. Materials

a) Luminol (5 amino-2,3 dihydro - 1,4 - phthalazinedione), Sigma no. A-8511.
FW=172.2
b) DMSO, chromatography grade

5.2. Procedure

Dissolve luminol to $10^{-2}$M in DMSO (17.7 mg/10ml). Store at 4C in the dark (wrap tube in foil). Stability is more than 1 month; make fresh 3-4 weeks.

**6. Antibody**

6.1. Materials and Procedure

Dilute antibody in GGVB to appropriate concentrations.

**Assay Design**

Test volumes per tube are normally 1 ml, composed of the following.
- 100 ul antibody (sensitivity to concentration is unknown, previous work has been with 5 ug/ml preps)
- 100 ul PMN (normal concentration at 2 x $10^6$/ml)
- mix by swirling gently and incubate 30 min, at room temperature (RT) (cover all tubes with one sheet of Parafilm)
- 100 ul Zymosan (high concentrations, around 50mg/ml,seem to work best)
- just before loading luminometer, add:
- 600 ul luminol (diluted in GGVB , normal concentrations are $10^{-4}$ or 5 x $10^{-5}$M)
- 100 ul complement (low concentrations, on the order of 1-2%, seem to work better)

**Staging of Assay**

Set up CL tubes (Clinicon 2174-089, available through LKB). Keep in the dark (in a drawer) to prevent spontaneous luminescence from absorbed fluorescent light.
Thaw complement at RT and hold on ice, just prior to assay complement may be unstable (may lost 50% of activity in 6-8 hours) at 4C.
Add antibody, PMN, and zymosan as described above to CL tubes.
Take tubes to luminometer. Program assay parameter into controlling computer.
Prepare final dilution of Luminol (and keep wrapped in foil) just before adding to CL tubes.
Prepare final dilution of complement in GGVB.
Add luminol and complement to CL tubes as described above and load into luminometer. Start the program immediately. Peak luminescence is reached 4-5 minutes after adding complement.
Throughout assay setup and during the first rotation of the CL tubes in the luminometer, apply anti-static charge with the anti-static gun to prevent CL tubes hanging up in the luminometer.

**Results**

The above procedure was utilized to measure the ability of murine, chimeric, and humanized 60.3 to inhibit CR3(CDIIb/CD18) medicated up take of opsonized zymosan by neutrophils. In the absence of 60.3. phagocytic uptake of opsonized zymosan results in an increase in hexase monophosphate shunt activity that is measured as light output by luminol-enhanced chemiluminescence.

% inhibition of chemiluminescence signal = 100 x ( 1 - signal of sample/signal of negative control antibody)

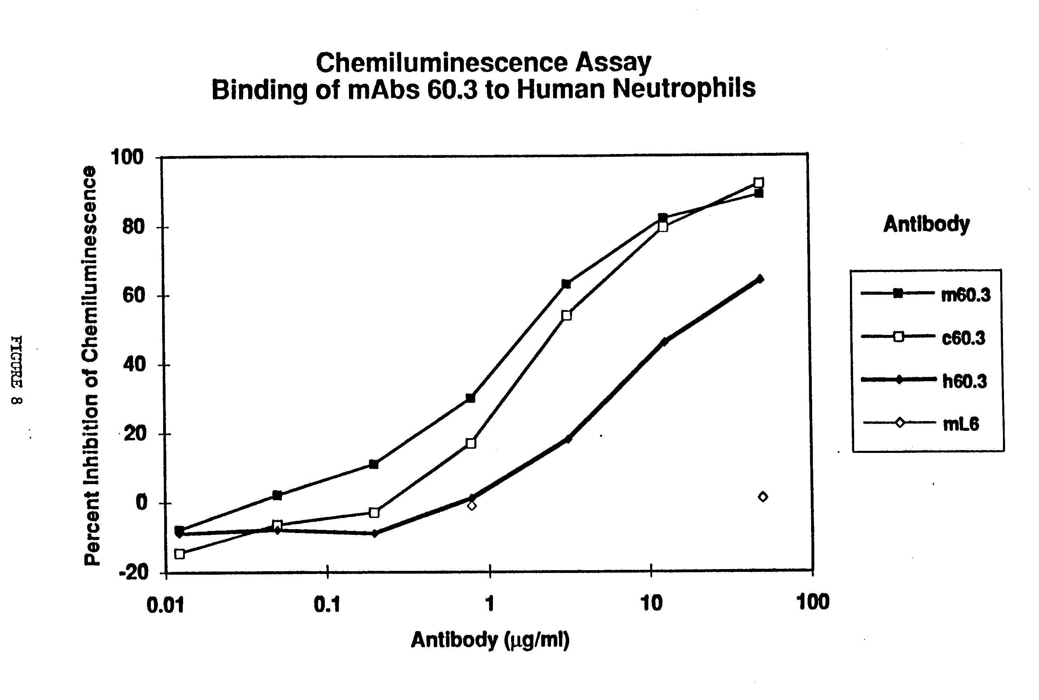

The results shown in Figure 8 illustrate that all of the 60.3 antibodies were reactive in this assay, while an irrelevant antibody (murine L6 anti-tumor antibody) showed no reactivity.

EXAMPLE 4

CONSTRUCTION OF pGK.11

In order to express either chimeric or humanized light chains, cassette vectors were constructed capable of expressing variable region genes, synthesized using PCR (polymerase chain reaction). In addition to sequences found on pSV2-gpt, these vectors contain an Ig promoter and leader, the 5' portion of an intron, a MCS for insertion of the variable region gene, the mouse heavy chain enhancer (MHE), the 3' portion of an intron and the human Ck gene. The variable region was PCRed so that in addition to the coding region it contained flanking intron sequences.

The cassette for the expression of the light chain was constructed as follows (and is illustrated in Figure 9):

1. The 121 bp Hind III to Bgl II fragment of pSV2-gpt (Mulligan and Berg (1980) Science <u>209</u>: 1422) was deleted from the 5' end of the Ecogpt by restriction with Hind III and Bgl II, filling in with Klenow polymerase, and religation. The product, pG.2, was detected by the absence of Hind III and Bgl II sites and linearization with EcoR I.
2. The pBR322 part of pG.2 (EcoR I-Pvu II fragment) was replaced with the analogous portion of pUC 18 (Pvu II - Pvu II fragment) to form pG.3 This product was screened for by digestion with Pst I, which linearizes pG.3 giving a 5.05 kb fragment.
3. pG.3 was made into pG.5 by the replacement of the 750 bp EcoR I to BamH I fragment with the 64 bp EcoR I to BamH I multiple cloning site (MCS) from pIC20R (Marsh et al. 1984). pG.5 was screened for the presence of an Xho I site (part of the MCS).
4. A Not I site was inserted in the Nde I site of pG.5 to form pG.12. Oligonucleotide linkers (Sequence I.D. numbers 33 and 34) were used for this purpose. pG.12 was screened for the presence of a Not I site.
5. A 2.75 kb EcoR I fragment containing the human Ck gene was inserted into the EcoR I site of the MCS of pG.12 to form pGk.3. When the Ck fragment was in the correct orientation, Sac I digestion produced 0.126, 0.509 and 6.7 kb fragments (vs. 0.509, 2.1 and 4.7 kb fragments in the wrong orientation).
6. A 140 bp portion of the SV140 enhancer was removed from pG.3 by restriction with Sph I, destroying the overhang with the exonuclease activity in Klenow polymerase, followed by digestion with Pvu II and blunt end ligation. The product, pG.9, was screened for the loss of Pvu II, Nsi I and Sph I sites.
7. A Not I site was inserted in the Nde I site of pG.9 to form pG.10. Oligonucleotide linkers, described in #4 above, were used for this purpose. pG.10 was screened for the presence of a Not I site.
8. The 195 bp Not I to BamH I fragment from pG.12 was inserted into the Not I to BamH I site of pG.10 to form **pG.11**. This served to place an 879bp fragment from pG.10 with a 195 base pair fragment containing a MCS. pG.11 was screened for the presence of Xho I site in the MCS region.

9. The 3kb Nar I to Cla I fragment from pGk.3 was directionally subcloned into the same sites of pG.11 to form pGk.4.

10. A 1kb fragment containing the mouse heavy chain enhancer was transferred from pICMHEXX to pGk.4 as a Cla I to Hind III fragment, thus forming pGk.5. EcoR I digestion of pGk.5. produced 0.3, 2.75 and 5.1 kb fragments. pICMHEXX was made by the insertion of the 1kb Xba I fragment (filled in with Klenow polymerase) from RBL 216 (Lang et al (1982) Nucl. Acid Res. 10: 611-620) into the filled in Bgl II site of pIC 19 R (Marsh et al (1984) Gene 32: 481-486).

11. A 579 bp Sau3a I fragment containing the 4B9 promoter pGkA1.9 (Raff et al, 1991) was inserted into the BamH I site (in the MCS) of pGk.5 to form pGk.11. The resulting plasmids were screened for the correct orientation of the insert : BamH I plus Asp718 I digestion gave a 1.2 kb fragment in the correct orientation vs. a 2.2 kb fragment in the wrong orientation. Also, BamH I plus Hind III digestion should give a 0.58 kb fragment.

The sequence of specific regions is contained in the following segments. The sequence is given in clockwise orientation beginning at the EcoR I site at 0° and is illustrated in Figure 10 and Sequence I.D. 10.

The ampicillin resistance gene is bp 7383 to 8241

The ecogpt gene is bp 5651 to 6107

The mouse heavy chain enhancer is bp 2770 to 3788

The Sau3a I fragment containing the Alk promoter and leader is bp 3827 to 4393, with the leader peptide encoded by bp 3951 to 3999.

The EcoR I fragment containing the human Ck gene is bp 6 to 2756, with the Ck region itself encoded by bp 2113 to 2435.

EXAMPLE 5

Construction of pNγ1.16

In order to express either chimeric or humanized heavy chains, cassette vectors were constructed capable of expressing variable region genes synthesized using PCR (polymerase chain reaction). In addition to sequences found on pSV2-neo, these vectors contain an Ig promoter and leader, the 5' portion of an intron, a MCS for insertion of the variable region gene, the moue heavy chain enhancer (MHE), the 3' portion of an intron and the human C γ1 gene. The variable region gene is PCRed so that in addition to the coding region it contains flanking intron sequences.

The cassette for the expression of the heavy chain was constructed as follows (as illustrated in Figure 11)

1. The Hind III site in pSV2-neo was removed by digestion with Hind III, fill in with Klenow polymerase and religation. The product, **pN.1** was screened for the absence of the Hind III site.

2. The 750 bp EcoR I to BamH I fragment from pN.1 was replaced by a 64 bp EcoR I to BamH I multiple cloning site (MCS) from pIC20R to form **pN.5**

3. A PCR region was done on p1CMHEXX in a manner that primers were chosen to delete the EcoR I site while generating a new MCS region. This resulted in a product consisting of: a) recognition sequences for EcoR V, Asp 718 I, Sac I and Xho I; b) a 695 bp of the mouse heavy chain enhancer from the 5'Xba I site to the EcoR I site; and c) recognition sequences for Hind III, Sal I and BamH I. This 723 bp PCR product was cloned into p1C20R to form **pMHE.per.**

4. The 723 EcoR V - BamH I fragment from pMHE.per was subcloned into the same sites in the MCS region of pN.5. This removed the previous MCS, while inserting the one associated with the MHE. The product, **pN.8**, was screened for the presence of 0.7 and 5.0 kb Xho I - Hind III fragments and for linearization to 5.7 kb with EcoR I.

5. Two PCR SOEing (Horton et al, (1990) Biotech. 8 : 528-535) reactions were used to create several mutations in the L6 heavy chain promoter. Outer primers had enzyme sites EcoR V and SacI for subcloning into pIC20R to form **pMUTL6HCP**. The sequence of this insert is shown as bp 7793-8495 in Fig.12 and in Sequence I.D. number 9.

6. The 703 bp EcoR V to SacI fragment from pMUTL6HCP was inserted into the same sites of pN.8 to form **pN.9**.

7. A 3.5 kb Xho I - BamH I fragment from pNγ1A2.5, containing the PCRed MHEXR (Sequence I.D. Number 31) plus the 2.8 kb Hindm - BamH I fragment encoding the human γ1 gene (Sequence I.D. Number 32) was inserted into the same sites of pN.9 to form pNγ1.16. The sequence of this insert is shown as bp 2-2799 in Fig. 12 and in Sequence I.D. Number 9.

The foregoing description and the Examples are intended as illustrative and are not to be taken as limiting. Still other variations within the spirit and scope of this invention are possible and will readily present themselves to those skilled in the art.

SEQUENCE LISTING

(1) GENERAL INFORMATION:

(i) APPLICANT:
(A) NAME: Bristol-Myers Squibb Company
(B) STREET: 345 Park Avenue
(C) CITY: New York
(D) STATE OR PROVINCE: New York
(E) COUNTRY: USA
(F) POSTAL CODE: 10154
(G) TELEPHONE: 206-728-4800
(H) FAX: 206-727-3601

(ii) TITLE OF THE INVENTION: HUMAMIZED MONOCLONAL ANTIBODIES

(iii) NUMBER OF SEQUENCES 34

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(v) CURRENT APPLICATION DATA:
(A) APPLICATION NUMBER: EP93401328.5
(B) FILING DATE: 24-MAY-1993
(C) CLASSIFICATION:

(vi) PRIOR APPLICATION DATA:
(A) APPLICATION NUMBER: US07/888233
(B) FILING DATE: 26-MAY-1992
(C) CLASSIFICATION:

(2) INFORMATION FOR SEQ ID NO:1:

(i) SEQUENCE CHARACTERISTICS:
(A) LENGTH: 361 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:1:

```
CAGGTCCAAC TTGTCCAGTC CGGTGCCGAA GTTAAGAAGC CTGGCGCTTC TGTGAAGGTC      60

TCCTGCAAGG CTTCTGGCTA CACCTTCACC GACTACTGGA TGAACTGGGT TCGACAGGCA     120

CCTGGACAGG GCCTAGAGTG GATGGGAAGG ATTGATCCTT CCGATAGTGA AACTCACTAC     180

AATCAGAAGT TCCAGGGTAG GGTAACAATG ACCCGAGACA CATCCACCAG CACAGTCTAC     240

ATGGAACTCA GCAGCCTGCG ATCTGAGGAC ACCGCAGTCT ATTACTGTGC ACGAGGTGGA     300

CGGCTCGGTT CCTTTGCTAT GGACTACTGG GGTCAAGGCA CCCTCGTCAC CGTCTCCTCA     360
```

G 361

(2) INFORMATION FOR SEQ ID NO:2:

(i) SEQUENCE CHARACTERISTICS:
(A) LENGTH: 120 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:2:

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Trp Met Asn Trp Val Arg Gln Ala Pro Gly Gln Gly Leu Glu Trp Met
        35                  40                  45

Gly Arg Ile Asp Pro Ser Asp Ser Glu Thr His Tyr Asn Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Gly Arg Leu Gly Ser Phe Ala Met Asp Tyr Trp Gly Gln
            100                 105                 110

Gly Thr Leu Val Thr Val Ser Ser
        115                 120
```

(2) INFORMATION FOR SEQ ID NO:3:

(i) SEQUENCE CHARACTERISTICS:
(A) LENGTH: 334 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:3:

```
GAAATTGTGC TAACACAATC TCCAGCTACA TTGTCTTTGT CTCCAGGTGA GAGAGCCACT     60

CTATCCTGCA GAGCCAGTGA AAGTGTTGAT AGTTATGGCA ATAGTTTTAT GCACTGGTAC    120
```

CAGCAGAAAC CAGGACAGGC ACCAAGGCTC CTCATCTATC GTGCATCCAA CCTAGAAACT 180

GGTATCCCTG CCAGGTTCAG TGGCAGTGGT TCTAGGACAG ACTTCACTCT CACCTATTCT 240

TCTCTAGAGC CTGAAGATTT TGCAGTGTAT TACTGTCAGC AAAGTAATGA GGATCCTCGG 300

ACGTTCGGTG GAGGCACCAA GGTGGAAGAG AAAC 334

(2) INFORMATION FOR SEQ ID NO:4:

(i) SEQUENCE CHARACTERISTICS:
(A) LENGTH: 111 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:4:

```
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Glu Ser Val Asp Ser Tyr
            20                  25                  30

Gly Asn Ser Phe Met His Trp Tyr Gln Gln Lys Pro Gly Gln Ala Pro
        35                  40                  45

Arg Leu Leu Ile Tyr Arg Ala Ser Asn Leu Glu Thr Gly Ile Pro Ala
    50                  55                  60

Arg Phe Ser Gly Ser Gly Ser Arg Thr Asp Phe Thr Leu Thr Tyr Ser
65                  70                  75                  80

Ser Leu Glu Pro Glu Asp Phe Ala Val Tyr Tyr Cys Gln Gln Ser Asn
                85                  90                  95

Glu Asp Pro Arg Thr Phe Gly Gly Gly Thr Lys Val Glu Glu Lys
            100                 105                 110
```

(2) INFORMATION FOR SEQ ID NO:5:

(i) SEQUENCE CHARACTERISTICS:
(A) LENGTH: 361 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:5:

CAGGTCCAAC TTCAGCAGCC TGGGCCTGAC CTTCTGAAGC CTGGGGCTCC AGTGAAGCTG 60

```
TCCTGCAAGG CTTCTGGCTA CACCTTCACC GACTACTGGA TGAACTGGGT TAAGCAGAGG     120
CCTGGACGAG GCCTCGAGTG GATTGGAAGG ATTGATCCTT CCGATAGTGA AACTCACTAC     180
AATCAGAAGT TCAAGGACAA GGCCACACTG ACTGTAGACA AATCCTCCAG CACAGCCTAC     240
ATCCAACTCA GCAGCCTGAC ATCTGAGGAC TCTGCAGTCT ATTACTGTGC ACGAGGGGGA     300
CGGCTCGGGT CCTTTGCTAT GGACTACTGG GGTCAAGGCA CCTCAGTCAC CGTCTCCTCA     360
G                                                                     361
```

(2) INFORMATION FOR SEQ ID NO:6:

(i) SEQUENCE CHARACTERISTICS:
(A) LENGTH: 120 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:6:

```
Gln Val Gln Leu Gln Gln Pro Gly Pro Asp Leu Leu Lys Pro Gly Ala
1               5                   10                  15

Pro Val Lys Leu Ser Cys Lys Ala Ser Gly Tyr Thr Phe Thr Asp Tyr
            20                  25                  30

Trp Met Asn Trp Val Lys Gln Arg Pro Gly Arg Gly Leu Glu Trp Ile
        35                  40                  45

Gly Arg Ile Asp Pro Ser Asp Ser Glu Thr His Tyr Asn Gln Lys Phe
    50                  55                  60

Lys Asp Lys Ala Thr Leu Thr Val Asp Lys Ser Ser Ser Thr Ala Tyr
65                  70                  75                  80

Ile Gln Leu Ser Ser Leu Thr Ser Glu Asp Ser Ala Val Tyr Tyr Cys
                85                  90                  95

Ala Arg Gly Gly Arg Leu Gly Ser Phe Ala Met Asp Tyr Trp Gly Gln
            100                 105                 110

Gly Thr Ser Val Thr Val Ser Ser
        115                 120
```

(2) INFORMATION FOR SEQ ID NO:7:

(i) SEQUENCE CHARACTERISTICS:
(A) LENGTH: 334 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:7:

```
GACATTGTGC TAACACAATC TCCAGCTTCT TTGGCTGTGT CTCTAGGGCA GAGGGCCACC    60
ATATCCTGCA GAGCCAGTGA AAGTGTTGAT AGTTATGGCA ATAGTTTTAT GCACTGGTAC   120
CAGCAGAAAC CAGGACAGCC ACCCAAACTC CTCATCTATC GTGCATCCAA CCTAGAATCT   180
GGGATCCCTG CCAGGTTCAG TGGCAGTGGG TCTAGGACAG ACTTCACCCT CACCATTAAT   240
CCTGTGGAGG CTGATGATGT TGCAACCTAT TACTGTCAGC AAAGTAATGA GGATCCTCGG   300
ACGTTCGGTG GAGGCACCAA GCTGGAAATC AAAC                                334
```

(2) INFORMATION FOR SEQ ID NO:8:

(i) SEQUENCE CHARACTERISTICS:
(A) LENGTH: 111 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:8:

```
Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Asp Ser Tyr
            20                  25                  30

Gly Asn Ser Phe Met His Trp Tyr Gln Gln Lys Pro Gly Gln Pro Pro
        35                  40                  45

Lys Leu Leu Ile Tyr Arg Ala Ser Asn Leu Glu Ser Gly Ile Pro Ala
    50                  55                  60

Arg Phe Ser Gly Ser Gly Ser Arg Thr Asp Phe Thr Leu Thr Ile Asn
65                  70                  75                  80

Pro Val Glu Ala Asp Asp Val Ala Thr Tyr Tyr Cys Gln Gln Ser Asn
                85                  90                  95

Glu Asp Pro Arg Thr Phe Gly Gly Gly Thr Lys Leu Glu Ile Lys
            100                 105                 110
```

(2) INFORMATION FOR SEQ ID NO:9:

(i) SEQUENCE CHARACTERISTICS:
(A) LENGTH: 9201 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:9:

```
AAGCTTTCTG GGGCAGGCCA GGCCTGACCT TGGCTTTGGG GCAGGGAGGG GGCTAAGGTG     60
AGGCAGGTGG CGCCAGCCAG GTGCACACCC AATGCCCATG AGCCCAGACA CTGGACGCTG    120
AACCTCGCGG ACAGTTAAGA ACCCAGGGGC CTCTGCGCCC TGGGCCCAGC TCTGTCCCAC    180
ACCGCGGTCA CATGGCACCA CCTCTCTTGC AGCCTCCACC AAGGGCCCAT CGGTCTTCCC    240
CCTGGCACCC TCCTCCAAGA GCACCTCTGG GGGCACAGCG GCCCTGGGCT GCCTGGTCAA    300
GGACTACTTC CCCGAACCGG TGACGGTGTC GTGGAACTCA GGCGCCCTGA CCAGCGGCGT    360
GCACACCTTC CCGGCTGTCC TACAGTCCTC AGGACTCTAC TCCCTCAGCA GCGTGGTGAC    420
CGTGCCCTCC AGCAGCTTGG GCACCCAGAC CTACATCTGC AACGTGAATC ACAAGCCCAG    480
CAACACCAAG GTGGACAAAC GCGTTGGTGA GAGGCCAGCA CAGGGAGGGA GGGTGTCTGC    540
TGGAAGCCAG GCTCAGCGCT CCTGCCTGGA CGCATCCCGG CTATGCAGCC CCAGTCCAGG    600
GCAGCAAGGC AGGCCCCGTC TGCCTCTTCA CCCGGAGGCC TCTGCCCGCC CCACTCATGC    660
TCAGGGAGAG GGTCTTCTGG CTTTTTCCCC AGGCTCTGGG CAGGCACAGG CTAGGTGCCC    720
CTAACCCAGG CCCTGCACAC AAAGGGGCAG GTGCTGGGCT CAGACCTGCC AAGAGCCATA    780
TCCGGGAGGA CCCTGCCCCT GACCTAAGCC CACCCCAAAG GCCAAACTCT CCACTCCCTC    840
AGCTCGGACA CCTTCTCTCC TCCCAGATTC CAGTAACTCC CAATCTTCTC TCTGCAGAGC    900
CCAAATCTTG TGACAAAACT CACACATGCC CACCGTGCCC AGGTAAGCCA GCCCAGGCCT    960
CGCCCTCCAG CTCAAGGCGG GACAGGTGCC CTAGAGTAGC CTGCATCCAG GGACAGGCCC   1020
CAGCCGGGTG CTGACACGTC CACCTCCATC TCTTCCTCAG CACCTGAACT CCTGGGGGGA   1080
CCGTCAGTCT TCCTCTTCCC CCCAAAACCC AAGGACACCC TCATGATCTC CCGGACCCCT   1140
GAGGTCACAT GCGTGGTGGT GGACGTGAGC CACGAAGACC CTGAGGTCAA GTTCAACTGG   1200
TACGTGGACG GCGTGGAGGT GCATAATGCC AAGACAAAGC CGCGGGAGGA GCAGTACAAC   1260
AGCACGTACC GTGTGGTCAG CGTCCTCACC GTCCTGCACC AGGACTGGCT GAATGGCAAG   1320
GAGTACAAGT GCAAGGTCTC CAACAAAGCC CTCCCAGCCC CCATCGAGAA AACCATCTCC   1380
AAAGCCAAAG GTGGGACCCG TGGGGTGCGA GGGCCACATG GACAGAGGCC GGCTCGGCCC   1440
ACCCTCTGCC CTGAGAGTGA CCGCTGTACC AACCTCTGTC CCTACAGGGC AGCCCCGAGA   1500
ACCACAGGTG TACACCCTGC CCCCATCTAG AGAGGAGATG ACCAAGAACC AGGTCAGCCT   1560
GACCTGCCTG GTCAAAGGCT TCTATCCCAG CGACATCGCC GTGGAGTGGG AGAGCAATGG   1620
```

GCAGCCGGAG AACAACTACA AGACCACGCC TCCCGTGCTG GACTCCGACG GCTCCTTCTT 1680
CCTCTACAGC AAGCTCACCG TGGACAAGAG CAGGTGGCAG CAGGGGAACG TCTTCTCATG 1740
CTCCGTGATG CATGAGGCTC TGCACAACCA CTACACGCAG AAGAGCCTCT CCCTGTCTCC 1800
GGGTAAATGA GTGCGACGGC CGGCAAGCCC CCGCTCCCCG GGCTCTCGCG GTCGCACGAG 1860
GATGCTTGGC ACGTACCCCC TGTACATACT TCCCGGGCGC CCAGCATGGA AATAAAGCAC 1920
CCAGCGCTGC CCTGGGCCCC TGCGAGACTG TGATGGTTCT TTCCACGGGT CAGGCCGAGT 1980
CTGAGGCCTG AGTGGCATGA GGGAGGCAGA GCGGGTCCCA CTGTCCCCAC ACTGGCCCAG 2040
GCTGTGCAGG TGTGCCTGGG CCCCCTAGGG TGGGGCTCAG CCAGGGGCTG CCCTCGGCAG 2100
GGTGGGGGAT TTGCCAGCGT GGCCCTCCCT CCAGCAGCAC CTGCCCTGGG CTGGGCCACG 2160
GGAAGCCCTA GGAGCCCCTG GGGACAGACA CACAGCCCCT GCCTCTGTAG GAGACTGTCC 2220
TGTTCTGTGA GCGCCCCTGT CCTCCCGACC TCCATGCCCA CTCGGGGGCA TGCCTAGTCC 2280
ATGTGCGTAG GGACAGGCCC TCCCTCACCC ATCTACCCCC ACGGCACTAA CCCCTGGCTG 2340
CCCTGCCCAG CCTCGCACCC GCATGGGGAC ACAACCGACT CCGGGGACAT GCACTCTCGG 2400
GCCCTGTGGA GGGACTGGTG CAGATGCCCA CACACACACT CAGCCCAGAC CCGTTCAACA 2460
AACCCCGCAC TGAGGTTGGC CGGCCACACG GCCACCACAC ACACACGTGC ACGCCTCACA 2520
CACGGAGCCT CACCCGGGCG AACTGCACAG CACCCAGACC AGAGCAAGGT CCTCGCACAC 2580
GTGAACACTC CTCGGACACA GGCCCCCACG AGCCCCACGC GGCACCTCAA GGCCCACGAG 2640
CCTCTCGGCA GCTTCTCCAC ATGCTGACCT GCTCAGACAA ACCCAGCCCT CCTCTCACAA 2700
GGGTGCCCCT GCAGCCGCCA CACACACACA GGGGATCACA CACCACGTCA CGTCCCTGGC 2760
CCTGGCCCAC TTCCCAGTGC CGCCCTTCCC TGCAGGACGG ATCCAGACAT GATAAGATAC 2820
ATTGATGAGT TTGGACAAAC CACAACTAGA ATGCAGTGAA AAAAATGCTT TATTTGTGAA 2880
ATTTGTGATG CTATTGCTTT ATTTGTAACC ATTATAAGCT GCAATAAACA AGTTAACAAC 2940
AACAATTGCA TTCATTTTAT GTTTCAGGTT CAGGGGGAGG TGTGGGAGGT TTTTTAAAGC 3000
AAGTAAAACC TCTACAAATG TGGTATGGCT GATTATGATC TCTAGTCAAG GCACTATACA 3060
TCAAATATTC CTTATTAACC CCTTTACAAA TTAAAAAGCT AAAGGTACAC AATTTTTGAG 3120
CATAGTTATT AATAGCAGAC ACTCTATGCC TGTGTGGAGT AAGAAAAAAC AGTATGTTAT 3180
GATTATAACT GTTATGCCTA CTTATAAAGG TTACAGAATA TTTTTCCATA ATTTTCTTGT 3240
ATAGCAGTGC AGCTTTTTCC TTTGTGGTGT AAATAGCAAA GCAAGCAAGA GTTCTATTAC 3300
TAAACACAGC ATGACTCAAA AAACTTAGCA ATTCTGAAGG AAAGTCCTTG GGGTCTTCTA 3360
CCTTTCTCTT CTTTTTTGGA GGAGTAGAAT GTTGAGAGTC AGCAGTAGCC TCATCATCAC 3420

```
TAGATGGCAT TTCTTCTGAG CAAAACAGGT TTTCCTCATT AAAGGCATTC CACCACTGCT   3480
CCCATTCATC AGTTCCATAG GTTGGAATCT AAAATACACA AACAATTAGA ATCAGTAGTT   3540
TAACACATTA TACACTTAAA AATTTTATAT TTACCTTAGA GCTTTAAATC TCTGTAGGTA   3600
GTTTGTCCAA TTATGTCACA CCACAGAAGT AAGGTTCCTT CACAAAGATC CGGGACCAAA   3660
GCGGCCATCG TGCCTCCCCA CTCCTGCAGT TCGGGGGCAT GGATGCGCGG ATAGCCGCTG   3720
CTGGTTTCCT GGATGCCGAC GGATTTGCAC TGCCGGTAGA ACTCCGCGAG GTCGTCCAGC   3780
CTCAGGCAGC AGCTGAACCA ACTCGCGAGG GGATCGAGCC CGGGGTGGGC GAAGAACTCC   3840
AGCATGAGAT CCCCGCGCTG GAGGATCATC CAGCCGGCGT CCCGGAAAAC GATTCCGAAG   3900
CCCAACCTTT CATAGAAGGC GGCGGTGGAA TCGAAATCTC GTGATGGCAG GTTGGGCGTC   3960
GCTTGGTCGG TCATTTCGAA CCCCAGAGTC CCGCTCAGAA GAACTCGTCA AGAAGGCGAT   4020
AGAAGGCGAT GCGCTGCGAA TCGGGAGCGG CGATACCGTA AAGCACGAGG AAGCGGTCAG   4080
CCCATTCGCC GCCAAGCTCT TCAGCAATAT CACGGGTAGC CAACGCTATG TCCTGATAGC   4140
GGTCCGCCAC ACCCAGCCGG CCACAGTCGA TGAATCCAGA AAAGCGGCCA TTTTCCACCA   4200
TGATATTCGG CAAGCAGGCA TCGCCATGGG TCACGACGAG ATCCTCGCCG TCGGGCATGC   4260
GCGCCTTGAG CCTGGCGAAC AGTTCGGCTG GCGCGAGCCC CTGATGCTCT TCGTCCAGAT   4320
CATCCTGATC GACAAGACCG GCTTCCATCC GAGTACGTGC TCGCTCGATG CGATGTTTCG   4380
CTTGGTGGTC GAATGGGCAG GTAGCCGGAT CAAGCGTATG CAGCCGCCGC ATTGCATCAG   4440
CCATGATGGA TACTTTCTCG GCAGGAGCAA GGTGAGATGA CAGGAGATCC TGCCCCGGCA   4500
CTTCGCCCAA TAGCAGCCAG TCCCTTCCCG CTTCAGTGAC AACGTCGAGC ACAGCTGCGC   4560
AAGGAACGCC CGTCGTGGCC AGCCACGATA GCCGCGCTGC CTCGTCCTGC AGTTCATTCA   4620
GGGCACCGGA CAGGTCGGTC TTGACAAAAA GAACCGGGCG CCCCTGCGCT GACAGCCGGA   4680
ACACGGCGGC ATCAGAGCAG CCGATTGTCT GTTGTGCCCA GTCATAGCCG AATAGCCTCT   4740
CCACCCAAGC GGCCGGAGAA CCTGCGTGCA ATCCATCTTG TTCAATCATG CGAAACGATC   4800
CTCATCCTGT CTCTTGATCA GATCTTGATC CCCTGCGCCA TCAGATCCTT GGCGGCAAGA   4860
AAGCCATCCA GTTTACTTTG CAGGGCTTCC CAACCTTACC AGAGGGCGCC CCAGCTGGCA   4920
ATTCCGGTTC GCTTGCTGTC CATAAAACCG CCCAGTCTAG CTATCGCCAT GTAAGCCCAC   4980
TGCAAGCTAC CTGCTTTCTC TTTGCGCTTG CGTTTTCCCT TGTCCAGATA GCCCAGTAGC   5040
TGACATTCAT CCGGGGTCAG CACCGTTTCT GCGGACTGGC TTTCTACGTG TTCCGCTTCC   5100
TTTAGCAGCC CTTGCGCCCT GAGTGCTTGC GGCAGCGTGA AGCTAGCTTT TTGCAAAAGC   5160
CTAGGCCTCC AAAAAAGCCT CCTCACTACT TCTGGAATAG CTCAGAGGCC GAGGCGGCCT   5220
```

CGGCCTCTGC ATAAATAAAA AAAATTAGTC AGCCATGGGG CGGAGAATGG GGCGGGATGG 5280
GCGGAGTTAG GGCGGAACTG GGCGGAGTTA GGGGCGGGAC TATGGTTGCT GACTAATTGA 5340
GATGCATGCT TTGCATACTT CTGCCTGCTG GGGAGCCTGG GGACTTTCCA CACCTGGTTG 5400
CTGACTAATT GAGATGCATG CTTTGCATAC TTCTGCCTGC TGGGGAGCCT GGGGACTTTC 5460
CACACCCTAA CTGACACACA TTCCACAGCT GCCTCGCGCG TTTCGGTGAT GACGGTGAAA 5520
ACCTCTGACA CATGCAGCTC CCGGAGACGG TCACAGCTTG TCTGTAAGCG GATGCCGGGA 5580
GCAGACAAGC CCGTCAGGGC GCGTCAGCGG GTGTTGGCGG GTGTCGGGGC GCAGCCATGA 5640
CCCAGTCACG TAGCGATAGC GGAGTGTATA CTGGCTTAAC TATGCGGCAT CAGAGCAGAT 5700
TGTACTGAGA GTGCACCATA TGCGGTGTGA AATACCGCAC AGATGCGTAA GGAGAAAATA 5760
CCGCATCAGG CGCTCTTCCG CTTCCTCGCT CACTGACTCG CTGCGCTCGG TCGTTCGGCT 5820
GCGGCGAGCG GTATCAGCTC ACTCAAAGGC GGTAATACGG TTATCCACAG AATCAGGGGA 5880
TAACGCAGGA AAGAACATGT GAGCAAAAGG CCAGCAAAAG GCCAGGAACC GTAAAAAGGC 5940
CGCGTTGCTG GCGTTTTTCC ATAGGCTCCG CCCCCCTGAC GAGCATCACA AAAATCGACG 6000
CTCAAGTCAG AGGTGGCGAA ACCCGACAGG ACTATAAAGA TACCAGGCGT TTCCCCCTGG 6060
AAGCTCCCTC GTGCGCTCTC CTGTTCCGAC CCTGCCGCTT ACCGGATACC TGTCCGCCTT 6120
TCTCCCTTCG GGAAGCGTGG CGCTTTCTCA TAGCTCACGC TGTAGGTATC TCAGTTCGGT 6180
GTAGGTCGTT CGCTCCAAGC TGGGCTGTGT GCACGAACCC CCCGTTCAGC CCGACCGCTG 6240
CGCCTTATCC GGTAACTATC GTCTTGAGTC CAACCCGGTA AGACACGACT TATCGCCACT 6300
GGCAGCAGCC ACTGGTAACA GGATTAGCAG AGCGAGGTAT GTAGGCGGTG CTACAGAGTT 6360
CTTGAAGTGG TGGCCTAACT ACGGCTACAC TAGAAGGACA GTATTTGGTA TCTGCGCTCT 6420
GCTGAAGCCA GTTACCTTCG GAAAAAGAGT TGGTAGCTCT TGATCCGGCA AACAAACCAC 6480
CGCTGGTAGC GGTGGTTTTT TTGTTTGCAA GCAGCAGATT ACGCGCAGAA AAAAAGGATC 6540
TCAAGAAGAT CCTTTGATCT TTTCTACGGG GTCTGACGCT CAGTGGAACG AAAACTCACG 6600
TTAAGGGATT TTGGTCATGA GATTATCAAA AAGGATCTTC ACCTAGATCC TTTTAAATTA 6660
AAAATGAAGT TTTAAATCAA TCTAAAGTAT ATATGAGTAA ACTTGGTCTG ACAGTTACCA 6720
ATGCTTAATC AGTGAGGCAC CTATCTCAGC GATCTGTCTA TTTCGTTCAT CCATAGTTGC 6780
CTGACTCCCC GTCGTGTAGA TAACTACGAT ACGGGAGGGC TTACCATCTG GCCCCAGTGC 6840
TGCAATGATA CCGCGAGACC CACGCTCACC GGCTCCAGAT TTATCAGCAA TAAACCAGCC 6900
AGCCGGAAGG GCCGAGCGCA GAAGTGGTCC TGCAACTTTA TCCGCCTCCA TCCAGTCTAT 6960
TAATTGTTGC CGGGAAGCTA GAGTAAGTAG TTCGCCAGTT AATAGTTTGC GCAACGTTGT 7020

TGCCATTGCT GCAGGCATCG TGGTGTCACG CTCGTCGTTT GGTATGGCTT CATTCAGCTC 7080
CGGTTCCCAA CGATCAAGGC GAGTTACATG ATCCCCCATG TTGTGCAAAA AAGCGGTTAG 7140
CTCCTTCGGT CCTCCGATCG TTGTCAGAAG TAAGTTGGCC GCAGTGTTAT CACTCATGGT 7200
TATGGCAGCA CTGCATAATT CTCTTACTGT CATGCCATCC GTAAGATGCT TTTCTGTGAC 7260
TGGTGAGTAC TCAACCAAGT CATTCTGAGA ATAGTGTATG CGGCGACCGA GTTGCTCTTG 7320
CCCGGCGTCA ACACGGGATA ATACCGCGCC ACATAGCAGA ACTTTAAAAG TGCTCATCAT 7380
TGGAAAACGT TCTTCGGGGC GAAAACTCTC AAGGATCTTA CCGCTGTTGA GATCCAGTTC 7440
GATGTAACCC ACTCGTGCAC CCAACTGATC TTCAGCATCT TTTACTTTCA CCAGCGTTTC 7500
TGGGTGAGCA AAAACAGGAA GGCAAAATGC CGCAAAAAAG GGAATAAGGG CGACACGGAA 7560
ATGTTGAATA CTCATACTCT TCCTTTTTCA ATATTATTGA AGCATTTATC AGGGTTATTG 7620
TCTCATGAGC GGATACATAT TTGAATGTAT TTAGAAAAAT AAACAAATAG GGGTTCCGCG 7680
CACATTTCCC CGAAAAGTGC CACCTGACGT CTAAGAAACC ATTATTATCA TGACATTAAC 7740
CTATAAAAAT AGGCGTATCA CGAGGCCCTT TCGTCTTCAA GAATTCATCG ATATCGGAAA 7800
ATGAAAAAAA ATATTTTTTA ATTTTAAAAT GAAATGTTTA TTTTCAATTT CTCCAAATTT 7860
CACAAGGAAA GATTAGTCAC GGGTATGGGA GAGCAGAGGA CCATAAGAGT TCAGGAATAG 7920
AATCCATTAT GATTCTGGAG TCAAGGAAGT ACTGATGCCA AGGTTTCAGT ATAAGAGCAG 7980
TATCCACTGG AAAGGATAAA GTCACTACAA CTGAGCACAG AGCAGGACAG CTACCTAATG 8040
AGTGGTCACT AATGGGCCAC TGTTACACTG TTATACGGCT TAGGAATGAG CACTGAGGCT 8100
GTGAGGTGTA TGGGTAAGGA CATCAGGATG TAAACCCAGC TCAGGTAGAG GACTCAGAGC 8160
ACAGCACAAT CAGCACGAAC TAATAAACAA CAGATAAGAT AAGGCACAAG CTCAGCAATA 8220
TTGGATCAGG GATCTTTGTA AATCTGACTG TGTATTCAGT CTAGTTCAAT GTGACTCATG 8280
AAGCCCACCC ATATGCAAAT CTAGAGAAGA CTTTAGAGTA TAAATCTGAG GCTCACCTCA 8340
CATACCAGCA AGGGAGTGAC CAGCTTGTCT TAAGGCACCA CTGAGCCCAA GTCTTAGACA 8400
TCATGGATTG GCTGTGGAAC TTGCTATTCC TGATGGCAGC TGCCCAAGGT AAGTCATCAG 8460
AAAAAAGAGT TCCAAGGGAA ATTGAAGCAG TTCCGAGCTC GGTACCCTCG AGATCCTAGA 8520
GAGGTCTGGT GGAGCCTGCA AAAGTCCAGC TTTCAAAGGA ACACAGAAGT ATGTGTATGG 8580
AATATTAGAA GATGTTGCTT TTACTCTTAA GTTGGTTCCT AGGAAAAATA GTTAAATACT 8640
GTGACTTTAA AATGTGAGAG GGTTTTCAAG TACTCATTTT TTTAAATGTC CAAAATTTTT 8700
GTCAATCAAT TTGAGGTCTT GTTTGTGTAG AACTGACATT ACTTAAAGTT TAACCGAGGA 8760
ATGGGAGTGA GGCTCTCTCA TACCCTATCC AGAACTGACT TTTAACAATA ATAAATTAAG 8820

TTTAAAATAT TTTTAAATGA ATTGAGCAAT GTTGAGTTGA GTCAAGATGG CCGATCAGAA 8880

CCAGAACACC TGCAGCAGCT GGCAGGAAGC AGGTCATGTG GCAAGGCTAT TTGGGGAAGG 8940

GAAAATAAAA CCACTAGGTA AACTTGTAGC TGTGGTTTGA AGAAGTGGTT TTGAAACACT 9000

CTGTCCAGCC CCACCAAACC GAAAGTCCAG GCTGAGCAAA ACACCACCTG GGTAATTTGC 9060

ATTTCTAAAA TAAGTTGAGG ATTCAGCCGA AACTGGAGAG GTCCTCTTTT AACTTATTGA 9120

GTTCAACCTT TTAATTTTAG CTTGAGTAGT TCTAGTTTCC CCAAACTTAA GTTTATCGAC 9180

TTCTAAAATG TATTTAGAAT T 9201

(2) INFORMATION FOR SEQ ID NO:10:

(i) SEQUENCE CHARACTERISTICS:
(A) LENGTH: 7059 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:10:

GGATCCAGAC ATGATAAGAT ACATTGATGA GTTTGGACAA ACCACAACTA GAATGCAGTG 60

AAAAAAATGC TTTATTTGTG AAATTTGTGA TGCTATTGCT TTATTTGTAA CCATTATAAG 120

CTGCAATAAA CAAGTTAACA ACAACAATTG CATTCATTTT ATGTTTCAGG TTCAGGGGGA 180

GGTGTGGGAG GTTTTTTAAA GCAAGTAAAA CCTCTACAAA TGTGGTATGG CTGATTATGA 240

TCTCTAGTCA AGGCACTATA CATCAAATAT TCCTTATTAA CCCCTTTACA AATTAAAAAG 300

CTAAAGGTAC ACAATTTTTG AGCATAGTTA TTAATAGCAG ACACTCTATG CCTGTGTGGA 360

GTAAGAAAAA ACAGTATGTT ATGATTATAA CTGTTATGCC TACTTATAAA GGTTACAGAA 420

TATTTTTCCA TAATTTTCTT GTATAGCAGT GCAGCTTTTT CCTTTGTGGT GTAAATAGCA 480

AAGCAAGCAA GAGTTCTATT ACTAAACACA GCATGACTCA AAAAACTTAG CAATTCTGAA 540

GGAAAGTCCT TGGGGTCTTC TACCTTTCTC TTCTTTTTTG GAGGAGTAGA ATGTTGAGAG 600

TCAGCAGTAG CCTCATCATC ACTAGATGGC ATTTCTTCTG AGCAAAACAG GTTTTCCTCA 660

TTAAAGGCAT TCCACCACTG CTCCCATTCA TCAGTTCCAT AGGTTGGAAT CTAAAATACA 720

CAAACAATTA GAATCAGTAG TTTAACACAT TATACACTTA AAAATTTTAT ATTTACCTTA 780

GAGCTTTAAA TCTCTGTAGG TAGTTTGTCC AATTATGTCA CACCACAGAA GTAAGGTTCC 840

TTCACAAAGA TCCGGGGCCC ACTCATAAAT CCAGTTGCCG CCACGGTAGC CAATCACCGT 900

ATCGTATAAA TCATCGTCGG TACGTTCGGC ATCGCTCATC ACAATACGTG CCTGGACGTC 960

GAGGATTTCG CGTGGGTCAA TGCCGCGCCA GATCCACATC AGACGGTTAA TCATGCGATA 1020

CCAGTGAGGG ATGGTTTTAC CATCAAGGGC CGACTGCACA GGCGGTTGTG CGCCGTGATT 1080

AAAGCGGCGG ACTAGCGTCG AGGTTTCAGG ATGTTTAAAG CGGGGTTTGA ACAGGGTTTC 1140

GCTCAGGTTT GCCTGTGTCA TGGATGCAGC CTCCAGAATA CTTACTGGAA ACTATTGTAA 1200

CCCGCCTGAA GTTAAAAAGA ACAACGCCCG GCAGTGCCAG GCGTTGAAAA GATTAGCGAC 1260

CGGAGATTGG CGGGACGAAT ACGACGCCCA TATCCCACGG CTGTTCAATC CAGGTATCTT 1320

GCGGGATATC AACAACATAG TCATCAACCA GCGGACGACC AGCCGGTTTT GCGAAGATGG 1380

TGACAAAGTG CGCTTTTGGA TACATTTCAC GAATCGCAAC CGCAGTACCA CCGGTATCCA 1440

CCAGGTCATC AATAACGATG AAGCCTTCGC CATCGCCTTC TGCGCGTTTC AGCACTTTAA 1500

GCTCGCGCTG GTTGTCGTGA TCGTAGCTGG AAATACAAAC GGTATCGACA TGACGAATAC 1560

CCAGTTCACG CGCCAGTAAC GCACCCGGTA CCAGACCGCC ACGGCTTACG GCAATAATGC 1620

CTTTCCATTG TTCAGAAGGC ATCAGTCGGC TTGCGAGTTT ACGTGCATGG ATCTGCAACA 1680

TGTCCCAGGT GACGATGTAT TTTTCGCTCA TGTGAAGTGT CCCAGCCTGT TTATCTACGG 1740

CTTAAAAAGT GTTCGAGGGG AAAATAGGTT GCGCGAGATT ATAGAGATCA GCTTTTTGCA 1800

AAAGCCTAGG CCTCCAAAAA AGCCTCCTCA CTACTTCTGG AATAGCTCAG AGGCCGAGGC 1860

GGCCTCGGCC TCTGCATAAA TAAAAAAAAT TAGTCAGCCA TGGGGCGGAG AATGGGCGG 1920

GATGGGCGGA GTTAGGGCGG AACTGGGCGG AGTTAGGGGC GGGACTATGG TTGCTGACTA 1980

ATTGAGATGC TGCATTAATG AATCGGCCAA CGCGCGGGGA GAGGCGGTTT GCGTATTGGG 2040

CGCTCTTCCG CTTCCTCGCT CACTGACTCG CTGCGCTCGG TCGTTCGGCT GCGGCGAGCG 2100

GTATCAGCTC ACTCAAAGGC GGTAATACGG TTATCCACAG AATCAGGGGA TAACGCAGGA 2160

AAGAACATGT GAGCAAAAGG CCAGCAAAAG GCCAGGAACC GTAAAAAGGC CGCGTTGCTG 2220

GCGTTTTTCC ATAGGCTCCG CCCCCCTGAC GAGCATCACA AAAATCGACG CTCAAGTCAG 2280

AGGTGGCGAA ACCCGACAGG ACTATAAAGA TACCAGGCGT TTCCCCCTGG AAGCTCCCTC 2340

GTGCGCTCTC CTGTTCCGAC CCTGCCGCTT ACCGGATACC TGTCCGCCTT TCTCCCTTCG 2400

GGAAGCGTGG CGCTTTCTCA ATGCTCACGC TGTAGGTATC TCAGTTCGGT GTAGGTCGTT 2460

CGCTCCAAGC TGGGCTGTGT GCACGAACCC CCCGTTCAGC CCGACCGCTG CGCCTTATCC 2520

GGTAACTATC GTCTTGAGTC CAACCCGGTA AGACACGACT TATCGCCACT GGCAGCAGCC 2580

ACTGGTAACA GGATTAGCAG AGCGAGGTAT GTAGGCGGTG CTACAGAGTT CTTGAAGTGG 2640

TGGCCTAACT ACGGCTACAC TAGAAGGACA GTATTTGGTA TCTGCGCTCT GCTGAAGCCA 2700

GTTACCTTCG GAAAAAGAGT TGGTAGCTCT TGATCCGGCA AACAAACCAC CGCTGGTAGC 2760

GGTGGTTTTT TTGTTTGCAA GCAGCAGATT ACGCGCAGAA AAAAAGGATC TCAAGAAGAT 2820
CCTTTGATCT TTTCTACGGG GTCTGACGCT CAGTGGAACG AAAACTCACG TTAAGGGATT 2880
TTGGTCATGA GATTATCAAA AAGGATCTTC ACCTAGATCC TTTTAAATTA AAAATGAAGT 2940
TTTAAATCAA TCTAAAGTAT ATATGAGTAA ACTTGGTCTG ACAGTTACCA ATGCTTAATC 3000
AGTGAGGCAC CTATCTCAGC GATCTGTCTA TTTCGTTCAT CCATAGTTGC CTGACTCCCC 3060
GTCGTGTAGA TAACTACGAT ACGGGAGGGC TTACCATCTG GCCCCAGTGC TGCAATGATA 3120
CCGCGAGACC CACGCTCACC GGCTCCAGAT TTATCAGCAA TAAACCAGCC AGCCGGAAGG 3180
GCCGAGCGCA GAAGTGGTCC TGCAACTTTA TCCGCCTCCA TCCAGTCTAT TAATTGTTGC 3240
CGGGAAGCTA GAGTAAGTAG TTCGCCAGTT AATAGTTTGC GCAACGTTGT TGCCATTGCT 3300
ACAGGCATCG TGGTGTCACG CTCGTCGTTT GGTATGGCTT CATTCAGCTC CGGTTCCCAA 3360
CGATCAAGGC GAGTTACATG ATCCCCCATG TTGTGCAAAA AAGCGGTTAG CTCCTTCGGT 3420
CCTCCGATCG TTGTCAGAAG TAAGTTGGCC GCAGTGTTAT CACTCATGGT TATGGCAGCA 3480
CTGCATAATT CTCTTACTGT CATGCCATCC GTAAGATGCT TTTCTGTGAC TGGTGAGTAC 3540
TCAACCAAGT CATTCTGAGA ATAGTGTATG CGGCGACCGA GTTGCTCTTG CCCGGCGTCA 3600
ATACGGGATA ATACCGCGCC ACATAGCAGA ACTTTAAAAG TGCTCATCAT TGGAAAACGT 3660
TCTTCGGGGC GAAAACTCTC AAGGATCTTA CCGCTGTTGA GATCCAGTTC GATGTAACCC 3720
ACTCGTGCAC CCAACTGATC TTCAGCATCT TTTACTTTCA CCAGCGTTTC TGGGTGAGCA 3780
AAAACAGGAA GGCAAAATGC CGCAAAAAAG GGAATAAGGG CGACACGGAA ATGTTGAATA 3840
CTCATACTCT TCCTTTTTCA ATATTATTGA AGCATTTATC AGGGTTATTG TCTCATGAGC 3900
GGATACATAT TTGAATGTAT TTAGAAAAAT AAACAAATAG GGGTTCCGCG CACATTTCCC 3960
CGAAAAGTGC CACCTGACGT CTAAGAAACC ATTATTATCA TGACATTAAC CTATAAAAAT 4020
AGGCGTATCA CGAGGCCCTT TCGTCTCGCG CGTTTCGGTG ATGACGGTGA AAACCTCTGA 4080
CACATGCAGC TCCCGGAGAC GGTCACAGCT TGTCTGTAAG CGGATGCCGG GAGCAGACAA 4140
GCCCGTCAGG GCGCGTCAGC GGGTGTTGGC GGGTGTCGGG GCTGGCTTAA CTATGCGGCA 4200
TCAGAGCAGA TTGTACTGAG AGTGCACCAT AGCGGCCGCA TATGCGGTGT GAAATACCGC 4260
ACAGATGCGT AAGGAGAAAA TACCGCATCA GGCGCCATTC GCCATTCAGG CTGCGCAACT 4320
GTTGGGAAGG GCGATCGGTG CGGGCCTCTT CGCTATTACG CCAGAATTCG GCCCAGGGGA 4380
CTGTGAGGAC AGAAGGCTTG TGGGTTTGAG GGAGGACTGT CTTGCAGAGG ATGATAGGGT 4440
AAAATAGAAT GAAGGATGAT TTTTATAAAT GGTTACGTGC CTTAGGATGA CTACATATTT 4500
AGTCCCTTAT AAGAGAAATT GAGTAGTTGG TAAAACAACA GATAATAATT ATTAAATGAG 4560

GAAAGAGAGA AACCACAGGT GCAAAGATTC ACTTTATTTA TTCATTCTCC TCCAACATTA 4620
GCATAATTAA AGCCAAGGAG GAGGAGGGGG GTGAGGTGAA AGATGAGCTG GAGGACCGCA 4680
ATAGGGGTAG GTCCCCTGTG GAAAAAGGGT CAGAGGCCAA AGGATGGGAG GGGGTCAGGC 4740
TGGAACTGAG GAGCAGGTGG GGGCACTTCT CCCTCTAACA CTCTCCCCTG TTGAAGCTCT 4800
TTGTGACGGG CGAGCTCAGG CCCTGATGGG TGACTTCGCA GGCGTAGACT TTGTGTTTCT 4860
CGTAGTCTGC TTTGCTCAGC GTCAGGGTGC TGCTGAGGCT GTAGGTGCTG TCCTTGCTCT 4920
CCTGCTCTGT GACACTCTCC TGGGAGTTAC CCGATTGGAG GGCGTTATCC ACCTTCCACT 4980
GTACTTTGGC CTCTCTGGGA TAGAAGTTAT TCAGCAGGCA CACAACAGAG GCAGTTCCAG 5040
ATTTCAACTG CTCATCAGAT GGCGGGAAGA TGAAGACAGA TGGTGCAGCC ACAGTTCCTG 5100
AGGAAAGAAG CAAACAGGAT GGTGTTTAAG TAACAAAGTT CTGCCCTTGG GTGTGTTGTT 5160
TGCGGATAAG GGCATGTTAG GGACAGACAG AAAACAGCAT GCTTATCCCA GATAATTATA 5220
GCAAGGAGAC CAAGAAGCGT ATTTAAAATC TTGATGTTTT GAGTTTCTTC CTAGCTTCCC 5280
CCTATTCCTT AATAAAGTTC TAAATTGTTT TGTTGGAGCT CTTTGCAGCC ATTCTGAGGG 5340
CTTTGCATGC TTTTCTGACC TTGCAGTAAA CTCAATGCTT TAGGCAAAGA ATGGCCACGT 5400
CATCCGACCC CCTCAGAGTT TAGAATTCAT CGATATCTAG ATCCTAGATA ATTGCATTCA 5460
TTTAAAAAAA AAATATTTCT CCTAAAATGA ATACTCAGAA AGTGGTCTTG AAAAAGATTT 5520
GTGAAGCCGT TTTGACCAGA ATGTCAAAGT CTTAATAGTA AGGCAAAACA AACAACTAAA 5580
AAAGATCATG AACAAAGTCA CTGTAAAGAC TTCGGGTATT GGAAAATAAT TGAATGGAGA 5640
CCAATAATCA GAGGGAAGAA TAATAGAGTA ATTTTAAGAA GTTTTCTAAA TATATTAGAA 5700
ATTAAAGACA CTAAAGTCCT TCAATTTCTT ACATAACCTA ATTTTGAAAA TGAATTCTAA 5760
ATACATTTTA GAAGTCGATA AACTTAAGTT TGGGGAAACT AGAACTACTC AAGCTAAAAT 5820
TAAAAGGTTG AACTCAATAA GTTAAAAGAG GACCTCTCCA GTTTCGGCTG AATCCTCAAC 5880
TTATTTTAGA AATGCAAATT ACCCAGGTGG TGTTTTGCTC AGCCTGGACT TTCGGTTTGG 5940
TGGGGCTGGA CAGAGTGTTT CAAAACCACT TCTTCAAACC ACAGCTACAA GTTTACCTAG 6000
TGGTTTTATT TTCCCTTCCC CAAATAGCCT TGCCACATGA CCTGCTTCCT GCCAGCTGCT 6060
GCAGGTGTTC TGGTTCTGAT CGGCCATCTT GACTCAACTC AACATTGCTC AATTCATTTA 6120
AAAATATTTT AAACTTAATT TATTATTGTT AAAAGTCAGT TCTGGATAGG GTATGAGAGA 6180
GCCTCACTCC CATTCCTCGG TTAAACTTTA AGTAATGTCA GTTCTACACA AACAAGACCT 6240
CAAATTGATT GACAAAAATT TTGGACATTT AAAAAAATGA GTACTTGAAA ACCCTCTCAC 6300
ATTTTAAAGT CACAGTATTT AACTATTTTT CCTAGGAACC AACTTAAGAG TAAAAGCAAC 6360

ATCTTCTAAT ATTCCATACA CATACTTCTG TGTTCCTTTG AAAGCTGGAC TTTTGCAGGC 6420

TCCACCAGAC CTCTCTAGGA TCTCGAGCTC GCGAAAGCTT GCATGCCTGC AGGTCGACTC 6480

TAGAGGATCA AACCAACTGT CTTTGAGTAG AGCCAAAATT GTTGATATAC TTTGAATTTT 6540

AATTATATTT CTTGCTGAGC AGAGGTGGCA AGAGTTTTCA CTAATGTGCA AAACCACCTC 6600

ATGTTCCCCT CACCTGGGAG CCAGAGTAGC AGGAGGAAGA GAAGCTGAGC TGGGGCTTCC 6660

ATGGTTCCCT CTGGGTCCTA ACTGAGCAGT TCCTCCCCAG GGCTCTGACA CAGGCATTGA 6720

TATGGGCTCT GGAAGGTAGG GCAGCTGGGA GGGACATGCA AAGCAGCTGG GTGGGAGCTG 6780

AGCTTCCAGC TGCAGAGACC ACCTGCTTCT TCCTCTCTGC ACTGAGCATC CTGCGCCACC 6840

CTGGTTGTCA GGCCAGAAAA GTCTGTTGGC TCAGTCTGAG TGTAGAACTT CTCCCTTGTG 6900

CTCAGAGAAT TTCATTCCTA TGTCTTTCTT CTCCTCAATC ACCTAAATTC ACCCAGATGA 6960

TGTTTGGCAC AAGCCTGTTA AGAACAATAT AAAAGGCTGT GTTTTCATTT CTCTCTTCCT 7020

ATCCTCAATA TGCCCAGTCA TCTCCCTAAG TGCATTATT 7059

(2) INFORMATION FOR SEQ ID NO:11:

(i) SEQUENCE CHARACTERISTICS:
(A) LENGTH: 81 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:11:

AAAAAAGAAT TCGAGCTCTT CTGATAACGC TGTCCTTCTG TTTGCAGGTG TCCAGTGTCA 60

GGTCCAACTT GTCCAGTCCG G 81

(2) INFORMATION FOR SEQ ID NO:12:

(i) SEQUENCE CHARACTERISTICS:
(A) LENGTH: 93 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:12:

TTAACTTCGG CACCGGACTG GACAAGTTGG ACCTGACACT GGACACCTGC AAACAGAAGG 60

ACAGCGTTAT CAGAAGAGCT CGAATTCTTT TTT 93

(2) INFORMATION FOR SEQ ID NO:13:

(i) SEQUENCE CHARACTERISTICS:
(A) LENGTH: 90 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:13:

TGCCGAAGTT AAGAAGCCTG GCGCTTCTGT GAAGGTCTCC TGCAAGGCTT CTGGCTACAC 60

CTTCACCGAC TACTGGATGA ACTGGGTTCG 90

(2) INFORMATION FOR SEQ ID NO:14:

(i) SEQUENCE CHARACTERISTICS:
(A) LENGTH: 90 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:14:

CCAGGTGCCT GTCGAACCCA GTTCATCCAG TAGTCGGTGA AGGTGTAGCC AGAAGCCTTG 60

CAGGAGACCT TCACAGAAGC GCCAGGCTTC 90

(2) INFORMATION FOR SEQ ID NO:15:

(i) SEQUENCE CHARACTERISTICS:
(A) LENGTH: 90 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:15:

ACAGGCACCT GGACAGGGCC TAGAGTGGAT GGGAAGGATT GATCCTTCCG ATAGTGAAAC 60

TCACTACAAT CAGAAGTTCC AGGGTAGGGT 90

(2) INFORMATION FOR SEQ ID NO:16:

(i) SEQUENCE CHARACTERISTICS:
(A) LENGTH: 90 base pairs
(B) TYPE: nucleic acid

(C) STRANDEDNESS: single
(D) TOPOLOGY: linear

(ii) MOLECULE TYPE: DNA (genomic)

(xi) SEQUENCE DESCRIPTION: SEQ ID NO:16:

CGGGTCATTG TTACCCTACC CTGGAACTTC TGATTGTAGT GAGTTTCACT ATCGGAAGGA 60

TCAATCCTTC CCATCCACTC TAGGCCCTGT 90

(2) INFORMATION FOR SEQ ID NO:17:

(i) SEQUENCE CHARACTERISTICS:
(A) LENGTH: 90 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:17:

AACAATGACC CGAGACACAT CCACCAGCAC AGTCTACATG GAACTCAGCA GCCTGCGATC 60

TGAGGACACC GCAGTCTATT ACTGTGCACG 90

(2) INFORMATION FOR SEQ ID NO:18:

(i) SEQUENCE CHARACTERISTICS:
(A) LENGTH: 90 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:18:

AGCCGTCCAC CTCGTGCACA GTAATAGACT GCGGTGTCCT CAGATCGCAG GCTGCTGAGT 60

TCCATGTAGA CTGTGCTGGT GGATGTGTCT 90

(2) INFORMATION FOR SEQ ID NO:19:

(i) SEQUENCE CHARACTERISTICS:
(A) LENGTH: 93 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:19:

AGGTGGACGG CTCGGTTCCT TTGCTATGGA CTACTGGGGT CAAGGCACCC TCGTCACCGT 60

CTCCTCAGGT GAGTCCTCAC ACTCGAGGTC GAC 93

(2) INFORMATION FOR SEQ ID NO:20:

(i) SEQUENCE CHARACTERISTICS:
(A) LENGTH: 87 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:20:

AAAAAAGTCG ACCTCGAGTG TGAGGACTCA CCTGAGGAGA CGGTGACGAG GGTGCCTTGA 60

CCCCAGTAGT CCATAGCAAA GGAACCG 87

(2) INFORMATION FOR SEQ ID NO:21:

(i) SEQUENCE CHARACTERISTICS:
(A) LENGTH: 90 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:21:

TTTTTTGAAT TCAAGCTTTC CTGACTACAT GAGTGCATTT CTGTTTTATT TCCAATTTCA 60

GATACCACCG GAGAAATTGT GCTAACACAA 90

(2) INFORMATION FOR SEQ ID NO:22:

(i) SEQUENCE CHARACTERISTICS:
(A) LENGTH: 78 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:22:

AATTTCTCCG GTGGTATCTG AAATTGGAAA TAAAACAGAA ATGCACTCAT GTAGTCAGGA 60

AAGCTTGAAT TCAAAAAA 78

(2) INFORMATION FOR SEQ ID NO:23:

(i) SEQUENCE CHARACTERISTICS:
(A) LENGTH: 87 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:23:

TCTCCAGCTA CATTGTCTTT GTCTCCAGGT GAGAGAGCCA CTCTATCCTG CAGAGCCAGT 60

GAAAGTGTTG ATAGTTATGG CAATAGT 87

(2) INFORMATION FOR SEQ ID NO:24:

(i) SEQUENCE CHARACTERISTICS:
(A) LENGTH: 87 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:24:

ACTATCAACA CTTTCACTGG CTCTGCAGGA TAGAGTGGCT CTCTCACCTG GAGACAAAGA 60

CAATGTAGCT GGAGATTGTG TTAGCAC 87

(2) INFORMATION FOR SEQ ID NO:25:

(i) SEQUENCE CHARACTERISTICS:
(A) LENGTH: 87 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:25:

TTTATGCACT GGTACCAGCA GAAACCAGGA CAGGCACCAA GGCTCCTCAT CTATCGTGCA 60

TCCAACCTAG AAACTGGTAT CCCTGCC 87

(2) INFORMATION FOR SEQ ID NO:26:

(i) SEQUENCE CHARACTERISTICS:
(A) LENGTH: 87 base pairs
(B) TYPE: nucleic acid

(C) STRANDEDNESS: single
(D) TOPOLOGY: linear

(ii) MOLECULE TYPE: DNA (genomic)

(xi) SEQUENCE DESCRIPTION: SEQ ID NO:26:

AGTTTCTAGG TTGGATGCAC GATAGATGAG GAGCCTTGGT GCCTGTCCTG GTTTCTGCTG 60

GTACCAGTGC ATAAAACTAT TGCCATA 87

(2) INFORMATION FOR SEQ ID NO:27:

(i) SEQUENCE CHARACTERISTICS:
(A) LENGTH: 87 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:27:

AGGTTCAGTG GCAGTGGTTC TAGGACAGAC TTCACTCTCA CCTATTCTTC TCTAGAGCCT 60

GAAGATTTTG CAGTGTATTA CTGTCAG 87

(2) INFORMATION FOR SEQ ID NO:28:

(i) SEQUENCE CHARACTERISTICS:
(A) LENGTH: 87 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:28:

CACTGCAAAA TCTTCAGGCT CTAGAGAAGA ATAGGTGAGA GTGAAGTCTG TCCTAGAACC 60

ACTGCCACTG AACCTGGCAG GGATACC 87

(2) INFORMATION FOR SEQ ID NO:29:

(i) SEQUENCE CHARACTERISTICS:
(A) LENGTH: 87 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:29:

CAAAGTAATG AGGATCCTCG GACGTTCGGT GGAGGCACCA AGGTGGAAGA GAAACGTAAG 60

TGCACTTTCC TCGAGGTCGA CTTTTTT 87

(2) INFORMATION FOR SEQ ID NO:30:

(i) SEQUENCE CHARACTERISTICS:
(A) LENGTH: 99 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:30:

AAAAAAGTCG ACCTCGAGGA AAGTGCACTT ACGTTTCTCT TCCACCTTGG TGCCTCCACC 60

GAACGTCCGA GGATCCTCAT TACTTTGCTG ACAGTAATA 99

(2) INFORMATION FOR SEQ ID NO:31:

(i) SEQUENCE CHARACTERISTICS:
(A) LENGTH: 87 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:31:

AAAAAAGAAT TCGAGCTCTT TTCTGATAAC GTTGTCCTTC TGTTTCTTGC AGGTGTCCAG 60

TGTCAGGTCC AACTTCAGCA GCCTGGG 87

(2) INFORMATION FOR SEQ ID NO:32:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:32:

AAAAAAGTCG ACTGTGAGGA CTCACCTGAG GAGACGGTGA CTGAGGTGCC T 51

(2) INFORMATION FOR SEQ ID NO:33:

(i) SEQUENCE CHARACTERISTICS:
(A) LENGTH: 96 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:33:

AAAAAAGAAT TCAAGCTTTC CTGACTACAT GAGTGCATTT CTGTTTTATT TCCAATTTCA 60

GATACCACCG GAGACATTGT GCTAACACAA TCTCCA 96

(2) INFORMATION FOR SEQ ID NO:34:

(i) SEQUENCE CHARACTERISTICS:
(A) LENGTH: 54 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:34:

AAAAAAGTCG ACCTCGAGAT CACTTACGTT TGATTTCCAG CTTGGTGCCT CCAC 54

## Claims

1. A method for producing a humanized monoclonal antibody by utilizing a process of comparative model building comprising:
   a) selecting a monoclonal antibody to be humanized;
   b) searching computer databanks for protein crystal structures that demonstrate greater than 50 percent sequence homology to the variable region of said antibody to produce a structural template;
   c) determining the structure of the complementarity determining region, or CDR, loops and assigning the loops to canonical loop conformations;
   d) determining the framework residues which are crucial to the conformation of the CDR loops;
   e) replacing the CDR loops of the structural templates with canonical CDR backbone templates using interactive computer graphics;
   f) searching computer databanks to extract initial backbone approximations for each loop for non-canonical CDR loops;
   g) replacing all non-conserved amino-acid side chains in similar positions on said antibody and on the computer model with human amino acid residues using interactive computer graphics to produce a model having a combination of backbone fragments of different antibodies with replaced side chains;
   h) solvating the models with a water layer corresponding to about 7 angstroms;
   i) refining the structure with an energy minimization protocol to produce a structure wherein all atoms of the system are freely mobile;
   j) searching computer databanks to find homologous human sequences for the variable light and variable heavy chains of the antibody;
   k) combining the sequences found in (j) to obtain human templates;
   l) comparing the structural template of (a) with the human templates of (k) and selecting a human template with variable regions having greater than 50 percent sequence identity with the structural template;
   m) determining the CDR loops of the human template selected in (l);
   n) replacing the CDR loop region of the selected human template with the analogous sequences from the antibody to produce a Phase 1 humanized sequence;
   o) superimposing the models of the antibody and the Phase 1 humanized sequence to compare the binding site regions;
   p) identifying by the comparison in (o) all amino acids in the framework residues and CDR junction residues that interact with the antibody CDR loops that can be important to the structural integrity of the antibody binding site;
   q) reinserting into the Phase 1 humanized sequence all amino acid residues identified in (p) to be different from those in the antibody, and refining the resultant structure with an energy minimization protocol to produce a Phase II humanized sequence;
   r) refining the Phase II humanized sequence using iterative conformational search protocols on all regions of the binding site and by analysis of the binding site to determine which regions of the CDR surface or residues at the CDR -framework junction are not likely to involve antigen binding; and
   s) replacing the amino acids in the non-antigen binding regions of the binding site with amino acid residues corresponding to the human residues to produce a humanized monoclonal antibody.

2. The method of Claim 1 wherein the monoclonal antibody is a murine antibody.

3. The method of Claim 2 wherein the monoclonal antibody is an anti-CD18 monoclonal antibody.

4. The method of Claim 3 wherein the monoclonal antibody is 60.3

5. A humanized monoclonal antibody having the structural and binding characteristics of the anti- CD18 monoclonal antibody 60.3

6. The humanized monoclonal antibody of Claim 5 wherein the amino acid at position 5O in Figure 2 is changed from Arg to Asp.

7. The humanized monoclonal antibody of Claim 6, wherein the amino acid at position 54 is changed from Leu to Arg and the amino acid at position 55 is changed from Glu to Ala.

8. The humanized monoclonal antibody of Claim 7 wherein the amino acid at position 68 is changed from Arg to Gly.

## 60.3 Heavy Chain Sequences

| Region | Kabat # | hVhI/ Jh4 | 21-2 'CL | h60.3 template | h60.3 Phase IV | h60.3 Phase II/III | h60.3 Phase I | m60.3 | | Region |
|---|---|---|---|---|---|---|---|---|---|---|
| | 1 | Gln | Gln | Gln | Gln | Gln | Gln | Gln | | |
| | 2 | Val | Val | Val | Val | Val | Val | Val | | |
| | 3 | Gln | Gln | Gln | Gln | Gln | Gln | Gln | | |
| | 4 | Leu | Leu | Leu | Leu | Leu | Leu | Leu | | |
| | 5 | Val | Val | Val | Val | Val | Val | Gln | | |
| | 6 | Gln | Gln | Gln | Gln | Gln | Gln | Gln | | |
| | 7 | Ser | Ser | Ser | Ser | Ser | Ser | Pro | | |
| | 8 | Gly | Gly | Gly | Gly | Gly | Gly | Gly | | |
| | 9 | Ala | Ala | Ala | Ala | Ala | Ala | Pro | | |
| | 10 | Glu | Glu | Glu | Glu | Glu | Glu | Asp | | |
| | 11 | Val | Val | Val | Val | Val | Val | Leu | | |
| | 12 | Lys | Lys | Lys | Lys | Lys | Lys | Leu | | |
| | 13 | Lys | Lys | Lys | Lys | Lys | Lys | Lys | | |
| | 14 | Pro | Pro | Pro | Pro | Pro | Pro | Pro | | |
| FR 1 | 15 | Gly | Gly | Gly | Gly | Gly | Gly | Gly | | FR 1 |
| | 16 | Ala | Ala | Ala | Ala | Ala | Ala | Ala | | |
| | 17 | Ser | Ser | Ser | Ser | Ser | Ser | Pro | | |
| | 18 | Val | Val | Val | Val | Val | Val | Val | | |
| | 19 | Lys | Lys | Lys | Lys | Lys | Lys | Lys | | |
| | 20 | Val | Val | Val | Val | Val | Val | Leu | | |
| | 21 | Ser | Ser | Ser | Ser | Ser | Ser | Ser | | |
| | 22 | Cys | Cys | Cys | Cys | Cys | Cys | Cys | | |
| | 23 | Lys | Lys | Lys | Lys | Lys | Lys | Lys | | |
| | 24 | Ala | Ala | Ala | Ala | Ala | Ala | Ala | | |
| | 25 | Ser | Ser | Ser | Ser | Ser | Ser | Ser | | |
| | 26 | Gly | Gly | Gly | Gly | Gly | Gly | Gly | * H1 | |
| | 27 | Tyr | Tyr | Tyr | Tyr | Tyr | Tyr | Tyr | * H1 | |
| | 28 | Thr | Thr | Thr | Thr | Thr | Thr | Thr | | |
| | 29 | Phe | Phe | Phe | Phe | Phe | Phe | Phe | * H1 | H1 |
| | 30 | Thr | Thr | Thr | Thr | Thr | Thr | Thr | | |
| | 31 | Ser | Ser | Asn | Asn | Asp | Asp | Asp | | |
| | 32 | Tyr | Tyr | Tyr | Tyr | Tyr | Tyr | Tyr | | |
| HV 1 | 33 | Ala | Tyr | Tyr | Tyr | Trp | Tyr | Trp | | |
| | 34 | Ile | Met | Met | Met | Met | Met | Met | * H1 | |
| | 35 | Ser | His | His | His | Asn | His | Asn | | |
| | 36 | Trp | Trp | Trp | Trp | Trp | Trp | Trp | | |
| | 37 | Val | Val | Val | Val | Val | Val | Val | | |
| | 38 | Arg | Arg | Arg | Arg | Arg | Arg | Lys | | |
| | 39 | Gln | Gln | Gln | Gln | Gln | Gln | Gln | | |
| | 40 | Ala | Ala | Ala | Ala | Ala | Ala | Arg | | |
| FR 2 | 41 | Pro | Pro | Pro | Pro | Pro | Pro | Pro | | FR 2 |
| | 42 | Gly | Gly | Gly | Gly | Gly | Gly | Gly | | |
| | 43 | Gln | Gln | Gln | Gln | Gln | Gln | Arg | | |
| | 44 | Gly | Gly | Gly | Gly | Gly | Gly | Gly | | |

FIGURE 1 1/3

## 60.3 Heavy Chain Sequences

| | Kabat # | hVhI/ Jh4 | 21-2 *CL | h60.3 template | h60.3 Phase IV | h60.3 Phase II/III | h60.3 Phase I | m60.3 | |
|---|---|---|---|---|---|---|---|---|---|
| | 45 | Leu | Leu | Leu | Leu | Leu | Leu | Leu | |
| | 46 | Glu | Glu | Glu | Glu | Glu | Glu | Glu | |
| | 47 | Trp | Trp | Trp | Trp | Trp | Trp | Trp | |
| | 48 | Met | Met | Met | Met | Met | Met | Ile | |
| | 49 | Gly | Gly | Gly | Gly | Gly | Gly | Gly | |
| | 50 | Trp | Ile | Ile | Arg | Arg | Ile | Arg | |
| | 51 | Ile | Ile | Ile | Ile | Ile | Ile | Ile | |
| | 52 | Asn | Asn | Asn | Asp | Asp | Asp | Asp | |
| | 52a | Pro | Pro | Pro | Pro | Pro | Pro | Pro | |
| | 53 | Gly | Ser | Ser | Ser | Ser | Ser | Ser | H2 |
| | 54 | Asn | Gly | Gly | Asp | Asp | Asp | Asp | |
| | 55 | Gly | Gly | Asn | Ser | Ser | Ser | Ser | |
| | 56 | Asp | Ser | Ser | Glu | Glu | Ser | Glu | |
| HV 2 | 57 | Thr | Thr | Thr | Thr | Thr | Thr | Thr | |
| | 58 | Asn | Ser | Asn | His | His | Asn | His | |
| | 59 | Tyr | Tyr | Tyr | Tyr | Tyr | Tyr | Tyr | |
| | 60 | Ala | Ala | Ala | Asn | Asn | Ala | Asn | |
| | 61 | Gln | Gln | Gln | Gln | Gln | Gln | Gln | |
| | 62 | Lys | Lys | Lys | Lys | Lys | Lys | Lys | |
| | 63 | Phe | Phe | Phe | Phe | Phe | Phe | Phe | |
| | 64 | Gln | Gln | Gln | Gln | Gln | Gln | Lys | |
| | 65 | Gly | Gly | Gly | Gly | Gly | Gly | Asp | |
| | 66 | Arg | Arg | Arg | Arg | Arg | Arg | Lys | |
| | 67 | Val | Val | Val | Val | Val | Val | Ala | |
| | 68 | Thr | Thr | Thr | Thr | Thr | Thr | Thr | |
| | 69 | Ile | Met | Met | Met | Met | Met | Leu | |
| | 70 | Thr | Thr | Thr | Thr | Thr | Thr | Thr | |
| | 71 | Ala | Arg | Arg | Val | Val * | Val | Val | * H2 |
| | 72 | Asp | Asp | Asp | Asp | Asp | Asp | Asp | |
| | 73 | Thr | Thr | Thr | Thr | Thr | Thr | Lys | |
| | 74 | Ser | Ser | Ser | Ser | Ser | Ser | Ser | |
| | 75 | Thr | Thr | Thr | Thr | Thr | Thr | Ser | |
| | 76 | Ser | Ser | Ser | Ser | Ser | Ser | Ser | |
| | 77 | Thr | Thr | Thr | Thr | Thr | Thr | Thr | |
| | 78 | Ala | Val | Val | Val | Val | Val | Ala | |
| | 79 | Tyr | Tyr | Tyr | Tyr | Tyr | Tyr | Tyr | |
| | 80 | Met | Met | Met | Met | Met | Met | Ile | |
| | 81 | Glu | Glu | Glu | Glu | Glu | Glu | Gln | |
| FR 3 | 82 | Leu | Leu | Leu | Leu | Leu | Leu | Leu | FR 3 |
| | 82a | Ser | Ser | Ser | Ser | Ser | Ser | Ser | |
| | 82b | Ser | Ser | Ser | Ser | Ser | Ser | Ser | |
| | 82c | Leu | Leu | Leu | Leu | Leu | Leu | Leu | |
| | 83 | Arg | Arg | Arg | Arg | Arg | Arg | Thr | |
| | 84 | Ser | Ser | Ser | Ser | Ser | Ser | Ser | |

FIGURE 1 2/3

## 60.3 Heavy Chain Sequences

| | Kabat # | hVhI/ Jh4 | 21-2 'CL | h60.3 template | h60.3 Phase IV | h60.3 Phase II/III | h60.3 Phase I | m60.3 | |
|---|---|---|---|---|---|---|---|---|---|
| | 85 | Glu | Glu | Glu | Glu | Glu | Glu | Glu | |
| | 86 | Asp | Asp | Asp | Asp | Asp | Asp | Asp | |
| | 87 | Thr | Thr | Thr | Thr | Thr | Thr | Ser | |
| | 88 | Ala | Ala | Ala | Ala | Ala | Ala | Ala | |
| | 89 | Val | Val | Val | Val | Val | Val | Val | |
| | 90 | Tyr | Tyr | Tyr | Tyr | Tyr | Tyr | Tyr | |
| | 91 | Tyr | Tyr | Tyr | Tyr | Tyr | Tyr | Tyr | |
| | 92 | Cys | Cys | Cys | Cys | Cys | Cys | Cys | |
| | 93 | Ala | Ala | Ala | Ala | Ala | Ala | Ala | |
| | 94 | Arg | Arg | Arg | Arg | Arg | Arg | Arg | * H1 |
| | 95 | | | Glu | Gly | Gly | Gly | Gly | |
| | 96 | | | Lys | | | | | |
| | 97 | | | Leu | | | | | |
| | 98 | | | Ala | | | | | |
| | 99 | | | Thr | | | | | |
| | 100 | | | Thr | | | | | |
| | 100a | | | Ile | | | | | |
| | 100b | | | Phe | | | | | |
| HV 3 | 100c | | | Gly | Gly | Gly | Gly | Gly | |
| | 100d | | | Val | Arg | Arg | Arg | Arg | H3 |
| | 100e | | | Leu | Leu | Leu | Leu | Leu | |
| | 100f | | | Ile | Gly | Gly | Gly | Gly | |
| | 100g | | | Ile | Ser | Ser | Ser | Ser | |
| | 100h | | | Thr | Phe | Phe | Phe | Phe | |
| | 100i | Tyr | | Gly | Ala | Ala | Ala | Ala | |
| | 100j | Phe | | Met | Met | Met | Met | Met | |
| | 101 | Asp | | Asp | Asp | Asp | Asp | Asp | |
| | 102 | Tyr | | Tyr | Tyr | Tyr | Tyr | Tyr | |
| | 103 | Trp | | Trp | Trp | Trp | Trp | Trp | |
| | 104 | Gly | | Gly | Gly | Gly | Gly | Gly | |
| | 105 | Gln | | Gln | Gln | Gln | Gln | Gln | |
| | 106 | Gly | | Gly | Gly | Gly | Gly | Gly | |
| | 107 | Thr | | Thr | Thr | Thr | Thr | Thr | |
| FR 4 | 108 | Leu | | Leu | Leu | Leu | Leu | Ser | FR 4 |
| | 109 | Val | | Val | Val | Val | Val | Val | |
| | 110 | Thr | | Thr | Thr | Thr | Thr | Thr | |
| | 111 | Val | | Val | Val | Val | Val | Val | |
| | 112 | Ser | | Ser | Ser | Ser | Ser | Ser | |
| | 113 | Ser | | Ser | Ser | Ser | Ser | Ser | |

* The h60.3 heavy chain was made with the sequence shown in the Phase II/III column, with the following exception:

H71: Arg instead of Val

FIGURE 1 3/3

## 60.3 Light Chain Sequences

| | Kabat # | hVkIII/Jk | h60.3 template | h60.3 Phase IV | h60.3 Phase II/III | h60.3 Phase I | m60.3 | | |
|---|---|---|---|---|---|---|---|---|---|
| | 1 | Glu | Glu | Glu | Glu | Glu | · Asp | | |
| | 2 | Ile | Ile | Ile | Ile | Ile | Ile | *L1 | |
| | 3 | Val | Val | Val | Val | Val | Val | | |
| | 4 | Leu | Leu | Leu | Leu | Leu | Leu | | |
| | 5 | Thr | Thr | Thr | Thr | Thr | Thr | | |
| | 6 | Gln | Gln | Gln | Gln | Gln | Gln | | |
| | 7 | Ser | Ser | Ser | Ser | Ser | Ser | | |
| | 8 | Pro | Pro | Pro | Pro | Pro | Pro | | |
| | 9 | Gly | Ala | Ala | Ala | Ala | Ala | | |
| FR 1 | 10 | Thr | Thr | Thr | Thr | Thr | Ser | | FR 1 |
| | 11 | Leu | Leu | Leu | Leu | Leu | Leu | | |
| | 12 | Ser | Ser | Ser | Ser | Ser | Ala | | |
| | 13 | Leu | Leu | Leu | Leu | Leu | Val | | |
| | 14 | Ser | Ser | Ser | Ser | Ser | Ser | | |
| | 15 | Pro | Pro | Pro | Pro | Pro | Leu | | |
| | 16 | Gly | Gly | Gly | Gly | Gly | Gly | | |
| | 17 | Glu | Glu | Glu | Glu | Glu | Gln | | |
| | 18 | Arg | Arg | Arg | Arg | Arg | Arg | | |
| | 19 | Ala | Ala | Ala | Ala | Ala | Ala | | |
| | 20 | Thr | Thr | Thr | Thr | Thr | Thr | | |
| | 21 | Leu | Leu | Leu | Leu | Leu | Ile | | |
| | 22 | Ser | Ser | Ser | Ser | Ser | Ser | | |
| | 23 | Cys | Cys | Cys | Cys | Cys | Cys | | |
| | 24 | | Arg | Arg | Arg | Arg | Arg | | |
| | 25 | | Ala | Ala | Ala | Ala | Ala | *L1 | |
| | 26 | | Ser | Ser | Ser | Ser | Ser | | |
| | 27 | | Gln | Glu | Glu | Glu | Glu | | |
| | 28 | | Ser | Ser | Ser | Ser | Ser | | |
| | 29 | | Val | Val | Val | Val | Val | *L1 | |
| | 30 | | Ser | Asp | Asp | Asp | Asp | | |
| HV 1 | 31 | | Ser | Ser | Ser | Ser | Ser | | L1 |
| | 31a | | | Tyr | Tyr | Tyr | Tyr | | |
| | 31b | | | Gly | Gly | Gly | Gly | | |
| | 31c | | | Asn | Asn | Asn | Asn | | |
| | 31d | | | Ser | Ser | Ser | Ser | | |
| | 32 | | Tyr | Phe | Phe | Phe | Phe | | |
| | 33 | | Leu | Met | Met | Met | Met | *L1 | |
| | 34 | | Ala | His | His | Ala | His | | |
| | 35 | Trp | Trp | Trp | Trp | Trp | Trp | | |
| | 36 | Tyr | Tyr | Tyr | Tyr | Tyr | Tyr | | |
| | 37 | Gln | Gln | Gln | Gln | Gln | Gln | | |
| | 38 | Gln | Gln | Gln | Gln | Gln | Gln | | |
| | 39 | Lys | Lys | Lys | Lys | Lys | Lys | | |
| | 40 | Pro | Pro | Pro | Pro | Pro | Pro | | |
| FR 2 | 41 | Gly | Gly | Gly | Gly | Gly | Gly | | FR 2 |
| | 42 | Gln | Gln | Gln | Gln | Gln | Gln | | |
| | 43 | Ala | Ala | Ala | Ala | Ala | Pro | | |
| | 44 | Pro | Pro | Pro | Pro | Pro | Pro | | |
| | 45 | Arg | Arg | Arg | Arg | Arg | Lys | | |
| | 46 | Leu | Leu | Leu | Leu | Leu | Leu | | |

FIGURE 2 1/3

## 60.3 Light Chain Sequences

| Region | Kabat # | hVkIII/ Jk | h60.3 template | h60.3 Phase IV | h60.3 Phase II/III | h60.3 Phase I | m60.3 | |
|---|---|---|---|---|---|---|---|---|
| | 47 | Leu | Leu | Leu | Leu | Leu | Leu | |
| | 48 | Ile | Ile | Ile | Ile | Ile | Ile | *L2 |
| | 49 | Tyr | Tyr | Tyr | Tyr | Tyr | Tyr | |
| HV 2 | 50 | | Asp | Asp | Arg | Arg | Arg | L2 |
| HV 2 | 51 | | Ala | Ala | Ala | Ala | Ala | L2 |
| HV 2 | 52 | | Ser | Ser | Ser | Ser | Ser | L2 |
| HV 2 | 53 | | Asn | Asn | Asn | Asn | Asn | L2 |
| HV 2 | 54 | | Arg | Arg | Leu | Arg | Leu | |
| HV 2 | 55 | | Ala | Ala | Glu | Ala | Glu | |
| HV 2 | 56 | | Thr | Thr | Thr | Thr | Ser | |
| FR 3 | 57 | Gly | Gly | Gly | Gly | Gly | Gly | |
| FR 3 | 58 | Ile | Ile | Ile | Ile | Ile | Ile | |
| FR 3 | 59 | Pro | Pro | Pro | Pro | Pro | Pro | |
| FR 3 | 60 | Asp | Ala | Ala | Ala | Ala | Ala | |
| FR 3 | 61 | Arg | Arg | Arg | Arg | Arg | Arg | |
| FR 3 | 62 | Phe | Phe | Phe | Phe | Phe | Phe | |
| FR 3 | 63 | Ser | Ser | Ser | Ser | Ser | Ser | |
| FR 3 | 64 | Gly | Gly | Gly | Gly | Gly | Gly | *L2 |
| FR 3 | 65 | Ser | Ser | Ser | Ser | Ser | Ser | |
| FR 3 | 66 | Gly | Gly | Gly | Gly | Gly | Gly | |
| FR 3 | 67 | Ser | Ser | Ser | Ser | Ser | Ser | FR 3 |
| FR 3 | 68 | Gly | Gly | Gly | Gly* | Gly | Arg | |
| FR 3 | 69 | Thr | Thr | Thr | Thr | Thr | Thr | |
| FR 3 | 70 | Asp | Asp | Asp | Asp | Asp | Asp | |
| FR 3 | 71 | Phe | Phe | Phe | Phe | Phe | Phe | *L1 |
| FR 3 | 72 | Thr | Thr | Thr | Thr | Thr | Thr | |
| FR 3 | 73 | Leu | Leu | Leu | Leu | Leu | Leu | |
| FR 3 | 74 | Thr | Thr | Thr | Thr | Thr | Thr | |
| FR 3 | 75 | Ile | Ile | Ile | Ile* | Ile | Ile | |
| FR 3 | 76 | Ser | Ser | Ser | Ser | Ser | Asn | |
| FR 3 | 77 | Arg | Ser | Ser | Ser | Ser | Pro | |
| FR 3 | 78 | Leu | Leu | Leu | Leu | Leu | Val | |
| FR 3 | 79 | Glu | Glu | Glu | Glu | Glu | Glu | |
| FR 3 | 80 | Pro | Pro | Pro | Pro | Pro | Ala | |
| FR 3 | 81 | Glu | Glu | Glu | Glu | Glu | Asp | |

FIGURE 2 2/3

60.3 Light Chain Sequences

| | Kabat # | hVkIII/ Jk | h60.3 template | h60.3 Phase IV | h60.3 Phase II/III | h60.3 Phase I | m60.3 | | |
|---|---|---|---|---|---|---|---|---|---|
| | 82 | Asp | Asp | Asp | Asp | Asp | Asp | | |
| | 83 | Phe | Phe | Phe | Phe | Phe | Val | | |
| | 84 | Ala | Ala | Ala | Ala | Ala | Ala | | |
| | 85 | Val | Val | Val | Val | Val | Thr | | |
| | 86 | Tyr | Tyr | Tyr | Tyr | Tyr | Tyr | | |
| | 87 | Tyr | Tyr | Tyr | Tyr | Tyr | Tyr | | |
| | 88 | Cys | Cys | Cys | Cys | Cys | Cys | | |
| | 89 | | Gln | Gln | Gln | Gln | Gln | | |
| | 90 | | Gln | Gln | Gln | Gln | Gln | *L3 | |
| | 91 | | Arg | Ser | Ser | Ser | Ser | | |
| | 92 | | Ser | Asn | Asn | Asn | Asn | | |
| HV3 | 93 | | Asn | Glu | Glu | Glu | Glu | | L3 |
| | 94 | | Trp | Asp | Asp | Asp | Asp | | |
| | 95 | | Pro | Pro | Pro | Pro | Pro | *L3 | |
| | 96 | Trp, Tyr, Phe, Leu, Ile | | Arg | Arg | Arg | Arg | | |
| | 97 | Thr | | Thr | Thr | Thr | Thr | | |
| | 98 | Phe | | Phe | Phe | Phe | Phe | | |
| | 99 | Gly | | Gly | Gly | Gly | Gly | | |
| | 100 | Gln, Pro, Gly | | Gly | Gly | Gly | Gly | | |
| | 101 | Gly | | Gly | Gly | Gly | Gly | | |
| FR 4 | 102 | Thr | | Thr | Thr | Thr | Thr | | FR 4 |
| | 103 | Lys, Arg | | Lys | Lys | Lys | Lys | | |
| | 104 | Val, Leu | | Leu * | Leu * | Leu | Leu | | |
| | 105 | Glu, Asp | | Glu | Glu | Glu | Glu | | |
| | 106 | Ile | | Ile * | Ile * | Ile | Ile | | |
| | 107 | Lys | | Lys | Lys | Lys | Lys | | |
| | 108 | Arg | | Arg | Arg | Arg | Arg | | |

* The h60.3 light chain was made with the sequence shown in the Phase II/III column, with the following exceptions:

| | | |
|---|---|---|
| L68: | Arg | instead of Gly |
| L75: | Tjr | instead of Ile |
| L104: | Val | instead of Leu |
| L106: | Glu | instead of Ile |

FIGURE 2 3/3

FIGURE 3

Oligonucleotide #1:

TTTTTTGAATTCAAGCTTTCCTGACTACATGAGTGCATTTCTGTTTTATTTCCAAT
TTCAGATACCACCGGAGAAATTGTGCTAACACAA

Oligonucleotide #2

AATTTCTCCGGTGGTATCTGAAATTGGAAATAAAACAGAAATGCACTCATGTAG
TCAGGAAAGCTTGAATTCAAAAAA

Oligonucleotide #3

TCTCCAGCTACATTGTCTTTGTCTCCAGGTGAGAGAGCCACTCTATCCTGCAGAG
CCAGTGAAAGTGTTGATAGTTATGGCAATAGT

Oligonucleotide #4

ACTATCAACACTTTCACTGGCTCTGCAGGATAGAGTGGCTCTCTCACCTGGAGAC
AAAGACAATGTAGCTGGAGATTGTGTTAGCAC

Oligonucleotide #5

TTTATGCACTGGTACCAGCAGAAACCAGGACAGGCACCAAGGCTCCTCATCTAT
CGTGCATCCAACCTAGAAACTGGTATCCCTGCC

Oligonucleotide #6

AGTTTCTAGGTTGGATGCACGATAGATGAGGAGCCTTGGTGCCTGTCCTGGTTTC
TGCTGGTACCAGTGCATAAAACTATTGCCATA

Oligonucleotide #7

AGGTTCAGTGGCAGTGGTTCTAGGACAGACTTCACTCTCACCTATTCTTCTCTAG
AGCCTGAAGATTTTGCAGTGTATTACTGTCAG

Oligonucleotide #8

CACTGCAAAATCTTCAGGCTCTAGAGAAGAATAGGTGAGAGTGAAGTCTGTCCT
AGAACCACTGCCACTGAACCTGGCAGGGATACC

Oligonucleotide #9

CAAAGTAATGAGGATCCTCGGACGTTCGGTGGAGGCACCAAGGTGGAAGAGAA
ACGTAAGTGCACTTTCCTCGAGGTCGACTTTTTT

Oligonucleotide #10

AAAAAAGTCGACCTCGAGGAAAGTGCACTTACGTTTCTCTTCCACCTTGGTGCCT
CCACCGAACGTCCGAGGATCCTCATTACTTTGCTGACAGTAATA

FIGURE 4

Oligonucleotide # 1

AAAAAAGAATTCGAGCTCTTCTGATAACGCTGTCCTTCTGTTTGCAGGTGTCCAG
TGTCAGGTCCAACTTGTCCAGTCCGG

Oligonucleotide #2

TTAACTTCGGCACCGGACTGGACAAGTTGGACCTGACACTGGACACCTGCAAAC
AGAAGGACAGCGTTATCAGAAGAGCTCGAATTCTTTTTT

Oligonucleotide #3
TGCCGAAGTTAAGAAGCCTGGCGCTTCTGTGAAGGTCTCCTGCAAGGCTTCTGG
CTACACCTTCACCGACTACTGGATGAACTGGGTTCG

Oligonucleotide #4

CCAGGTGCCTGTCGAACCCAGTTCATCCAGTAGTCGGTGAAGGTGTAGCCAGAA
GCCTTGCAGGAGACCTTCACAGAAGCGCCAGGCTTC

Oligonucleotide #5

ACAGGCACCTGGACAGGGCCTAGAGTGGATGGGAAGGATTGATCCTTCCGATAG
TGAAACTCACTACCATCAGAAGTTCCAGGGTAGGGT

Oligonucleotide #6

CGGGTCATTGTTACCCTACCCTGGAACTTCTGATTGTAGTGAGTTTCACTATCGG
AAGGATCAATCCTTCCCATCCACTCTAGGCCCTGT

Oligonucleotide #7

AACAATGACCCGAGACACATCCACCAGCACAGTCTACATGGAACTCAGCAGCCT
GCGATCTGAGGACACCGCAGTCTATTACTGTGCACG

Oligonucleotide #8

AGCCGTCCACCTCGTGCACAGTAATAGACTGCGGCGTCCTCAGATCGCAGGCTG
CTGAGTTCCATGTAGACTGTGCTGGTGGATGTGTCT

Oligonucleotide #9

AGGTGGACGGCTCGGTTCCTTTGCTATGGACTACTGGGGTCAAGGCACCCTCGTC
ACCGTCTCCTCAGGTGATGCCTCACACTCGAGGTCGACTTTTTT

Oligonucleotide #10

AAAAAAGTCGACCTCGAGTGTGAGGACTCACCTGAGGAGACGGTGACGAGGGT
GCCTTGACCCCAGTAGTCCATAGCAAAGGAACCG

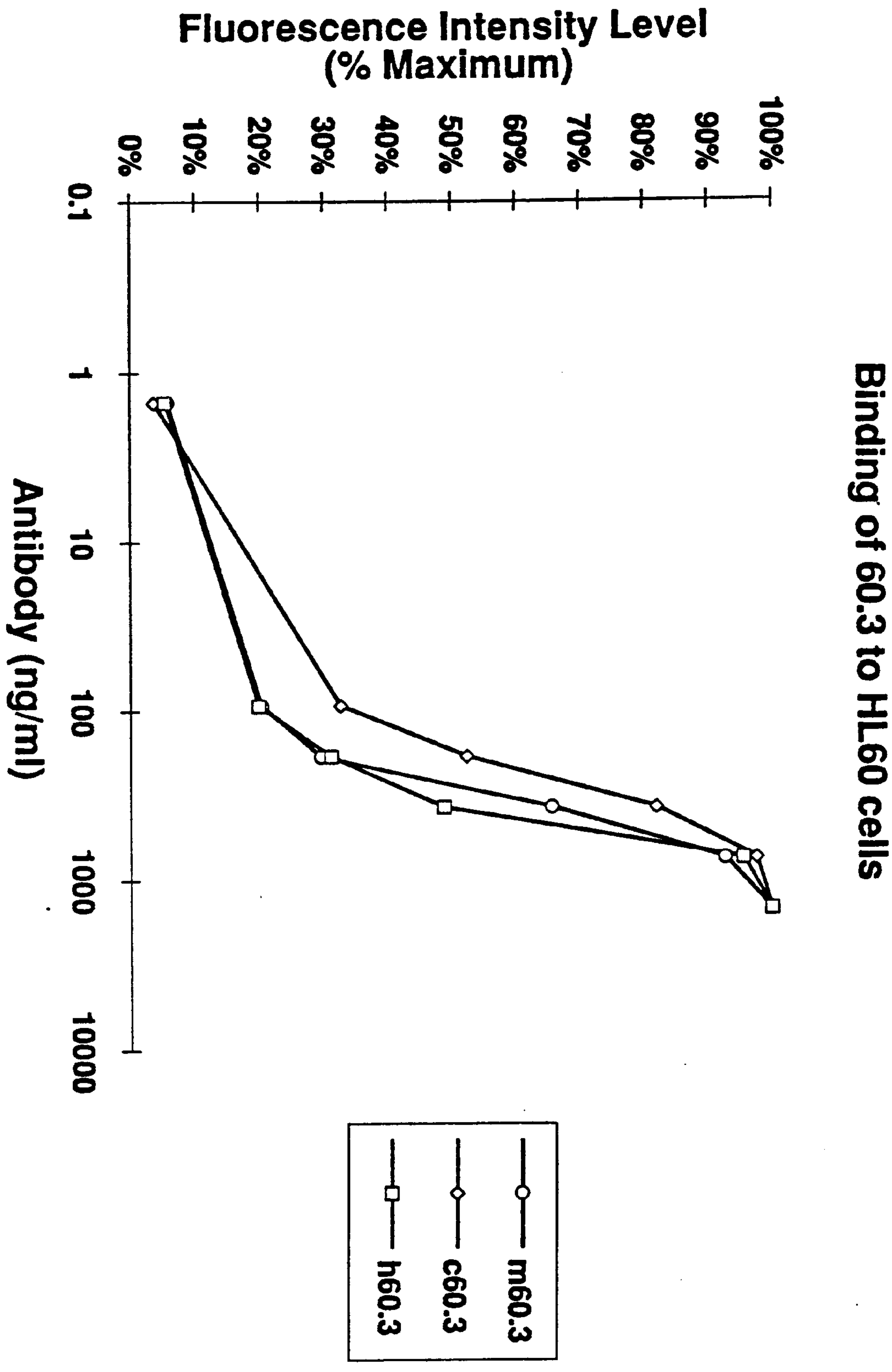

Binding of 60.3 to HL60 cells
Fluorescence Intensity Level (% Maximum)
0%
10%
20%
30%
40%
50%
60%
70%
80%
90%
100%
0.1
1
10
100
1000
10000
Antibody (ng/ml)
m60.3
c60.3
h60.3


FIGURE 5

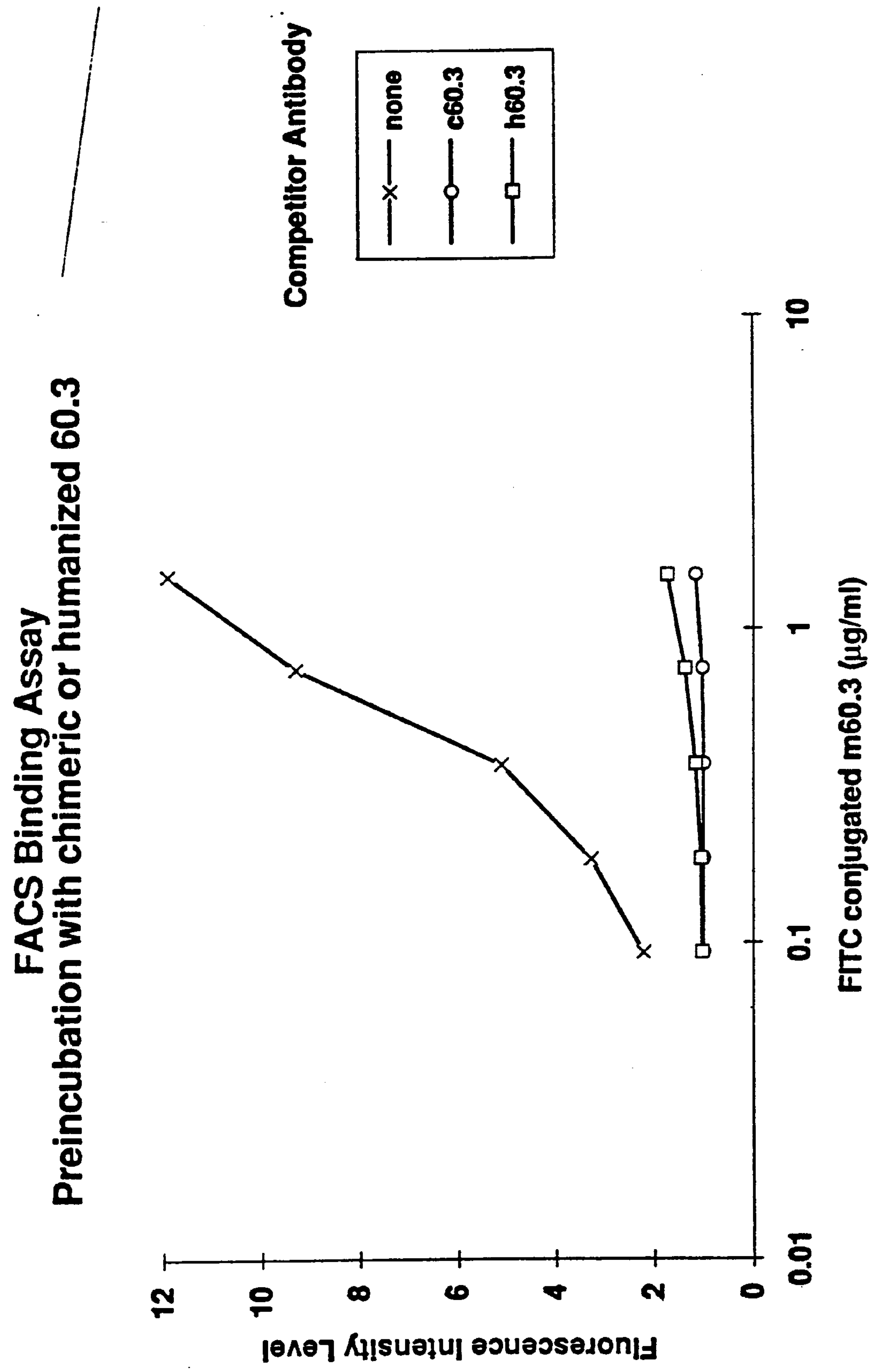
FACS Binding Assay
Preincubation with chimeric or humanized 60.3
Competitor Antibody
none
c60.3
h60.3
Fluorescence Intensity Level
0
2
4
6
8
10
12
0.01
0.1
1
10
FITC conjugated m60.3 (µg/ml)

FIGURE 6

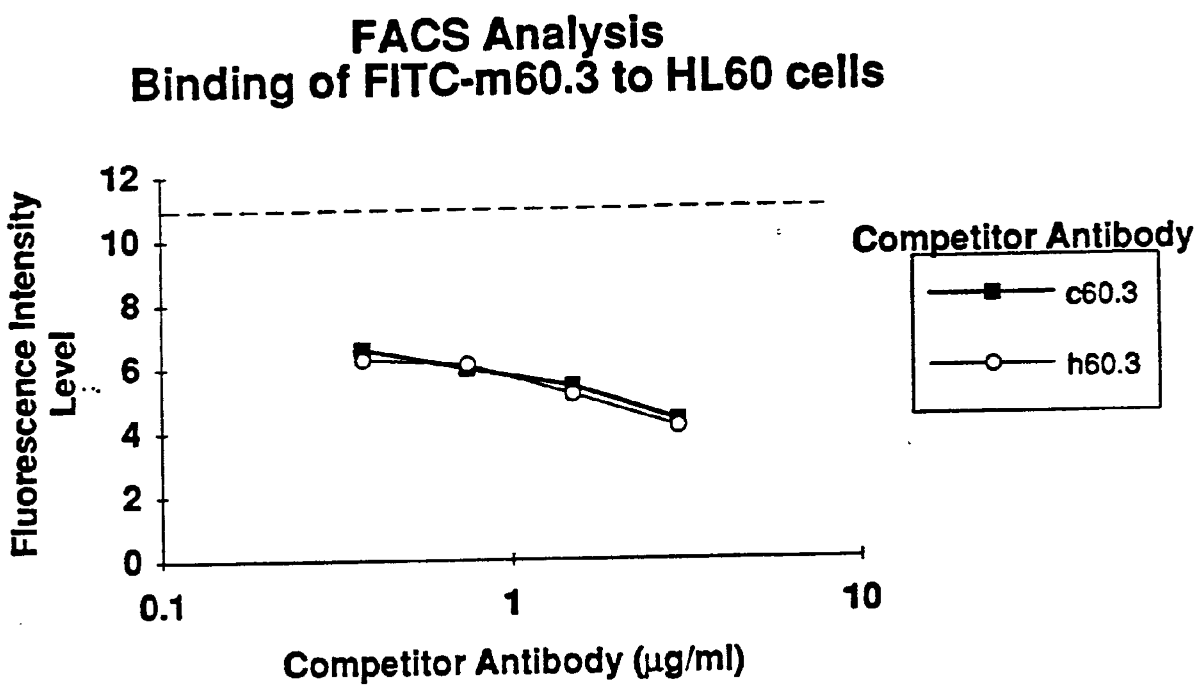
FACS Analysis
Binding of FITC-m60.3 to HL60 cells
Fluorescence Intensity Level
12
10
8
6
4
2
0
0.1
1
10
Competitor Antibody (µg/ml)
Competitor Antibody
c60.3
h60.3

FIGURE 7

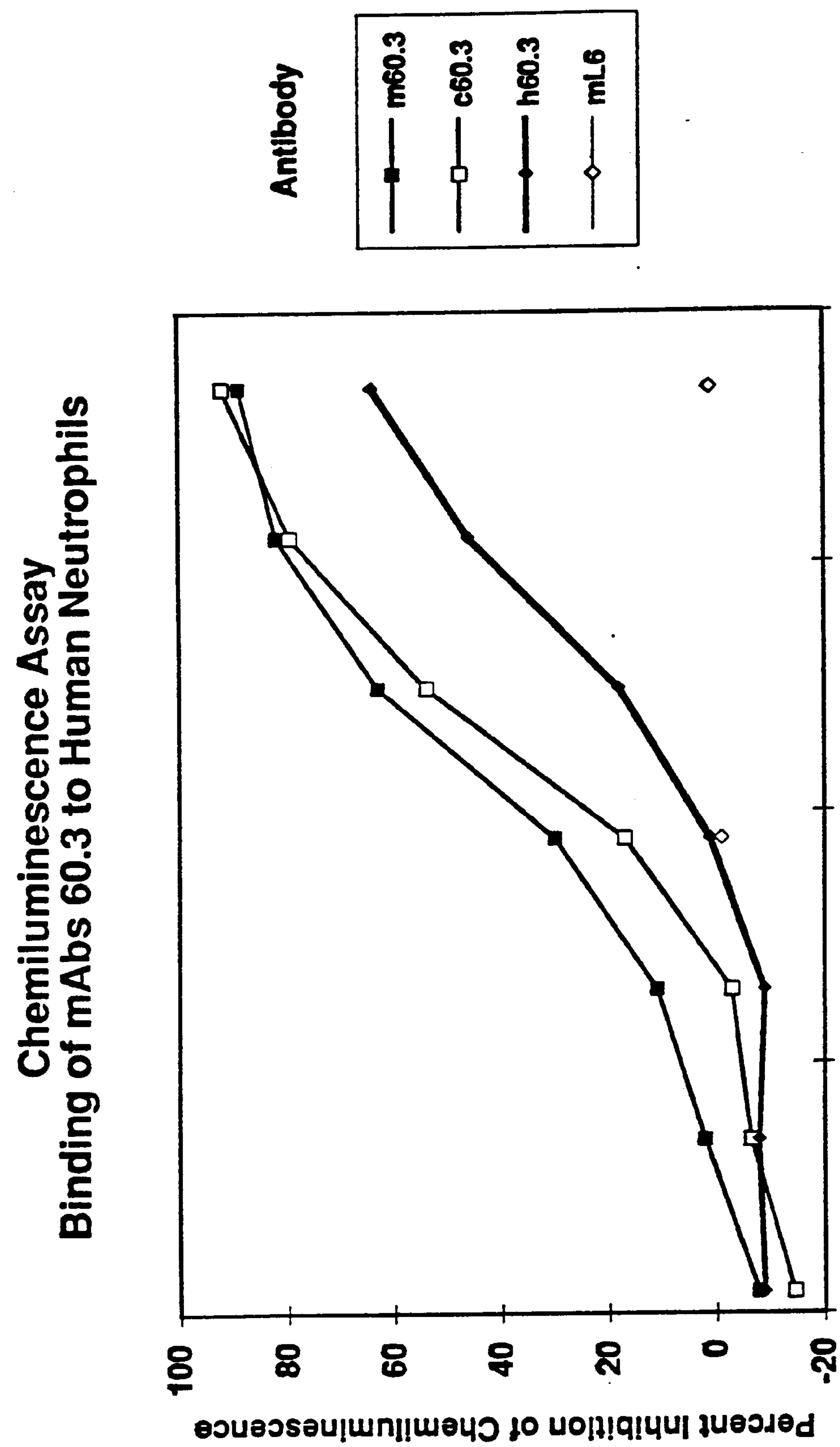
Chemiluminescence Assay
Binding of mAbs 60.3 to Human Neutrophils
Percent Inhibition of Chemiluminescence
100
80
60
40
20
0
-20
0.01
0.1
1
10
100
Antibody (µg/ml)
Antibody
m60.3
c60.3
h60.3
mL6

FIGURE 8

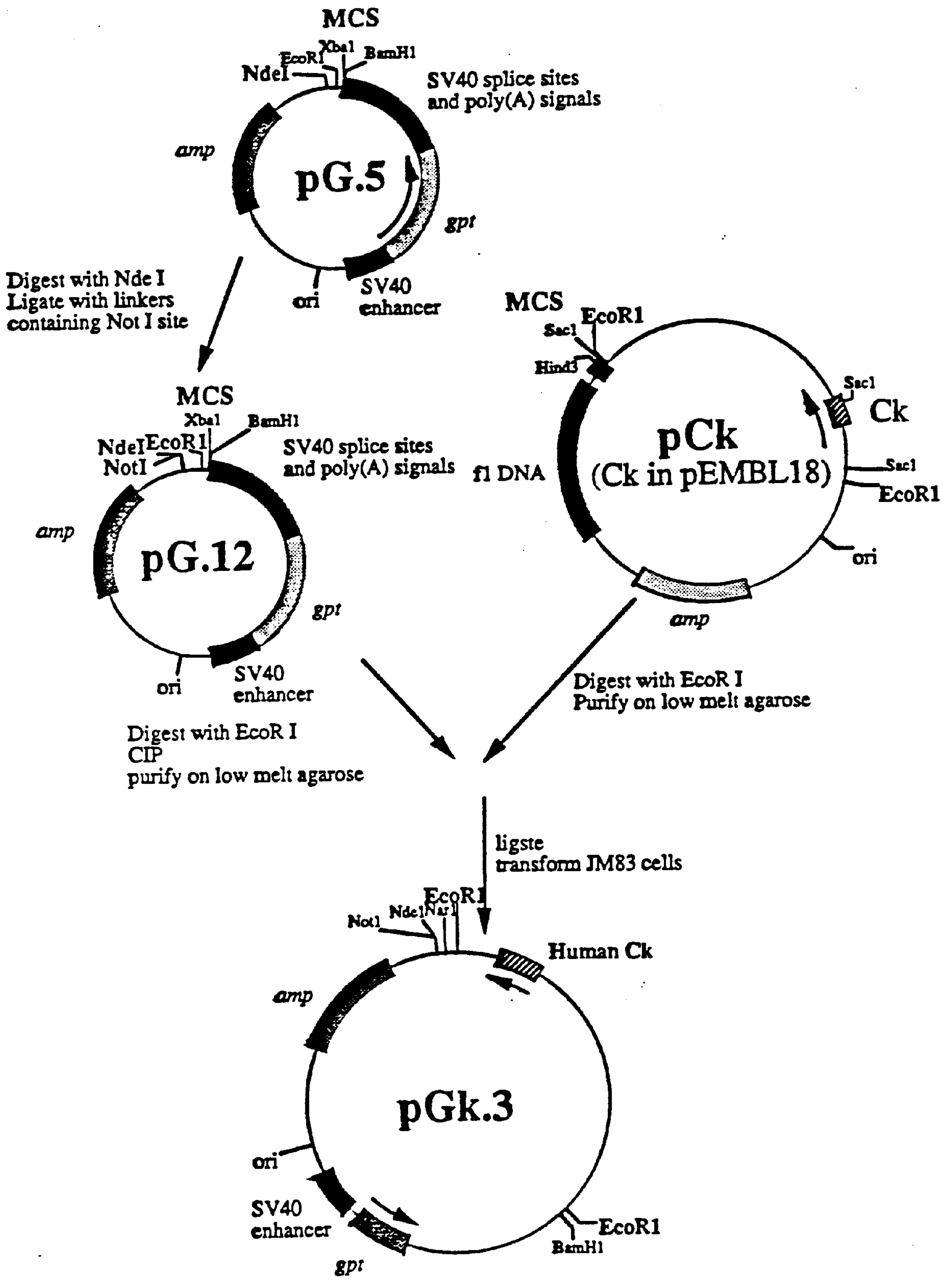
MCS
EcoR1
Xba1
BamH1
NdeI
SV40 splice sites
and poly(A) signals
amp
pG.5
gpt
ori
SV40
enhancer
Digest with Nde I
Ligate with linkers
containing Not I site
MCS
EcoR1
Sac1
Hind3
Sac1
Ck
pCk
(Ck in pEMBL18)
f1 DNA
Sac1
EcoR1
ori
amp
MCS
Xba1
BamH1
NdeI EcoR1
NotI
SV40 splice sites
and poly(A) signals
amp
pG.12
gpt
ori
SV40
enhancer
Digest with EcoR I
Purify on low melt agarose
Digest with EcoR I
CIP
purify on low melt agarose
ligste
transform JM83 cells
EcoR1
Not1
Nde1
Nar1
Human Ck
amp
pGk.3
ori
SV40
enhancer
gpt
EcoR1
BamH1

FIGURE 9 1/4

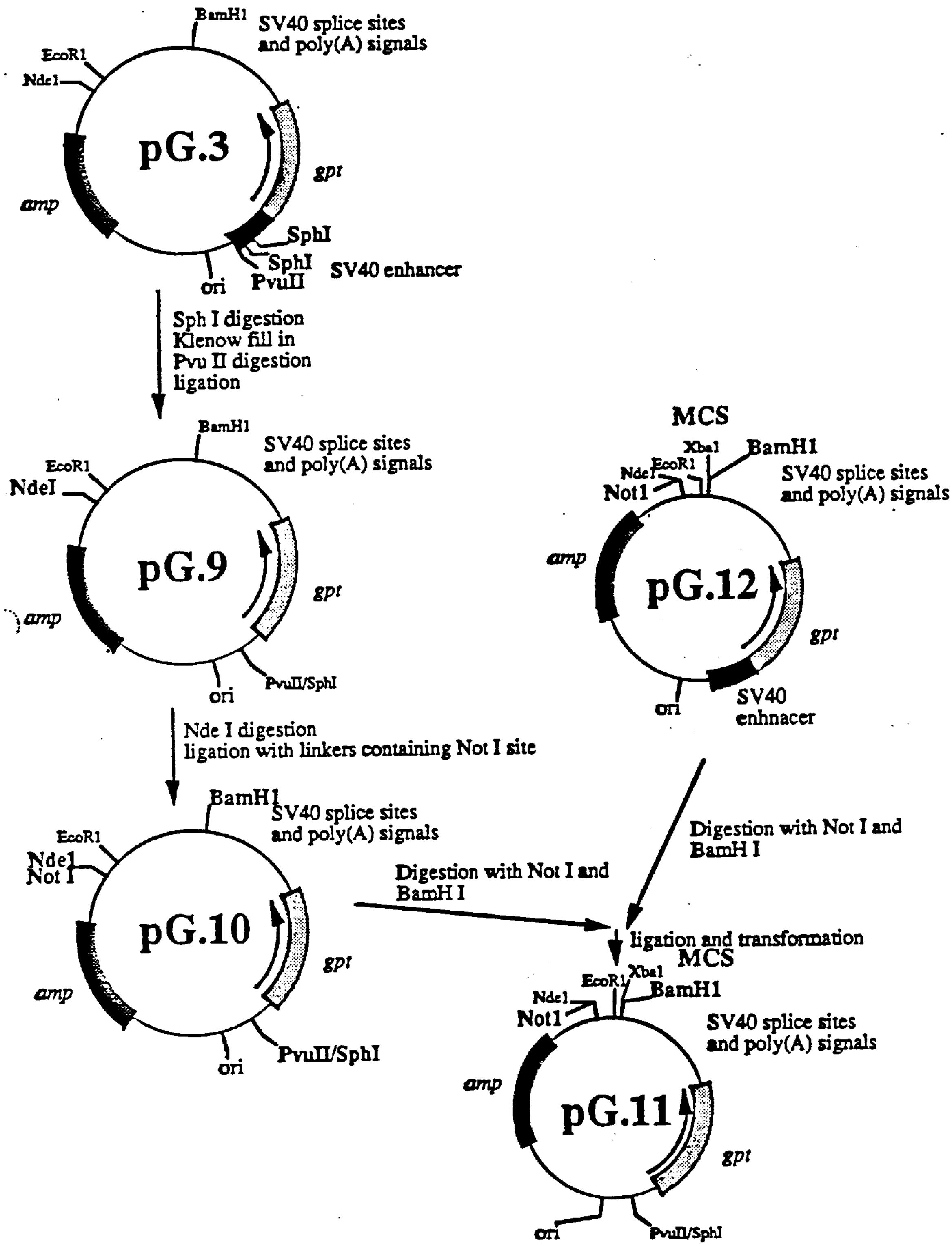
BamH1
SV40 splice sites
and poly(A) signals
EcoR1
Nde1
pG.3
gpt
amp
SphI
SphI
PvuII
SV40 enhancer
ori
Sph I digestion
Klenow fill in
Pvu II digestion
ligation
BamH1
SV40 splice sites
and poly(A) signals
EcoR1
NdeI
pG.9
gpt
amp
PvuII/SphI
ori
Nde I digestion
ligation with linkers containing Not I site
BamH1
SV40 splice sites
and poly(A) signals
EcoR1
Nde1
Not I
pG.10
gpt
amp
PvuII/SphI
ori
Digestion with Not I and
BamH I
MCS
Xba1
BamH1
Nde1
EcoR1
Not1
SV40 splice sites
and poly(A) signals
amp
pG.12
gpt
ori
SV40
enhnacer
Digestion with Not I and
BamH I
ligation and transformation
MCS
EcoR1
Xba1
BamH1
Nde1
Not1
SV40 splice sites
and poly(A) signals
amp
pG.11
gpt
ori
PvuII/SphI

FIGURE 9 2/4

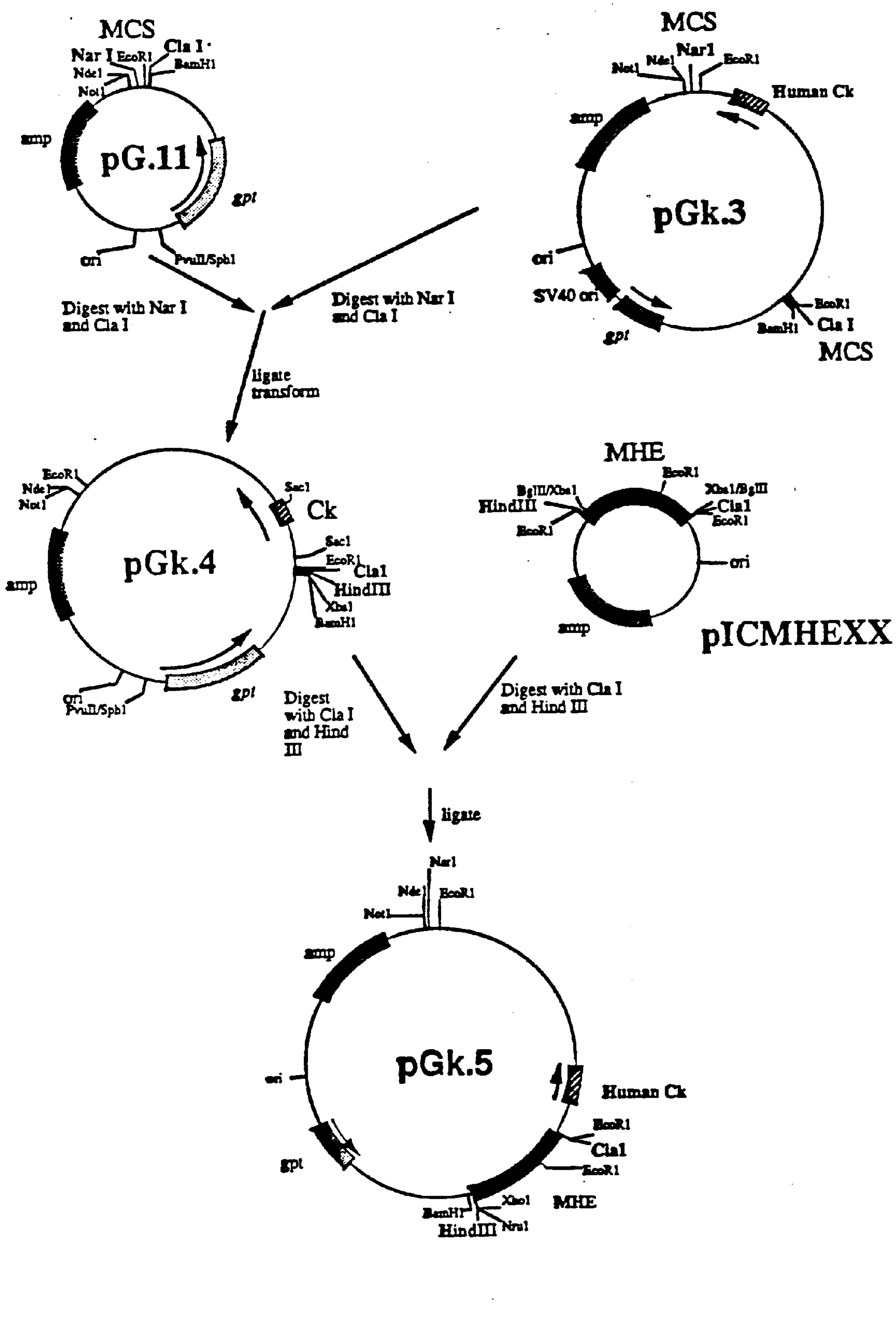
MCS
Nar I
EcoR1
Cla I
BamH1
Nde1
Not1
amp
pG.11
gpt
ori
PvuII/Sph1
MCS
Not1
Nde1
Nar1
EcoR1
Human Ck
amp
pGk.3
ori
SV40 ori
gpt
EcoR1
BamH1
Cla I
MCS
Digest with Nar I and Cla I
Digest with Nar I and Cla I
ligate transform
EcoR1
Nde1
Not1
Sac1
Ck
amp
pGk.4
Sac1
EcoR1
Cla1
HindIII
Xba1
BamH1
ori
PvuII/Sph1
gpt
MHE
EcoR1
BglII/Xba1
Xba1/BglII
HindIII
Cla1
EcoR1
EcoR1
ori
amp
pICMHEXX
Digest with Cla I and Hind III
Digest with Cla I and Hind III
ligate
Nar1
Nde1
EcoR1
Not1
amp
ori
pGk.5
Human Ck
EcoR1
Cla1
EcoR1
gpt
Xho1
MHE
BamH1
HindIII
Nru1

FIGURE 9 · 3/4

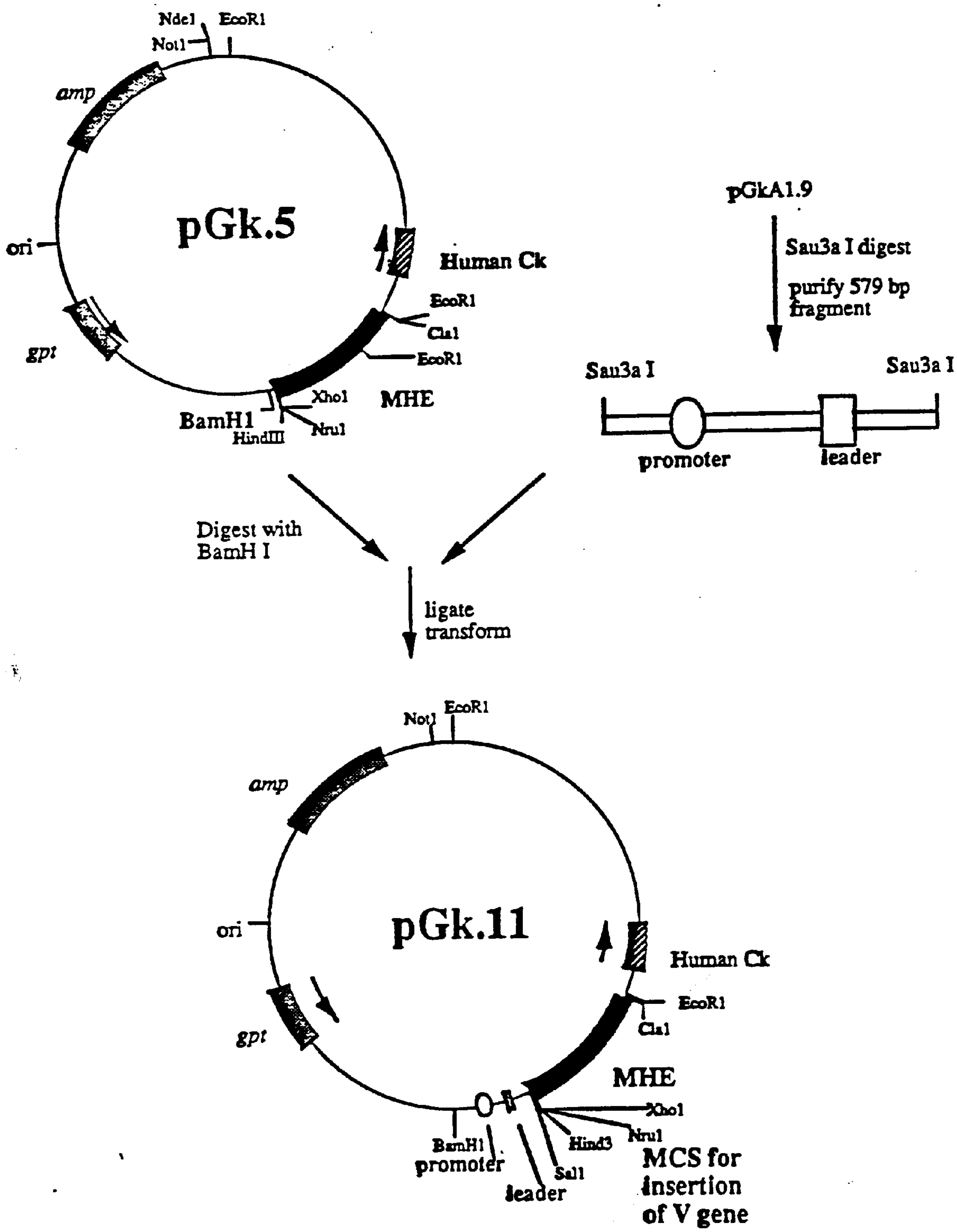
NdeI
EcoR1
NotI
amp
pGk.5
ori
Human Ck
EcoR1
ClaI
EcoR1
gpt
XhoI
MHE
BamH1
HindIII
NruI
pGkA1.9
Sau3a I digest
purify 579 bp fragment
Sau3a I
Sau3a I
promoter
leader
Digest with BamH I
ligate transform
NotI
EcoR1
amp
pGk.11
ori
Human Ck
EcoR1
ClaI
gpt
MHE
XhoI
NruI
BamH1
Hind3
promoter
SalI
leader
MCS for insertion of V gene

FIGURE 9 4/4

## pGk.11

```
   1 GAATTC---- ---------- ---------- ---------- -
  51 ---------- ---------- ---------- ---------- -
 101 ---------- ---------- ---------- ---------- -
 151 ---------- ---------- ---------- ---------- --
 201 ---------- ---------- ---------- ---------- --
 251 ---------- ---------- ---------- ---------- --
 301 ---------- ---------- ---------- ---------- ---
 351 ---------- ---------- ---------- ---------- ---
 401 ---------- ---------- ---------- ---------- ---     ---
 451 ---------- ---------- ---------- ---------- ----------
 501 ---------- ---------- ---------- ---------- ----------
 551 ---------- ---------- ---------- ---------- ----------
 601 ---------- ---------- ---------- ---------- ----------
 651 ---------- ---------- ---------- ---------- ----------
 701 ---------- ---------- ---------- ---------- ----------
 751 ---------- ---------- ---------- ---------- ----------
 801 ---------- ---------- ---------- ---------- ----------
 851 ---------- ---------- ---------- ---------- ----------
 901 ---------- ---------- ---------- ---------- ----------
 951 ---------- ---------- ---------- ---------- ----------
1001 ---------- ---------- ---------- ---------- ----------
1051 ---------- ---------- ---------- ---------- ----------
1101 ---------- ---------- ---------- ---------- ----------
1151 ---------- ---------- ---------- ---------- ----------
1201 ---------- ---------- ---------- ---------- ----------
1251 ---------- ---------- ---------- ---------- ----------
1301 ---------- ---------- ---------- ---------- ----------
1351 ---------- ---------- ---------- ---------- ----------
1401 ---------- ---------- ---------- ---------- ----------
1451 ---------- ---------- ---------- ---------- ----------
1501 ---------- ---------- ---------- ---------- ----------
1551 ---------- ---------- ---------- ---------- ----------
1601 ---------- ---------- ---------- ---------- ----------
1651 ---------- ---------- ---------- ---------- ----------
1701 ------GGCC CAGGGGACTG TGAGGACAGA AGGCTTGTGG GTTTGAGGGA
1751 GGACTGTCTT GCAGAGGATG ATAGGGTAAA ATAGAATGAA GGATGATTTT
1801 TATAAATGGT TACGTGCCTT AGGATGACTA CATATTTAGT CCCTTATAAG
1851 AGAAATTGAG TAGTTGGTAA AACAACAGAT AATAATTATT AAATGAGGAA
1901 AGAGAGAAAC CACAGGTGCA AAGATTCACT TTATTTATTC ATTCTCCTCC
1951 AACATTAGCA TAATTAAAGC CAAGGAGGAG GAGGGGGGTG AGGTGAAAGA
2001 TGAGCTGGAG GACCGCAATA GGGGTAGGTC CCCTGTGGAA AAAGGGTCAG
2051 AGGCCAAAGG ATGGGAGGGG GTCAGGCTGG AACTGAGGAG CAGGTGGGGG
2101 CACTTCTCCC TCTAACACTC TCCCCTGTTG AAGCTCTTTG TGACGGGCGA
2151 GCTCAGGCCC TGATGGGTGA CTTCGCAGGC GTAGACTTTG TGTTTCTCGT
2201 AGTCTGCTTT GCTCAGCGTC AGGGTGCTGC TGAGGCTGTA GGTGCTGTCC
2251 TTGCTCTCCT GCTCTGTGAC ACTCTCCTGG GAGTTACCCG ATTGGAGGGC
2301 GTTATCCACC TTCCACTGTA CTTTGGCCTC TCTGGGATAG AAGTTATTCA
2351 GCAGGCACAC AACAGAGGCA GTTCCAGATT TCAACTGCTC ATCAGATGGC
2401 GGGAAGATGA AGACAGATGG TGCAGCCACA GTTCCTGAGG AAAGAAGCAA
2451 ACAGGATGGT GTTTAAGTAA CAAAGTTCTG CCCTTGGGTG TGTTGTTTGC
2501 GGATAAGGGC ATGTTAGGGA CAGACAGAAA ACAGCATGCT TATCCCAGAT
2551 AATTATAGCA AGGAGACCAA GAAGCGTATT TAAAATCTTG ATGTTTTGAG
2601 TTTCTTCCTA GCTTCCCCCT ATTCCTTAAT AAAGTTCTAA ATTGTTTTGT
2651 TGGAGCTCTT TGCAGCCATT CTGAGGGCTT TGCATGCTTT TCTGACCTTG
2701 CAGTAAACTC AATGCTTTAG GCAAAGAATG GCCACGTCAT CCGACCCCCT
2751 CAGAGTTTAG AATTCATCGA TATCTAGATC CTAGATAATT GCATTCATTT
```

FIGURE 10 1/4

pGk.11

```
2801 AAAAAAAAAA TATTTCTCCT AAAATGAATA CTCAGAAAGT GGTCTTGAAA
2851 AAGATTTGTG AAGCCGTTTT GACCAGAATG TCAAAGTCTT AATAGTAAGG
2901 CAAAACAAAC AACTAAAAAA GATCATGAAC AAAGTCACTG TAAAGACTTC
2951 GGGTATTGGA AAATAATTGA ATGGAGACCA ATAATCAGAG GGAAGAATAA
3001 TAGAGTAATT TTAAGAAGTT TTCTAAATAT ATTAGAAATT AAAGACACTA
3051 AAGTCCTTCA ATTTCTTACA TAACCTAATT TTGAAAATGA ATTCTAAATA
3101 CATTTTAGAA GTCGATAAAC TTAAGTTTGG GGAAACTAGA ACTACTCAAG
3151 CTAAAATTAA AAGGTTGAAC TCAATAAGTT AAAAGAGGAC CTCTCCAGTT
3201 TCGGCTGAAT CCTCAACTTA TTTTAGAAAT GCAAATTACC CAGGTGGTGT
3251 TTTGCTCAGC CTGGACTTTC GGTTTGGTGG GGCTGGACAG AGTGTTTCAA
3301 AACCACTTCT TCAAACCACA GCTACAAGTT TACCTAGTGG TTTTATTTTC
3351 CCTTCCCCAA ATAGCCTTGC CACATGACCT GCTTCCTGCC AGCTGCTGCA
3401 GGTGTTCTGG TTCTGATCGG CCATCTTGAC TCAACTCAAC ATTGCTCAAT
3451 TCATTTAAAA ATATTTTAAA CTTAATTTAT TATTGTTAAA AGTCAGTTCT
3501 GGATAGGGTA TGAGAGAGCC TCACTCCCAT TCCTCGGTTA AACTTTAAGT
3551 AATGTCAGTT CTACACAAAC AAGACCTCAA ATTGATTGAC AAAAATTTTG
3601 GACATTTAAA AAAATGAGTA CTTGAAAACC CTCTCACATT TTAAAGTCAC
3651 AGTATTTAAC TATTTTTCCT AGGAACCAAC TTAAGAGTAA AAGCAACATC
3701 TTCTAATATT CCATACACAT ACTTCTGTGT TCCTTTGAAA GCTGGACTTT
3751 TGCAGGCTCC ACCAGACCTC TCTAGGATCT CGAGCTCGCG AAAGCTTGCA
3801 TGCCTGCAGG TCGACTCTAG AGGATCAAAC CAACTGTCTT TGAGTAGAGC
3851 CAAAATTGTT GATATACTTT GAATTTTAAT TATATTTCTT GCTGAGCAGA
3901 GGTGGCAAGA GTTTTCACTA ATGTGCAAAA CCACCTCATG TTCCCCTCAC
3951 CTGGGAGCCA GAGTAGCAGG AGGAAGAGAA GCTGAGCTGG GGCTTCCATG
4001 GTTCCCTCTG GGTCCTAACT GAGCAGTTCC TCCCCAGGGC TCTGACACAG
4051 GCATTGATAT GGGCTCTGGA AGGTAGGGCA GCTGGGAGGG ACATGCAAAG
4101 CAGCTGGGTG GGAGCTGAGC TTCCAGCTGC AGAGACCACC TGCTTCTTCC
4151 TCTCTGCACT GAGCATCCTG CGCCACCCTG GTTGTCAGGC CAGAAAAGTC
4201 TGTTGGCTCA GTCTGAGTGT AGAACTTCTC CCTTGTGCTC AGAGAATTTC
4251 ATTCCTATGT CTTTCTTCTC CTCAATCACC TAAATTCACC CAGATGATGT
4301 TTGGCACAAG CCTGTTAAGA ACAATATAAA AGGCTGTGTT TTCATTTCTC
4351 TCTTCCTATC CTCAATATGC CCAGTCATCT CCCTAAGTGC ATTATTGGAT
4401 CCAGACATGA TAAGATACAT TGATGAGTTT GGACAAACCA CAACTAGAAT
4451 GCAGTGAAAA AAATGCTTTA TTTGTGAAAT TTGTGATGCT ATTGCTTTAT
4501 TTGTAACCAT TATAAGCTGC AATAAACAAG TTAACAACAA CAATTGCATT
4551 CATTTTATGT TTCAGGTTCA GGGGGAGGTG TGGGAGGTTT TTTAAAGCAA
4601 GTAAAACCTC TACAAATGTG GTATGGCTGA TTATGATCTC TAGTCAAGGC
4651 ACTATACATC AAATATTCCT TATTAACCCC TTTACAAATT AAAAAGCTAA
4701 AGGTACACAA TTTTTGAGCA TAGTTATTAA TAGCAGACAC TCTATGCCTG
4751 TGTGGAGTAA GAAAAAACAG TATGTTATGA TTATAACTGT TATGCCTACT
4801 TATAAAGGTT ACAGAATATT TTTCCATAAT TTTCTTGTAT AGCAGTGCAG
4851 CTTTTTCCTT TGTGGTGTAA ATAGCAAAGC AAGCAAGAGT TCTATTACTA
4901 AACACAGCAT GACTCAAAAA ACTTAGCAAT TCTGAAGGAA AGTCCTTGGG
4951 GTCTTCTACC TTTCTCTTCT TTTTTGGAGG AGTAGAATGT TGAGAGTCAG
5001 CAGTAGCCTC ATCATCACTA GATGGCATTT CTTCTGAGCA AAACAGGTTT
5051 TCCTCATTAA AGGCATTCCA CCACTGCTCC CATTCATCAG TTCCATAGGT
5101 TGGAATCTAA AATACACAAA CAATTAGAAT CAGTAGTTTA ACACATTATA
5151 CACTTAAAAA TTTTATATTT ACCTTAGAGC TTTAAATCTC TGTAGGTAGT
5201 TTGTCCAATT ATGTCACACC ACAGAAGTAA GGTTCCTTCA CAAAGATCCG
5251 GGGCCCACTC ATAAATCCAG TTGCCGCCAC GGTAGCCAAT CACCGTATCG
5301 TATAAATCAT CGTCGGTACG TTCGGCATCG CTCATCACAA TACGTGCCTG
5351 GACGTCGAGG ATTTCGCGTG GGTCAATGCC GCGCCAGATC CACATCAGAC
5401 GGTTAATCAT GCGATACCAG TGAGGGATGG TTTTACCATC AAGGGCCGAC
5451 TGCACAGGCG GTTGTGCGCC GTGATTAAAG CGGCGGACTA GCGTCGAGGT
5501 TTCAGGATGT TTAAAGCGGG GTTTGAACAG GGTTTCGCTC AGGTTTGCCT
5551 GTGTCATGGA TGCAGCCTCC AGAATACTTA CTGGAAACTA TTGTAACCCG
```

FIGURE 10 2/4

pGk:11

```
2801 AAAAAAAAAA TATTTCTCCT AAAATGAATA CTCAGAAAGT GGTCTTGAAA
2851 AAGATTTGTG AAGCCGTTTT GACCAGAATG TCAAAGTCTT AATAGTAAGG
2901 CAAAACAAAC AACTAAAAAA GATCATGAAC AAAGTCACTG TAAAGACTTC
2951 GGGTATTGGA AAATAATTGA ATGGAGACCA ATAATCAGAG GGAAGAATAA
3001 TAGAGTAATT TTAAGAAGTT TTCTAAATAT ATTAGAAATT AAAGACACTA
3051 AAGTCCTTCA ATTTCTTACA TAACCTAATT TTGAAAATGA ATTCTAAATA
3101 CATTTTAGAA GTCGATAAAC TTAAGTTTGG GGAAACTAGA ACTACTCAAG
3151 CTAAAATTAA AAGGTTGAAC TCAATAAGTT AAAAGAGGAC CTCTCCAGTT
3201 TCGGCTGAAT CCTCAACTTA TTTTAGAAAT GCAAATTACC CAGGTGGTGT
3251 TTTGCTCAGC CTGGACTTTC GGTTTGGTGG GGCTGGACAG AGTGTTTCAA
3301 AACCACTTCT TCAAACCACA GCTACAAGTT TACCTAGTGG TTTTATTTTC
3351 CCTTCCCCAA ATAGCCTTGC CACATGACCT GCTTCCTGCC AGCTGCTGCA
3401 GGTGTTCTGG TTCTGATCGG CCATCTTGAC TCAACTCAAC ATTGCTCAAT
3451 TCATTTAAAA ATATTTTAAA CTTAATTTAT TATTGTTAAA AGTCAGTTCT
3501 GGATAGGGTA TGAGAGAGCC TCACTCCCAT TCCTCGGTTA AACTTTAAGT
3551 AATGTCAGTT CTACACAAAC AAGACCTCAA ATTGATTGAC AAAAATTTTG
3601 GACATTTAAA AAAATGAGTA CTTGAAAACC CTCTCACATT TTAAAGTCAC
3651 AGTATTTAAC TATTTTTCCT AGGAACCAAC TTAAGAGTAA AAGCAACATC
3701 TTCTAATATT CCATACACAT ACTTCTGTGT TCCTTTGAAA GCTGGACTTT
3751 TGCAGGCTCC ACCAGACCTC TCTAGGATCT CGAGCTCGCG AAAGCTTGCA
3801 TGCCTGCAGG TCGACTCTAG AGGATCAAAC CAACTGTCTT TGAGTAGAGC
3851 CAAAATTGTT GATATACTTT GAATTTTAAT TATATTTCTT GCTGAGCAGA
3901 GGTGGCAAGA GTTTTCACTA ATGTGCAAAA CCACCTCATG TTCCCCTCAC
3951 CTGGGAGCCA GAGTAGCAGG AGGAAGAGAA GCTGAGCTGG GGCTTCCATG
4001 GTTCCCTCTG GGTCCTAACT GAGCAGTTCC TCCCCAGGGC TCTGACACAG
4051 GCATTGATAT GGGCTCTGGA AGGTAGGGCA GCTGGGAGGG ACATGCAAAG
4101 CAGCTGGGTG GGAGCTGAGC TTCCAGCTGC AGAGACCACC TGCTTCTTCC
4151 TCTCTGCACT GAGCATCCTG CGCCACCCTG GTTGTCAGGC CAGAAAAGTC
4201 TGTTGGCTCA GTCTGAGTGT AGAACTTCTC CCTTGTGCTC AGAGAATTTC
4251 ATTCCTATGT CTTTCTTCTC CTCAATCACC TAAATTCACC CAGATGATGT
4301 TTGGCACAAG CCTGTTAAGA ACAATATAAA AGGCTGTGTT TTCATTTCTC
4351 TCTTCCTATC CTCAATATGC CCAGTCATCT CCCTAAGTGC ATTATTGGAT
4401 CCAGACATGA TAAGATACAT TGATGAGTTT GGACAAACCA CAACTAGAAT
4451 GCAGTGAAAA AAATGCTTTA TTTGTGAAAT TTGTGATGCT ATTGCTTTAT
4501 TTGTAACCAT TATAAGCTGC AATAAACAAG TTAACAACAA CAATTGCATT
4551 CATTTTATGT TTCAGGTTCA GGGGGAGGTG TGGGAGGTTT TTTAAAGCAA
4601 GTAAAACCTC TACAAATGTG GTATGGCTGA TTATGATCTC TAGTCAAGGC
4651 ACTATACATC AAATATTCCT TATTAACCCC TTTACAAATT AAAAAGCTAA
4701 AGGTACACAA TTTTTGAGCA TAGTTATTAA TAGCAGACAC TCTATGCCTG
4751 TGTGGAGTAA GAAAAAACAG TATGTTATGA TTATAACTGT TATGCCTACT
4801 TATAAAGGTT ACAGAATATT TTTCCATAAT TTTCTTGTAT AGCAGTGCAG
4851 CTTTTTCCTT TGTGGTGTAA ATAGCAAAGC AAGCAAGAGT TCTATTACTA
4901 AACACAGCAT GACTCAAAAA ACTTAGCAAT TCTGAAGGAA AGTCCTTGGG
4951 GTCTTCTACC TTTCTCTTCT TTTTTGGAGG AGTAGAATGT TGAGAGTCAG
5001 CAGTAGCCTC ATCATCACTA GATGGCATTT CTTCTGAGCA AAACAGGTTT
5051 TCCTCATTAA AGGCATTCCA CCACTGCTCC CATTCATCAG TTCCATAGGT
5101 TGGAATCTAA AATACACAAA CAATTAGAAT CAGTAGTTTA ACACATTATA
5151 CACTTAAAAA TTTTATATTT ACCTTAGAGC TTTAAATCTC TGTAGGTAGT
5201 TTGTCCAATT ATGTCACACC ACAGAAGTAA GGTTCCTTCA CAAAGATCCG
5251 GGGCCCACTC ATAAATCCAG TTGCCGCCAC GGTAGCCAAT CACCGTATCG
5301 TATAAATCAT CGTCGGTACG TTCGGCATCG CTCATCACAA TACGTGCCTG
5351 GACGTCGAGG ATTTCGCGTG GGTCAATGCC GCGCCAGATC CACATCAGAC
5401 GGTTAATCAT GCGATACCAG TGAGGGATGG TTTTACCATC AAGGGCCGAC
5451 TGCACAGGCG GTTGTGCGCC GTGATTAAAG CGGCGGACTA GCGTCGAGGT
5501 TTCAGGATGT TTAAAGCGGG GTTTGAACAG GGTTTCGCTC AGGTTTGCCT
5551 GTGTCATGGA TGCAGCCTCC AGAATACTTA CTGGAAACTA TTGTAACCCG
```

FIGURE 10 2/4

## pGk.11

```
5601 CCTGAAGTTA AAAAGAACAA CGCCCGGCAG TGCCAGGCGT TGAAAAGATT
5651 AGCGACCGGA GATTGGCGGG ACGAATACGA CGCCCATATC CCACGGCTGT
5701 TCAATCCAGG TATCTTGCGG GATATCAACA ACATAGTCAT CAACCAGCGG
5751 ACGACCAGCC GGTTTTGCGA AGATGGTGAC AAAGTGCGCT TTTGGATACA
5801 TTTCACGAAT CGCAACCGCA GTACCACCGG TATCCACCAG GTCATCAATA
5851 ACGATGAAGC CTTCGCCATC GCCTTCTGCG CGTTTCAGCA CTTTAAGCTC
5901 GCGCTGGTTG TCGTGATCGT AGCTGGAAAT ACAAACGGTA TCGACATGAC
5951 GAATACCCAG TTCACGCGCC AGTAACGCAC CCGGTACCAG ACCGCCACGG
6001 CTTACGGCAA TAATGCCTTT CCATTGTTCA GAAGGCATCA GTCGGCTTGC
6051 GAGTTTACGT GCATGGATCT GCAACATGTC CCAGGTGACG ATGTATTTTT
6101 CGCTCATGTG AAGTGTCCCA GCCTGTTTAT CTACGGCTTA AAAAGTGTTC
6151 GAGGGGAAAA TAGGTTGCGC GAGATTATAG AGATCAGCTT TTTGCAAAAG
6201 CCTAGGCCTC CAAAAAAGCC TCCTCACTAC TTCTGGAATA GCTCAGAGGC
6251 CGAGGCGGCC TCGGCCTCTG CATAAATAAA AAAAATTAGT CAGCCATGGG
6301 GCGGAGAATG GGCGGGATG GGCGGAGTTA GGGCGGAACT GGGCGGAGTT
6351 AGGGGCGGGA CTATGGTTGC TGACTAATTG AGATGCTGCA TTAATGAATC
6401 GGCCAACGCG CGGGGAGAGG CGGTTTGCGT ATTGGGCGCT CTTCCGCTTC
6451 CTCGCTCACT GACTCGCTGC GCTCGGTCGT TCGGCTGCGG CGAGCGGTAT
6501 CAGCTCACTC AAAGGCGGTA ATACGGTTAT CCACAGAATC AGGGGATAAC.
6551 GCAGGAAAGA ACATGTGAGC AAAAGGCCAG CAAAAGGCCA GGAACCGTAA
6601 AAAGGCCGCG TTGCTGGCGT TTTTCCATAG GCTCCGCCCC CCTGACGAGC
6651 ATCACAAAAA TCGACGCTCA AGTCAGAGGT GGCGAAACCC GACAGGACTA
6701 TAAAGATACC AGGCGTTTCC CCCTGGAAGC TCCCTCGTGC GCTCTCCTGT
6751 TCCGACCCTG CCGCTTACCG GATACCTGTC CGCCTTTCTC CCTTCGGGAA
6801 GCGTGGCGCT TTCTCAATGC TCACGCTGTA GGTATCTCAG TTCGGTGTAG
6851 GTCGTTCGCT CCAAGCTGGG CTGTGTGCAC GAACCCCCCG TTCAGCCCGA
6901 CCGCTGCGCC TTATCCGGTA ACTATCGTCT TGAGTCCAAC CCGGTAAGAC
6951 ACGACTTATC GCCACTGGCA GCAGCCACTG GTAACAGGAT TAGCAGAGCG
7001 AGGTATGTAG GCGGTGCTAC AGAGTTCTTG AAGTGGTGGC CTAACTACGG
7051 CTACACTAGA AGGACAGTAT TTGGTATCTG CGCTCTGCTG AAGCCAGTTA
7101 CCTTCGGAAA AAGAGTTGGT AGCTCTTGAT CCGGCAAACA AACCACCGCT
7151 GGTAGCGGTG GTTTTTTTGT TTGCAAGCAG CAGATTACGC GCAGAAAAAA
7201 AGGATCTCAA GAAGATCCTT TGATCTTTTC TACGGGGTCT GACGCTCAGT
7251 GGAACGAAAA CTCACGTTAA GGGATTTTGG TCATGAGATT ATCAAAAAGG
7301 ATCTTCACCT AGATCCTTTT AAATTAAAAA TGAAGTTTTA AATCAATCTA
7351 AAGTATATAT GAGTAAACTT GGTCTGACAG TTACCAATGC TTAATCAGTG
7401 AGGCACCTAT CTCAGCGATC TGTCTATTTC GTTCATCCAT AGTTGCCTGA
7451 CTCCCCGTCG TGTAGATAAC TACGATACGG GAGGGCTTAC CATCTGGCCC
7501 CAGTGCTGCA ATGATACCGC GAGACCCACG CTCACCGGCT CCAGATTTAT
7551 CAGCAATAAA CCAGCCAGCC GGAAGGGCCG AGCGCAGAAG TGGTCCTGCA
7601 ACTTTATCCG CCTCCATCCA GTCTATTAAT TGTTGCCGGG AAGCTAGAGT
7651 AAGTAGTTCG CCAGTTAATA GTTTGCGCAA CGTTGTTGCC ATTGCTACAG
7701 GCATCGTGGT GTCACGCTCG TCGTTTGGTA TGGCTTCATT CAGCTCCGGT
7751 TCCCAACGAT CAAGGCGAGT TACATGATCC CCCATGTTGT GCAAAAAAGC
7801 GGTTAGCTCC TTCGGTCCTC CGATCGTTGT CAGAAGTAAG TTGGCCGCAG
7851 TGTTATCACT CATGGTTATG GCAGCACTGC ATAATTCTCT TACTGTCATG
7901 CCATCCGTAA GATGCTTTTC TGTGACTGGT GAGTACTCAA CCAAGTCATT
7951 CTGAGAATAG TGTATGCGGC GACCGAGTTG CTCTTGCCCG GCGTCAATAC
8001 GGGATAATAC CGCGCCACAT AGCAGAACTT TAAAAGTGCT CATCATTGGA
8051 AAACGTTCTT CGGGGCGAAA ACTCTCAAGG ATCTTACCGC TGTTGAGATC
8101 CAGTTCGATG TAACCCACTC GTGCACCCAA CTGATCTTCA GCATCTTTTA
8151 CTTTCACCAG CGTTTCTGGG TGAGCAAAAA CAGGAAGGCA AAATGCCGCA
8201 AAAAAGGGAA TAAGGGCGAC ACGGAAATGT TGAATACTCA TACTCTTCCT
8251 TTTTCAATAT TATTGAAGCA TTTATCAGGG TTATTGTCTC ATGAGCGGAT
8301 ACATATTTGA ATGTATTTAG AAAAATAAAC AAATAGGGGT TCCGCGCACA
8351 TTTCCCCGAA AAGTGCCACC TGACGTCTAA GAAACCATTA TTATCATGAC
```

FIGURE 10 3/4

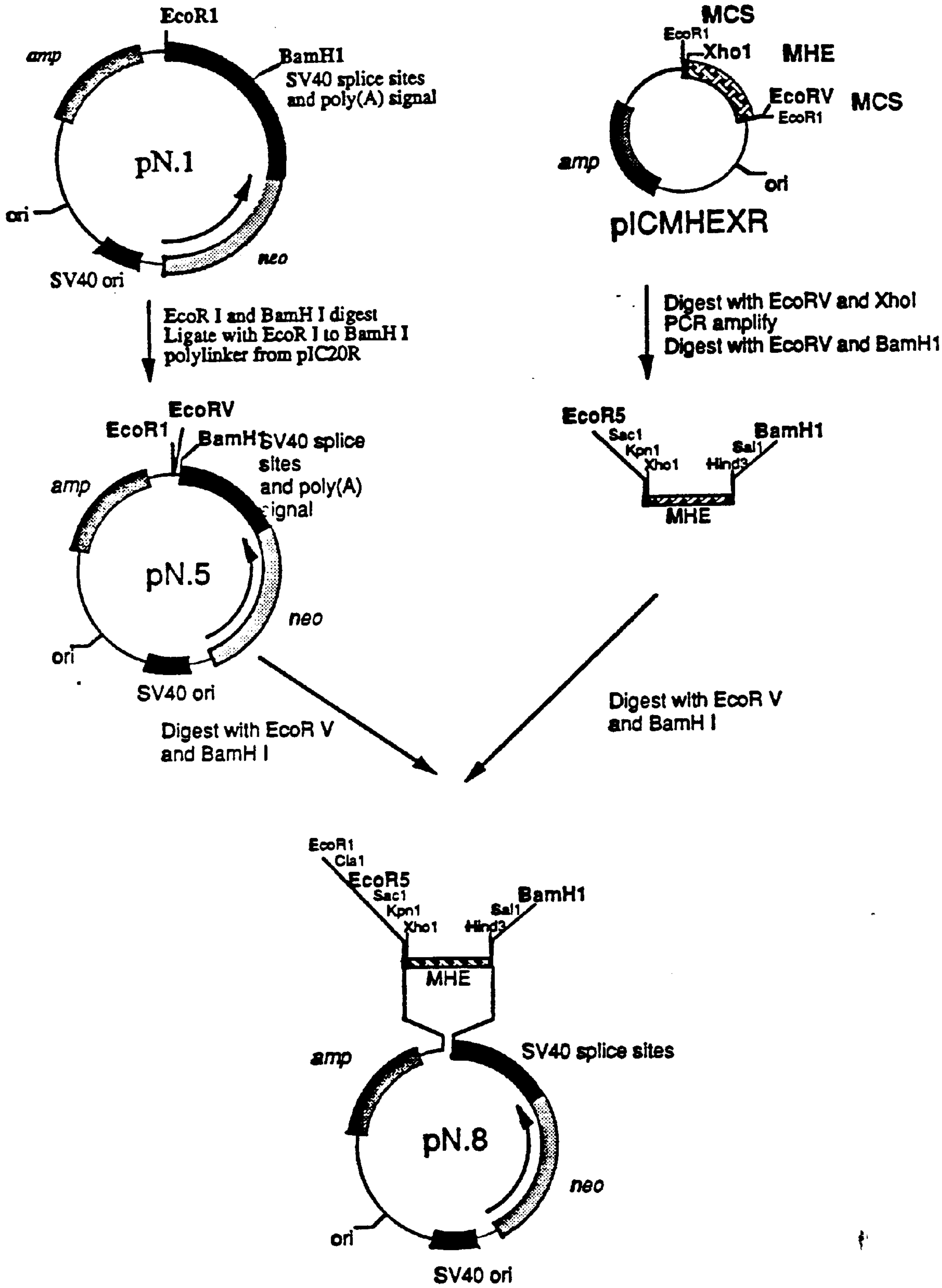
EcoR1
amp
BamH1
SV40 splice sites
and poly(A) signal
pN.1
ori
neo
SV40 ori
MCS
EcoR1
Xho1
MHE
EcoRV
EcoR1
MCS
amp
ori
pICMHEXR
EcoR I and BamH I digest
Ligate with EcoR I to BamH I
polylinker from pIC20R
Digest with EcoRV and XhoI
PCR amplify
Digest with EcoRV and BamH1
EcoRV
EcoR1
BamH1
SV40 splice sites and poly(A) signal
amp
pN.5
neo
ori
SV40 ori
EcoR5
Sac1
Kpn1
Xho1
Hind3
Sal1
BamH1
MHE
Digest with EcoR V and BamH I
Digest with EcoR V and BamH I
EcoR1
Cla1
EcoR5
Sac1
Kpn1
Xho1
Hind3
Sal1
BamH1
MHE
amp
SV40 splice sites
pN.8
neo
ori
SV40 ori

FIGURE 11 1/3

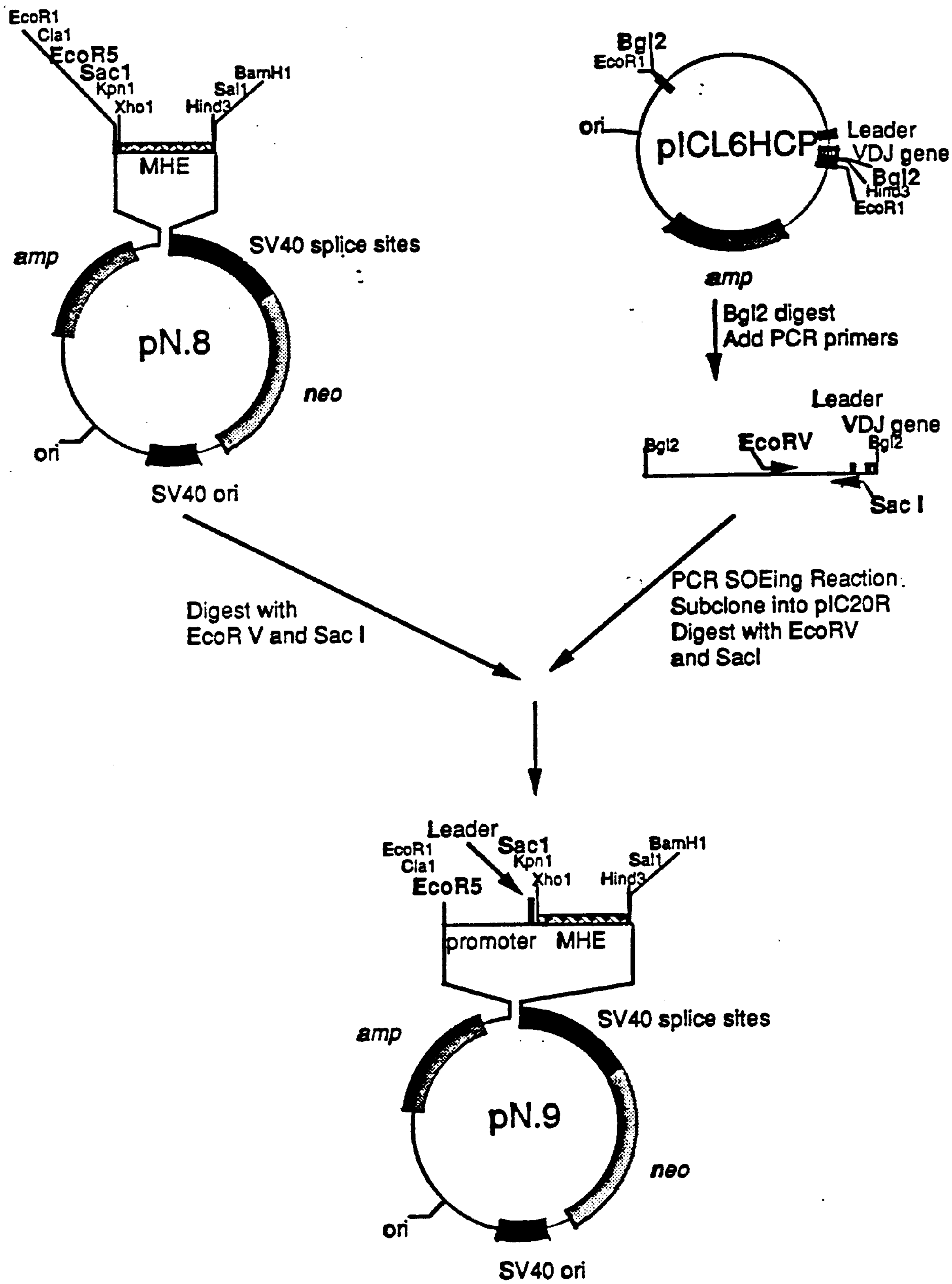
EcoR1
Cla1
EcoR5
Sac1
Kpn1
Xho1
BamH1
Sal1
Hind3
MHE
amp
SV40 splice sites
pN.8
neo
ori
SV40 ori
Bgl2
EcoR1
ori
pICL6HCP
Leader
VDJ gene
Bgl2
Hind3
EcoR1
amp
Bgl2 digest
Add PCR primers
Leader
VDJ gene
Bgl2
EcoRV
Bgl2
Sac I
Digest with
EcoR V and Sac I
PCR SOEing Reaction
Subclone into pIC20R
Digest with EcoRV
and SacI
Leader
EcoR1
Cla1
EcoR5
Sac1
Kpn1
Xho1
BamH1
Sal1
Hind3
promoter
MHE
amp
SV40 splice sites
pN.9
neo
ori
SV40 ori

FIGURE 11 2/3

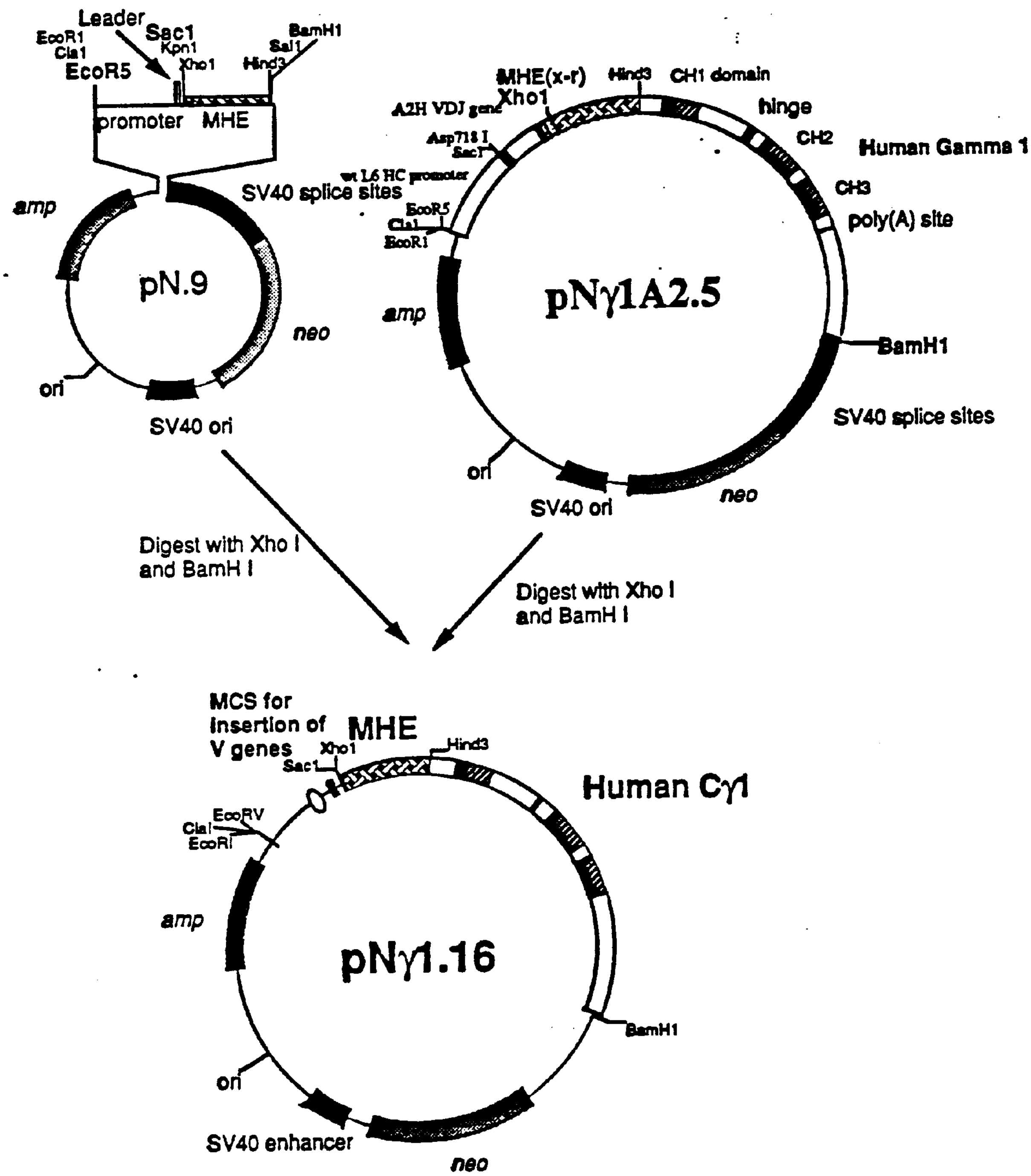
Leader
EcoR1
Cla1
EcoR5
Sac1
Kpn1
Xho1
BamH1
Sal1
Hind3
promoter MHE
amp
SV40 splice sites
pN.9
neo
ori
SV40 ori
MHE(x-r)
Xho1
Hind3
CH1 domain
A2H VDJ gene
hinge
Asp718 I
SacI
CH2
Human Gamma 1
wt L6 HC promoter
CH3
EcoR5
ClaI
EcoR1
poly(A) site
pNγ1A2.5
amp
BamH1
SV40 splice sites
ori
SV40 ori
neo
Digest with Xho I
and BamH I
Digest with Xho I
and BamH I
MCS for
insertion of
V genes
MHE
Xho1
Hind3
Sac1
Human Cγ1
EcoRV
ClaI
EcoRI
amp
pNγ1.16
BamH1
ori
SV40 enhancer
neo

FIGURE 11 3/3

## pNg1.16(2)

```
   1 AAGCTTTCTG GGGCAGGCCA GGCCTGACCT TGGCTTTGGG GCAGGGAGGG
  51 GGCTAAGGTG AGGCAGGTGG CGCCAGCCAG GTGCACACCC AATGCCCATG
 101 AGCCCAGACA CTGGACGCTG AACCTCGCGG ACAGTTAAGA ACCCAGGGGC
 151 CTCTGCGCCC TGGGCCCAGC TCTGTCCCAC ACCGCGGTCA CATGGCACCA
 201 CCTCTCTTGC AGCCTCCACC AAGGGCCCAT CGGTCTTCCC CCTGGCACCC
 251 TCCTCCAAGA GCACCTCTGG GGGCACAGCG GCCCTGGGCT GCCTGGTCAA
 301 GGACTACTTC CCCGAACCGG TGACGGTGTC GTGGAACTCA GGCGCCCTGA
 351 CCAGCGGCGT GCACACCTTC CCGGCTGTCC TACAGTCCTC AGGACTCTAC
 401 TCCCTCAGCA GCGTGGTGAC CGTGCCCTCC AGCAGCTTGG GCACCCAGAC
 451 CTACATCTGC AACGTGAATC ACAAGCCCAG CAACACCAAG GTGGACAAAC
 501 GCGTTGGTGA GAGGCCAGCA CAGGGAGGGA GGGTGTCTGC TGGAAGCCAG
 551 GCTCAGCGCT CCTGCCTGGA CGCATCCCGG CTATGCAGCC CCAGTCCAGG
 601 GCAGCAAGGC AGGCCCCGTC TGCCTCTTCA CCCGGAGGCC TCTGCCCGCC
 651 CCACTCATGC TCAGGGAGAG GGTCTTCTGG CTTTTTCCCC AGGCTCTGGG
 701 CAGGCACAGG CTAGGTGCCC CTAACCCAGG CCCTGCACAC AAAGGGGCAG
 751 GTGCTGGGCT CAGACCTGCC AAGAGCCATA TCCGGGAGGA CCCTGCCCCT
 801 GACCTAAGCC CACCCCAAAG GCCAAACTCT CCACTCCCTC AGCTCGGACA
 851 CCTTCTCTCC TCCCAGATTC CAGTAACTCC CAATCTTCTC TCTGCAGAGC
 901 CCAAATCTTG TGACAAAACT CACACATGCC CACCGTGCCC AGGTAAGCCA
 951 GCCCAGGCCT CGCCCTCCAG CTCAAGGCGG GACAGGTGCC CTAGAGTAGC
1001 CTGCATCCAG GGACAGGCCC CAGCCGGGTG CTGACACGTC CACCTCCATC
1051 TCTTCCTCAG CACCTGAACT CCTGGGGGGA CCGTCAGTCT TCCTCTTCCC
1101 CCCAAAACCC AAGGACACCC TCATGATCTC CCGGACCCCT GAGGTCACAT
1151 GCGTGGTGGT GGACGTGAGC CACGAAGACC CTGAGGTCAA GTTCAACTGG
1201 TACGTGGACG GCGTGGAGGT GCATAATGCC AAGACAAAGC CGCGGGAGGA
1251 GCAGTACAAC AGCACGTACC GTGTGGTCAG CGTCCTCACC GTCCTGCACC
1301 AGGACTGGCT GAATGGCAAG GAGTACAAGT GCAAGGTCTC CAACAAAGCC
1351 CTCCCAGCCC CCATCGAGAA AACCATCTCC AAAGCCAAAG GTGGGACCCG
1401 TGGGGTGCGA GGGCCACATG GACAGAGGCC GGCTCGGCCC ACCCTCTGCC
1451 CTGAGAGTGA CCGCTGTACC AACCTCTGTC CCTACAGGGC AGCCCCGAGA
1501 ACCACAGGTG TACACCCTGC CCCCATCTAG AGAGGAGATG ACCAAGAACC
1551 AGGTCAGCCT GACCTGCCTG GTCAAAGGCT TCTATCCCAG CGACATCGCC
1601 GTGGAGTGGG AGAGCAATGG GCAGCCGGAG AACAACTACA AGACCACGCC
1651 TCCCGTGCTG GACTCCGACG GCTCCTTCTT CCTCTACAGC AAGCTCACCG
1701 TGGACAAGAG CAGGTGGCAG CAGGGGAACG TCTTCTCATG CTCCGTGATG
1751 CATGAGGCTC TGCACAACCA CTACACGCAG AAGAGCCTCT CCCTGTCTCC
1801 GGGTAAATGA GTGCGACGGC CGGCAAGCCC CCGCTCCCCG GGCTCTCGCG
1851 GTCGCACGAG GATGCTTGGC ACGTACCCCC TGTACATACT TCCCGGGCGC
1901 CCAGCATGGA AATAAAGCAC CCAGCGCTGC CCTGGGCCCC TGCGAGACTG
1951 TGATGGTTCT TTCCACGGGT CAGGCCGAGT CTGAGGCCTG AGTGGCATGA
2001 GGGAGGCAGA GCGGGTCCCA CTGTCCCCAC ACTGGCCCAG GCTGTGCAGG
2051 TGTGCCTGGG CCCCCTAGGG TGGGGCTCAG CCAGGGGCTG CCCTCGGCAG
2101 GGTGGGGGAT TTGCCAGCGT GGCCCTCCCT CCAGCAGCAC CTGCCCTGGG
2151 CTGGGCCACG GGAAGCCCTA GGAGCCCCTG GGGACAGACA CACAGCCCCT
2201 GCCTCTGTAG GAGACTGTCC TGTTCTGTGA GCGCCCCTGT CCTCCCGACC
2251 TCCATGCCCA CTCGGGGGCA TGCCTAGTCC ATGTGCGTAG GGACAGGCCC
2301 TCCCTCACCC ATCTACCCCC ACGGCACTAA CCCCTGGCTG CCCTGCCCAG
2351 CCTCGCACCC GCATGGGGAC ACAACCGACT CCGGGGACAT GCACTCTCGG
2401 GCCCTGTGGA GGGACTGGTG CAGATGCCCA CACACACACT CAGCCCAGAC
2451 CCGTTCAACA AACCCCGCAC TGAGGTTGGC CGGCCACACG GCCACCACAC
2501 ACACACGTGC ACGCCTCACA CACGGAGCCT CACCCGGGCG AACTGCACAG
2551 CACCCAGACC AGAGCAAGGT CCTCGCACAC GTGAACACTC CTCGGACACA
2601 GGCCCCCACG AGCCCCACGC GGCACCTCAA GGCCCACGAG CCTCTCGGCA
2651 GCTTCTCCAC ATGCTGACCT GCTCAGACAA ACCCAGCCCT CCTCTCACAA
2701 GGGTGCCCCT GCAGCCGCCA CACACACACA GGGGATCACA CACCACGTCA
2751 CGTCCCTGGC CCTGGCCCAC TTCCCAGTGC CGCCCTTCCC TGCAGGACGG
```

FIGURE 12 1/4

## pNg1.16(2)

```
2801 ATCCAGACAT GATAAGATAC ATTGATGAGT TTGGACAAAC CACAACTAGA
2851 ATGCAGTGAA AAAAATGCTT TATTTGTGAA ATTTGTGATG CTATTGCTTT
2901 ATTTGTAACC ATTATAAGCT GCAATAAACA AGTTAACAAC AACAATTGCA
2951 TTCATTTTAT GTTTCAGGTT CAGGGGGAGG TGTGGGAGGT TTTTTAAAGC
3001 AAGTAAAACC TCTACAAATG TGGTATGGCT GATTATGATC TCTAGTCAAG
3051 GCACTATACA TCAAATATTC CTTATTAACC CCTTTACAAA TTAAAAAGCT
3101 AAAGGTACAC AATTTTTGAG CATAGTTATT AATAGCAGAC ACTCTATGCC
3151 TGTGTGGAGT AAGAAAAAAC AGTATGTTAT GATTATAACT GTTATGCCTA
3201 CTTATAAAGG TTACAGAATA TTTTTCCATA ATTTTCTTGT ATAGCAGTGC
3251 AGCTTTTTCC TTTGTGGTGT AAATAGCAAA GCAAGCAAGA GTTCTATTAC
3301 TAAACACAGC ATGACTCAAA AAACTTAGCA ATTCTGAAGG AAAGTCCTTG
3351 GGGTCTTCTA CCTTTCTCTT CTTTTTTGGA GGAGTAGAAT GTTGAGAGTC
3401 AGCAGTAGCC TCATCATCAC TAGATGGCAT TTCTTCTGAG CAAAACAGGT
3451 TTTCCTCATT AAAGGCATTC CACCACTGCT CCCATTCATC AGTTCCATAG
3501 GTTGGAATCT AAAATACACA AACAATTAGA ATCAGTAGTT TAACACATTA
3551 TACACTTAAA AATTTTATAT TTACCTTAGA GCTTTAAATC TCTGTAGGTA
3601 GTTTGTCCAA TTATGTCACA CCACAGAAGT AAGGTTCCTT CACAAAGATC
3651 CGGGACCAAA GCGGCCATCG TGCCTCCCCA CTCCTGCAGT TCGGGGGCAT
3701 GGATGCGCGG ATAGCCGCTG CTGGTTTCCT GGATGCCGAC GGATTTGCAC
3751 TGCCGGTAGA ACTCCGCGAG GTCGTCCAGC CTCAGGCAGC AGCTGAACCA
3801 ACTCGCGAGG GGATCGAGCC CGGGGTGGGC GAAGAACTCC AGCATGAGAT
3851 CCCCGCGCTG GAGGATCATC CAGCCGGCGT CCCGGAAAAC GATTCCGAAG
3901 CCCAACCTTT CATAGAAGGC GGCGGTGGAA TCGAAATCTC GTGATGGCAG
3951 GTTGGGCGTC GCTTGGTCGG TCATTTCGAA CCCCAGAGTC CCGCTCAGAA
4001 GAACTCGTCA AGAAGGCGAT AGAAGGCGAT GCGCTGCGAA TCGGGAGCGG
4051 CGATACCGTA AAGCACGAGG AAGCGGTCAG CCCATTCGCC GCCAAGCTCT
4101 TCAGCAATAT CACGGGTAGC CAACGCTATG TCCTGATAGC GGTCCGCCAC
4151 ACCCAGCCGG CCACAGTCGA TGAATCCAGA AAAGCGGCCA TTTTCCACCA
4201 TGATATTCGG CAAGCAGGCA TCGCCATGGG TCACGACGAG ATCCTCGCCG
4251 TCGGGCATGC GCGCCTTGAG CCTGGCGAAC AGTTCGGCTG GCGCGAGCCC
4301 CTGATGCTCT TCGTCCAGAT CATCCTGATC GACAAGACCG GCTTCCATCC
4351 GAGTACGTGC TCGCTCGATG CGATGTTTCG CTTGGTGGTC GAATGGGCAG
4401 GTAGCCGGAT CAAGCGTATG CAGCCGCCGC ATTGCATCAG CCATGATGGA
4451 TACTTTCTCG GCAGGAGCAA GGTGAGATGA CAGGAGATCC TGCCCCGGCA
4501 CTTCGCCCAA TAGCAGCCAG TCCCTTCCCG CTTCAGTGAC AACGTCGAGC
4551 ACAGCTGCGC AAGGAACGCC CGTCGTGGCC AGCCACGATA GCCGCGCTGC
4601 CTCGTCCTGC AGTTCATTCA GGGCACCGGA CAGGTCGGTC TTGACAAAAA
4651 GAACCGGGCG CCCCTGCGCT GACAGCCGGA ACACGGCGGC ATCAGAGCAG
4701 CCGATTGTCT GTTGTGCCCA GTCATAGCCG AATAGCCTCT CCACCCAAGC
4751 GGCCGGAGAA CCTGCGTGCA ATCCATCTTG TTCAATCATG CGAAACGATC
4801 CTCATCCTGT CTCTTGATCA GATCTTGATC CCCTGCGCCA TCAGATCCTT
4851 GGCGGCAAGA AAGCCATCCA GTTTACTTTG CAGGGCTTCC CAACCTTACC
4901 AGAGGGCGCC CCAGCTGGCA ATTCCGGTTC GCTTGCTGTC CATAAAACCG
4951 CCCAGTCTAG CTATCGCCAT GTAAGCCCAC TGCAAGCTAC CTGCTTTCTC
5001 TTTGCGCTTG CGTTTTCCCT TGTCCAGATA GCCCAGTAGC TGACATTCAT
5051 CCGGGGTCAG CACCGTTTCT GCGGACTGGC TTTCTACGTG TTCCGCTTCC
5101 TTTAGCAGCC CTTGCGCCCT GAGTGCTTGC GGCAGCGTGA AGCTAGCTTT
5151 TTGCAAAAGC CTAGGCCTCC AAAAAAGCCT CCTCACTACT TCTGGAATAG
5201 CTCAGAGGCC GAGGCGGCCT CGGCCTCTGC ATAAATAAAA AAAATTAGTC
5251 AGCCATGGGG CGGAGAATGG GGCGGGATGG GCGGAGTTAG GGCGGAACTG
5301 GGCGGAGTTA GGGGCGGGAC TATGGTTGCT GACTAATTGA GATGCATGCT
5351 TTGCATACTT CTGCCTGCTG GGGAGCCTGG GGACTTTCCA CACCTGGTTG
5401 CTGACTAATT GAGATGCATG CTTTGCATAC TTCTGCCTGC TGGGGAGCCT
5451 GGGGACTTTC CACACCCTAA CTGACACACA TTCCACAGCT GCCTCGCGCG
5501 TTTCGGTGAT GACGGTGAAA ACCTCTGACA CATGCAGCTC CCGGAGACGG
5551 TCACAGCTTG TCTGTAAGCG GATGCCGGGA GCAGACAAGC CCGTCAGGGC
```

FIGURE 12 2/4

## pNg1.16(2)

5601 GCGTCAGCGG GTGTTGGCGG GTGTCGGGGC GCAGCCATGA CCCAGTCACG
5651 TAGCGATAGC GGAGTGTATA CTGGCTTAAC TATGCGGCAT CAGAGCAGAT
5701 TGTACTGAGA GTGCACCATA TGCGGTGTGA AATACCGCAC AGATGCGTAA
5751 GGAGAAAATA CCGCATCAGG CGCTCTTCCG CTTCCTCGCT CACTGACTCG
5801 CTGCGCTCGG TCGTTCGGCT GCGGCGAGCG GTATCAGCTC ACTCAAAGGC
5851 GGTAATACGG TTATCCACAG AATCAGGGGA TAACGCAGGA AAGAACATGT
5901 GAGCAAAAGG CCAGCAAAAG GCCAGGAACC GTAAAAAGGC CGCGTTGCTG
5951 GCGTTTTTCC ATAGGCTCCG CCCCCCTGAC GAGCATCACA AAAATCGACG
6001 CTCAAGTCAG AGGTGGCGAA ACCCGACAGG ACTATAAAGA TACCAGGCGT
6051 TTCCCCCTGG AAGCTCCCTC GTGCGCTCTC CTGTTCCGAC CCTGCCGCTT
6101 ACCGGATACC TGTCCGCCTT TCTCCCTTCG GGAAGCGTGG CGCTTTCTCA
6151 TAGCTCACGC TGTAGGTATC TCAGTTCGGT GTAGGTCGTT CGCTCCAAGC
6201 TGGGCTGTGT GCACGAACCC CCCGTTCAGC CCGACCGCTG CGCCTTATCC
6251 GGTAACTATC GTCTTGAGTC CAACCCGGTA AGACACGACT TATCGCCACT
6301 GGCAGCAGCC ACTGGTAACA GGATTAGCAG AGCGAGGTAT GTAGGCGGTG
6351 CTACAGAGTT CTTGAAGTGG TGGCCTAACT ACGGCTACAC TAGAAGGACA
6401 GTATTTGGTA TCTGCGCTCT GCTGAAGCCA GTTACCTTCG GAAAAAGAGT
6451 TGGTAGCTCT TGATCCGGCA AACAAACCAC CGCTGGTAGC GGTGGTTTTT
6501 TTGTTTGCAA GCAGCAGATT ACGCGCAGAA AAAAAGGATC TCAAGAAGAT
6551 CCTTTGATCT TTTCTACGGG GTCTGACGCT CAGTGGAACG AAAACTCACG
6601 TTAAGGGATT TTGGTCATGA GATTATCAAA AAGGATCTTC ACCTAGATCC
6651 TTTTAAATTA AAAATGAAGT TTTAAATCAA TCTAAAGTAT ATATGAGTAA
6701 ACTTGGTCTG ACAGTTACCA ATGCTTAATC AGTGAGGCAC CTATCTCAGC
6751 GATCTGTCTA TTTCGTTCAT CCATAGTTGC CTGACTCCCC GTCGTGTAGA
6801 TAACTACGAT ACGGGAGGGC TTACCATCTG GCCCCAGTGC TGCAATGATA
6851 CCGCGAGACC CACGCTCACC GGCTCCAGAT TTATCAGCAA TAAACCAGCC
6901 AGCCGGAAGG GCCGAGCGCA GAAGTGGTCC TGCAACTTTA TCCGCCTCCA
6951 TCCAGTCTAT TAATTGTTGC CGGGAAGCTA GAGTAAGTAG TTCGCCAGTT
7001 AATAGTTTGC GCAACGTTGT TGCCATTGCT GCAGGCATCG TGGTGTCACG
7051 CTCGTCGTTT GGTATGGCTT CATTCAGCTC CGGTTCCCAA CGATCAAGGC
7101 GAGTTACATG ATCCCCCATG TTGTGCAAAA AAGCGGTTAG CTCCTTCGGT
7151 CCTCCGATCG TTGTCAGAAG TAAGTTGGCC GCAGTGTTAT CACTCATGGT
7201 TATGGCAGCA CTGCATAATT CTCTTACTGT CATGCCATCC GTAAGATGCT
7251 TTTCTGTGAC TGGTGAGTAC TCAACCAAGT CATTCTGAGA ATAGTGTATG
7301 CGGCGACCGA GTTGCTCTTG CCCGGCGTCA ACACGGGATA ATACCGCGCC
7351 ACATAGCAGA ACTTTAAAAG TGCTCATCAT TGGAAAACGT TCTTCGGGGC
7401 GAAAACTCTC AAGGATCTTA CCGCTGTTGA GATCCAGTTC GATGTAACCC
7451 ACTCGTGCAC CCAACTGATC TTCAGCATCT TTTACTTTCA CCAGCGTTTC
7501 TGGGTGAGCA AAAACAGGAA GGCAAAATGC CGCAAAAAAG GGAATAAGGG
7551 CGACACGGAA ATGTTGAATA CTCATACTCT TCCTTTTTCA ATATTATTGA
7601 AGCATTTATC AGGGTTATTG TCTCATGAGC GGATACATAT TTGAATGTAT
7651 TTAGAAAAAT AAACAAATAG GGGTTCCGCG CACATTTCCC CGAAAAGTGC
7701 CACCTGACGT CTAAGAAACC ATTATTATCA TGACATTAAC CTATAAAAAT
7751 AGGCGTATCA CGAGGCCCTT TCGTCTTCAA GAATTCATCG ATATCGGAAA
7801 ATGAAAAAAA ATATTTTTTA ATTTTAAAAT GAAATGTTTA TTTTCAATTT
7851 CTCCAAATTT CACAAGGAAA GATTAGTCAC GGGTATGGGA GAGCAGAGGA
7901 CCATAAGAGT TCAGGAATAG AATCCATTAT GATTCTGGAG TCAAGGAAGT
7951 ACTGATGCCA AGGTTTCAGT ATAAGAGCAG TATCCACTGG AAAGGATAAA
8001 GTCACTACAA CTGAGCACAG AGCAGGACAG CTACCTAATG AGTGGTCACT
8051 AATGGGCCAC TGTTACACTG TTATACGGCT TAGGAATGAG CACTGAGGCT
8101 GTGAGGTGTA TGGGTAAGGA CATCAGGATG TAAACCCAGC TCAGGTAGAG
8151 GACTCAGAGC ACAGCACAAT CAGCACGAAC TAATAAACAA CAGATAAGAT
8201 AAGGCACAAG CTCAGCAATA TTGGATCAGG GATCTTTGTA AATCTGACTG
8251 TGTATTCAGT CTAGTTCAAT GTGACTCATG AAGCCCACCC ATATGCAAAT
8301 CTAGAGAAGA CTTTAGAGTA TAAATCTGAG GCTCACCTCA CATACCAGCA
8351 AGGGAGTGAC CAGCTTGTCT TAAGGCACCA CTGAGCCCAA GTCTTAGACA

FIGURE 12 3/4

## pNg1.16(2)

```
8401 TCATGGATTG GCTGTGGAAC TTGCTATTCC TGATGGCAGC TGCCCAAGGT
8451 AAGTCATCAG AAAAAAGAGT TCCAAGGGAA ATTGAAGCAG TTCCGAGCTC
8501 GGTACCCTCG AGATCCTAGA GAGGTCTGGT GGAGCCTGCA AAAGTCCAGC
8551 TTTCAAAGGA ACACAGAAGT ATGTGTATGG AATATTAGAA GATGTTGCTT
8601 TTACTCTTAA GTTGGTTCCT AGGAAAAATA GTTAAATACT GTGACTTTAA
8651 AATGTGAGAG GGTTTTCAAG TACTCATTTT TTTAAATGTC CAAAATTTTT
8701 GTCAATCAAT TTGAGGTCTT GTTTGTGTAG AACTGACATT ACTTAAAGTT
8751 TAACCGAGGA ATGGGAGTGA GGCTCTCTCA TACCCTATCC AGAACTGACT
8801 TTTAACAATA ATAAATTAAG TTTAAAATAT TTTTAAATGA ATTGAGCAAT
8851 GTTGAGTTGA GTCAAGATGG CCGATCAGAA CCAGAACACC TGCAGCAGCT
8901 GGCAGGAAGC AGGTCATGTG GCAAGGCTAT TTGGGGAAGG GAAAATAAAA
8951 CCACTAGGTA AACTTGTAGC TGTGGTTTGA AGAAGTGGTT TTGAAACACT
9001 CTGTCCAGCC CCACCAAACC GAAAGTCCAG GCTGAGCAAA ACACCACCTG
9051 GGTAATTTGC ATTTCTAAAA TAAGTTGAGG ATTCAGCCGA AACTGGAGAG
9101 GTCCTCTTTT AACTTATTGA GTTCAACCTT TTAATTTTAG CTTGAGTAGT
9151 TCTAGTTTCC CCAAACTTAA GTTTATCGAC TTCTAAAATG TATTTAGAAT
9201 T
```

FIGURE 12 4/4